(19) Europäisches Patentamt
European Patent Office
Office européen des brevets

(11) EP 4 245 301 A1

(12) **EUROPEAN PATENT APPLICATION**
published in accordance with Art. 153(4) EPC

(43) Date of publication:
**20.09.2023 Bulletin 2023/38**

(21) Application number: **21891976.9**

(22) Date of filing: **12.11.2021**

(51) International Patent Classification (IPC):
***A61K 31/353*** *(2006.01)* ***A61K 31/282*** *(2006.01)*
***A61K 31/337*** *(2006.01)* ***A61K 31/513*** *(2006.01)*
***A61K 31/519*** *(2006.01)* ***A61K 31/7068*** *(2006.01)*
***A61K 39/395*** *(2006.01)* ***A61K 45/00*** *(2006.01)*
***A61P 1/00*** *(2006.01)* ***A61P 35/00*** *(2006.01)*
***A61P 35/04*** *(2006.01)* ***A61P 37/04*** *(2006.01)*
***A61P 43/00*** *(2006.01)*

(52) Cooperative Patent Classification (CPC):
**A61K 31/282; A61K 31/337; A61K 31/353; A61K 31/513; A61K 31/519; A61K 31/7068; A61K 39/395; A61K 45/00; A61P 1/00; A61P 35/00; A61P 35/04; A61P 37/04; A61P 43/00**

(86) International application number:
**PCT/JP2021/041650**

(87) International publication number:
**WO 2022/102731 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2020 JP 2020189152**
**21.12.2020 JP 2020211006**
**20.01.2021 JP 2021006828**

(71) Applicant: **ONO Pharmaceutical Co., Ltd.**
**Osaka-shi, Osaka 541-8526 (JP)**

(72) Inventor: **KONDO, Maki**
**Osaka-shi, Osaka 541-8564 (JP)**

(74) Representative: **Grünecker Patent- und Rechtsanwälte**
**PartG mbB**
**Leopoldstraße 4**
**80802 München (DE)**

(54) **CANCER TREATMENT BY COMBINED USE OF EP4 ANTAGONIST AND IMMUNE CHECKPOINT INHIBITOR**

(57) The object of the present invention is to provide an effective method for treating cancer. Provided is a method for treating cancer including a combination of a standard therapy, an $EP_4$ antagonist and an immune checkpoint inhibitor (for example, an anti-PD-1 antibody). The therapeutic method ofthe present invention is useful for cancer treatment.

*FIG. 1*

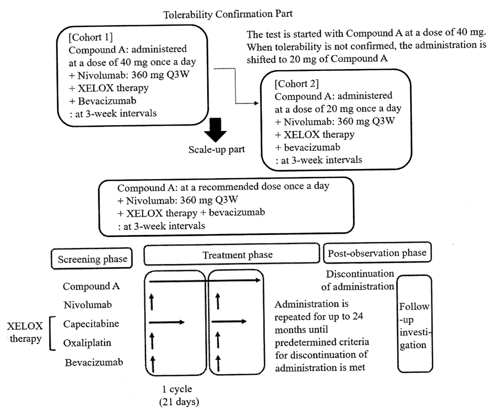

## Description

TECHNICAL FIELD

The present disclosure relates to: a cancer treatment method using a combination of a standard therapy with an $EP_4$ antagonist and an immune checkpoint inhibitor; and others.

BACKGROUND ART

The prostaglandin $E_2$ ($PGE_2$), a known metabolite of the arachidonic acid cascade, is known to have a range of effects including cytoprotection, uterine contraction, lowering of the threshold of pain, promotion of peristalsis in the digestive tract, wakefulness, inhibition of stomach acid secretion, hypotensive effect, and diuretic effect.

Recent studies have found that there are subtypes of $PGE_2$ receptors with different roles. To date, four broad subtypes are known, and these are called $EP_1$, $EP_2$, $EP_3$, and $EP_4$ (Non-Patent Literature 1).

In these subtypes, the $EP_4$ receptor is thought to be involved in inhibition of MCP-1 production from macrophages, inhibition of TNF-$\alpha$, IL-2, and IFN-$\gamma$ production from lymphocytes. This subtype is also believed to have involvement in anti-inflammation by enhanced IL-10 production, vasodilatation, angiogenesis, inhibition of elastic fiber formation, and regulation of MMP-9 expression. Other possible involvement of the $EP_4$ receptor includes immune control in cancer via myeloid derived suppressor cells, regulatory T cells, and natural killer cells.

It is therefore thought that compounds that strongly bind to the $EP_4$ receptor, and show antagonistic activity are useful for the prevention and/or treatment of diseases caused by $EP_4$ receptor activation, including, for example, a bone disease, a cancer, a systemic granulomatous disease, an immune disease, allergy, atopy, asthma, alveolar pyorrhea, gingivitis, periodontitis, Alzheimer's, Kawasaki disease, burn, multiple organ failure, chronic headache, pain, vasculitis, venous incompetence, varicose veins, aneurysm, aortic aneurysm, anal fistula, diabetes insipidus, stress, endometriosis, uterine adenomyosis, patent ductus arteriosus in neonates, and cholelithiasis (Non-Patent Literature 2-7).

Patent Literature 1 discloses that a compound represented by general formula (I) has an $EP_4$ antagonistic activity and is useful as a cancer therapeutic agent (see Patent Literature 1).

Various immune checkpoint molecules that prevent the immune response to cancer are in cancer cells or cancer microenvironment. Immune checkpoint inhibitors provide a new therapeutic method which deactivates the immunosuppression mechanism and which activates the immune reaction to cancer. As immune checkpoint inhibitors, an anti-CTLA-4 (cytotoxic T lymphocyte-associated antigen-4) antibody, ipilimumab, anti-PD-1 (programmed cell death-1) antibodies, nivolumab and pembrolizumab, and the like have already been approved in and outside Japan and are used for the treatment of cancer.

Patent Literature 2 discloses that a combination of a compound represented by general formula (I) with an immune checkpoint inhibitor is useful for cancer treatment (see Patent Literature 2).

The main treatment for unresectable advanced/recurrent colonic/rectal cancer is a drug therapy. As the drug therapy, standard therapies using a fluorinated pyrimidine-based antineoplastic agent, oxaliplatin, irinotecan, and the like are known. One of the standard therapies is a combination of XELOX and Bevacizumab (hereinafter, also abbreviated as "XELOX plus Bevacizumab therapy").

In addition, the main treatment for unresectable pancreatic cancer having distant metastasis is a drug therapy. As the standard therapy for the drug therapy, there are known a FOLFIRINOX therapy (hereinafter, also abbreviated as "FFX therapy") in which a regimen containing Fluorouracil (5-FU) is used in combination with Oxaliplatin and irinotecan, a modified FOLFIRINOX therapy (hereinafter, also abbreviated as "mFFX therapy") in which rapid administration of fluorouracil is omitted and the dose of irinotecan is reduced, or a combination of Gemcitabine and nab-Paclitaxel (hereinafter, also abbreviated as "GnP therapy") in expectation of reduction in toxicity from the FFX therapy.

Furthermore, the main treatment for stage IV or recurrent non-small cell lung cancer is a drug therapy. As a standard therapy for the drug therapy, there is known a Docetaxel-and-Ramucirumab combination therapy (hereinafter, also abbreviated as "DTX plus RAM therapy") or a Docetaxel therapy (hereinafter, also abbreviated as "DTX therapy").

Although a certain effect is observed in these therapy methods, there is an unmet need for a treatment that further prolongs a survival period.

CITATIONS LISTS

PATENT LITERATURES

Patent Literature 1: WO2016/111347

Patent Literature 2: WO2018/008711

NON-PATENT LITERATURES

Non-Patent Literature 1: Journal of Lipid Mediators and Cell Signalling, vol. 12, pp. 379-391, 1995
Non-Patent Literature 2: Pharmacological Reviews, vol. 65, pp. 1010-1052, July, 2013
Non-Patent Literature 3: 105th Annual Meeting of American Association for Cancer Research (AACR), Abstract: LB-265, Title of Presentation: ONO-AE3-208 inhibits myeloid derived suppressor cells and glioma growth, Date of Presentation: April 8, 2014
Non-Patent Literature 4: FEBS Letters, Vol. 364, pp.339-341, 1995
Non-Patent Literature 5: Cancer Science, Vol. 105, pp.1142-1151, 2014
Non-Patent Literature 6: Cancer Research, Vol. 70, pp. 1606-1615, 2010
Non-Patent Literature 7: Cancer Research, Vol. 62, pp. 28-32, 2002

SUMMARY OF INVENTION

TECHNICAL PROBLEM

An object of the present invention is to provide a novel method for treating cancer (e.g., colorectal cancer, pancreatic cancer, and lung cancer).

SOLUTIONS TO PROBLEM

The present inventors conducted intensive studies to achieve the object. As a result, the inventors have found that combination use of an $EP_4$ receptor antagonist and an immune checkpoint inhibitor in a standard therapy can be an effective cancer therapy, and additional administration of an $EP_4$ receptor antagonist or both of an $EP_4$ receptor antagonist and an immune checkpoint inhibitor to a patient who has received a preoperative chemoradiotherapy can be an effective cancer therapy (in which both of these therapies are sometimes collectively referred to as "the therapies of the present invention").

Accordingly, in one aspect,

[1] an agent for suppressing the progression of, suppressing the recurrence of and/or treating cancer, containing an $EP_4$ antagonist, as an active ingredient, which is characterized by being administered in combination with a standard therapy ((i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) Docetaxel-and-Ramucirumab therapy) and an administration of an immune checkpoint inhibitor,
[2] an agent for suppressing the progression of, suppressing the recurrence of and/or treating cancer containing an immune checkpoint inhibitor, as an active ingredient, which is characterized by being administered in combination with a standard therapy ((i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) Docetaxel-and-Ramucirumab therapy) and an administration of an $EP_4$ antagonist,
[3] an agent for suppressing the progression of, suppressing the recurrence of and/or treating cancer containing an $EP_4$ antagonist, as an active ingredient, which is characterized by being administered to a cancer patient who has undergone a preoperative chemoradiotherapy,
[4] the agent according to [3] above, wherein the agent is administered in further combination with an immune checkpoint inhibitor, or
[5] an agent for suppressing the progression of, suppressing the recurrence of and/or treating cancer containing an immune checkpoint inhibitor, as an active ingredient, which is characterized by being administered to a cancer patient who has undergone a preoperative chemoradiotherapy in combination with an $EP_4$ antagonist
is provided.

ADVANTAGEOUS EFFECTS OF INVENTION

The present invention provides a novel method for treating cancer.

BRIEF DESCRIPTION OF DRAWINGS

Fig. 1 shows an outline of a multi-center open-label, uncontrolled study for evaluating tolerability, safety and efficacy of the combination of Compound A as mentioned below, Nivolumab and XELOX plus Bevacizumab therapy in patients with curatively unresectable advanced or recurrent colonic/rectal cancer.

Fig. 2 shows an outline of a multi-center open-label, uncontrolled study for evaluating safety, efficacy, and pharmacokinetics of the combination of Compound A as mentioned below and Nivolumab as a preoperative adjuvant therapy after a preoperative chemoradiotherapy for locally advanced rectal cancer that can be curatively resected.

Fig. 3 shows an outline of a multi-center open-label, uncontrolled study for evaluating tolerability, safety, and efficacy of the combination of Compound A as mentioned below, Nivolumab, and mFFX therapy or GnP therapy in pancreatic cancer patients with distant metastasis.

Fig. 4 shows an outline of a multi-center open-label, uncontrolled study for evaluating tolerability, safety, and efficacy of the combination of Compound A as mentioned below, Nivolumab and Docetaxel-and-Ramucirumab therapy in patients with advanced or recurrent non-small cell lung cancer refractory to a combination therapy including an anti-PD-1 or anti-PD-L1 antibody and a platinum preparation.

DESCRIPTION OF EMBODIMENTS

(1) $EP_4$ antagonists

In the present invention, the $EP_4$ antagonist is not particularly limited as long as the $EP_4$ antagonist is a compound having an $EP_4$ antagonistic activity. In one embodiment, the $EP_4$ antagonist is a compound or a salt thereof represented by general formula (I) described in WO 2016/111347:

[Formula 1]

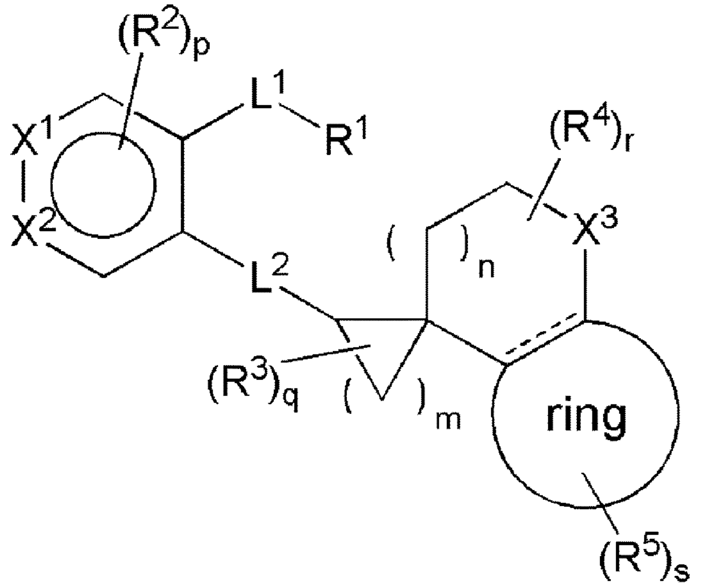

(I)

(wherein $R^1$ represents $COOR^8$, tetrazole, $SO_3H$, $SO_2NH_2$, $SO_2NHR^{8-1}$, $CONHSO_2R^{8-1}$, $SO_2NHCOR^{8-1}$, or hydroxamic acid,

wherein $R^8$ represents a hydrogen atom, C1-4 alkyl, or benzyl, and

$R^{8-1}$ represents C1-4 alkyl, C1-4 haloalkyl, a C3-10 carbon ring, or a three- to ten-membered heterocyclic ring, wherein the C3-10 carbon ring and the three- to ten-membered heterocyclic ring each may be substituted with C1-4 alkyl, C1-4 haloalkyl, C1-4 alkoxy, - O(C1-4 haloalkyl), C1-4 alkylthio, -S(C1-4 haloalkyl), halogen, or nitrile (here and below, "-CN"),

$L^1$ represents C1-5 alkylene, C2-5 alkenylene, or C2-5 alkynylene,

$R^2$ represents halogen, C1-4 alkyl, C1-4 alkoxy, C1-4 alkylthio, C2-4 alkenyl, C2-4 alkynyl, -O(C1-4 haloalkyl), -S(C1-4 haloalkyl), -C(O)(C1-4 alkyl), $-SO_2$(C1-4 alkyl), - CONH(C1-4 alkyl), -CON(C1-4 alkyl)$_2$, -NHC(O)(C1-4

alkyl), -N(C1-4 alkyl)C(O)(C1-4 alkyl), -NHSO$_2$(C1-4 alkyl), -N(C1-4 alkyl)SO$_2$(C1-4 alkyl), -SO$_2$NH(C1-4 alkyl), -SO$_2$N(C1-4 alkyl)$_2$, -NR$^{17}$R$^{17}$, nitro, nitrile, a hydroxyl group, aldehyde (here and below, formyl), or carboxyl, wherein the C1-4 alkyl groups each may be substituted with halogen, and

the (C1-4 alkyl)$_2$ in R$^2$ represents two independent C1-4 alkyl groups which may be the same or different,

X$^1$ represents CR$^6$ or a nitrogen atom, wherein R$^6$ represents a hydrogen atom or R$^2$,

X$^2$ represents CR$^7$ or a nitrogen atom, wherein R$^7$ represents a hydrogen atom, R$^2$, or -L$^3$-R$^9$, wherein L$^3$ represents methylene, an oxygen atom, or a sulfur atom which may be oxidized, and R$^9$ represents a four- to ten-membered heterocyclic ring which may be substituted with a substituent selected from the group consisting of halogen, C1-4 alkyl, and C1-4 haloalkyl,

L$^2$ represents -CH$_2$CH$_2$-, -CH=CH-, -CH$_2$O-, -OCH$_2$-, -CH$_2$S-, -SCH$_2$-, -CH$_2$S(O)-, -S(O)CH$_2$-, -CH$_2$SO$_2$-, -SO$_2$CH$_2$-, -CH$_2$NH-, -NHCH$_2$-, -NHCO-, -CONH-, -NHSO$_2$-, or - SO$_2$NH-,

R$^3$ represents C1-4 alkyl or halogen,

R$^4$ represents halogen, C1-4 alkyl, or C1-4 haloalkyl,

X$^3$ represents methylene, an oxygen atom, a sulfur atom which may be oxidized, or NR$^{10}$, wherein R$^{10}$ represents C1-4 alkyl, -C(O)(C1-4 alkyl), -C(O)O(C1-4 alkyl), or - SO$_2$(C1-4 alkyl), wherein the C1-4 alkyl groups each may be substituted with halogen,

the ring represents a benzene ring or a five- or six-membered monocyclic aromatic heterocyclic ring,

[Formula 2]

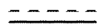

represents a single bond or a double bond,

R$^5$ represents (1) halogen, (2) C1-4 alkyl, (3) carboxyl, (4) nitrile, (5) -CONHR$^{11}$, (6) -C(O)R$^{12}$, (7) -OR$^{14}$, (8) -S(O)$_t$R$^{15}$, (9) -CH$_2$R$^{16}$, (10) -NR$^{17}$R$^{17}$, (11) -NHCOR$^{11}$, (12) a C4-10 carbon ring, or (13) a four- to ten-membered heterocyclic ring, wherein the C4-10 carbon ring or the four- to ten-membered heterocyclic ring may be substituted with one to three R$^{18}$, wherein, when a plurality of R$^{18}$ exists, the plurality of R$^{18}$ each independently may be the same or different,

R$^{11}$ represents C1-6 alkyl, C3-6 cycloalkyl, phenyl, or a four- to six-membered heterocyclic ring and may be substituted with one to three R$^{13}$, wherein, when a plurality of R$^{13}$ exists, the plurality of R$^{13}$ each independently may be the same or different, and

R$^{13}$ represents halogen, C1-6 alkyl, C3-6 cycloalkyl, C1-4 alkoxy, a hydroxyl group, -NR$^{20}$R$^{21}$, benzene, or a four- to six-membered heterocyclic ring,

wherein R$^{20}$ and R$^{21}$ each independently represent a hydrogen atom or C1-4 alkyl,

R$^{12}$ represents C1-6 alkyl, C3-6 cycloalkyl, benzene, or a four- to six-membered heterocyclic ring, wherein the C3-6 cycloalkyl, the benzene, and the four- to six-membered heterocyclic ring each independently may be substituted with halogen, C1-4 alkyl, or C1-4 alkoxy,

R$^{14}$ represents a hydrogen atom, C1-6 alkyl, C3-6 cycloalkyl, benzene, or benzyl, wherein the C1-6 alkyl may be substituted with one to three R$^{19}$, wherein, when a plurality of R$^{19}$ exists, the plurality of R$^{19}$ each independently may be the same or different, and

R$^{19}$ represents C1-4 alkoxy, -CONH(C1-4 alkyl), -CON(C1-4 alkyl)$_2$, or a five- or six-membered monocyclic aromatic heterocyclic ring which may be substituted with a substituent selected from the group consisting of C1-4 alkyl and C1-4 haloalkyl,

wherein the (C1-4 alkyl)$_2$ in R$^{19}$ represents two independent C1-4 alkyl groups which may be the same or different,

R$^{15}$ represents C1-6 alkyl, C3-6 cycloalkyl, benzene, or benzyl,

R$^{16}$ represents a hydroxyl group or C1-4 alkoxy,

each R$^{17}$ independently represents a hydrogen atom, C1-6 alkyl, or C3-6 cycloalkyl, and

R$^{18}$ represents halogen, C1-6 alkyl, C3-6 cycloalkyl, C1-4 alkoxy, oxo, nitrile, a hydroxyl group, hydroxymethyl, 1-methyl-1-hydroxyethyl, (C1-4 alkyl)SO$_2$-, a four- to six-membered heterocyclic ring, (C1-4 alkyl)NH-, or (C1-4 alkyl)$_2$N-,

wherein the (C1-4 alkyl)$_2$ in R$^{18}$ represents two independent C1-4 alkyl groups which may be the same or different,

m represents an integer of 1 to 4,

n represents an integer of 0 to 4,

p represents an integer of 0 to 2,

q represents an integer of 0 to 6,

r represents an integer of 0 to 6,

s represents an integer of 0 to 4,

t represents an integer of 0 to 2, and

$R^2$, $R^3$, $R^4$, and $R^5$ each independently may be the same or different when p, q, r, and s are each an integer of 2 or more).

In another aspect, the $EP_4$ antagonist is AN0025, E7046, IK-007, RMX-1002, grapiprant, AAT-007, CR6086, INV-1120, BYD-001, TT-038, DT095895, P-001, ER-819762, MK-2894, MF498, evatanepag, CJ-042794, EP4A, BGC201531, CJ-023423, GW627368, AH23848, DT-9081, and compounds respectively described in WO2001/062708, WO2002/020462, WO2002/032900, WO2002/050031, WO2002/050032, WO2002/050033, WO2002/016311, WO2003/086390, WO2003/087061, WO2003/099857, WO2003/016254, WO2005/021508, WO2004/067524, WO2005/037812, WO2005/061475, WO2005/105732, WO2005/105733, WO2006/122403, WO2007/121578, WO2008/017164, WO2008/104055, WO2008/116304, WO2008/123207, WO2007/143825, WO2009/005076, WO2009/139373, WO2010/019796, WO2010/034110, WO2012/039972, WO2012/043634, WO2012/076063, WO2012/103071, WO2013/004290, WO2013/096496, WO2013/096501, WO2013/101733, WO2013/101598, WO2014/004229, WO2014/004230, WO2014/086739, WO2014/126746, WO2014/186218, WO2014/200075, WO2015/094902, WO2015/094912, WO2015/091475, WO2015/113057, WO2015/147020, WO2016/021742, WO2016/088903, WO2017/014323, WO2017/066633, WO2017/085198, WO2018/195123, WO2018/195123, WO2018/210987, WO2018/210988, WO2018/210992, WO2018/210994, WO2018/210995, WO2018/216640, WO2019/038156, WO2019/245590, WO2019/101171, WO2019/152982, WO2019/166022, CN108929281, WO2020/012305, WO2020/014465, WO2020/151566, WO2020/160075, and WO2020/161275.

In the present invention, "C1-4 alkyl" is, for example, methyl, ethyl, n-propyl, isopropyl, n-butyl, sec-butyl, tert-butyl, or isobutyl.

In the present invention, "C1-3 alkyl" is, for example, methyl, ethyl, n-propyl, or isopropyl.

In the present invention, "C1-5 alkylene" is, for example, methylene, ethylene, propylene, butylene, or pentylene.

In the present invention, "C2-5 alkenylene" is, for example, ethenylene, 1-propenylene, 2-propenylene, 1-butenylene, 2-butenylene, 3-butenylene, 1-pentenylene, 2-pentenylene, 3-pentenylene, or 4-pentenylene.

In the present invention, "C2-5 alkynylene" is, for example, ethynylene, 1-propynylene, 2-propynylene, 1-butynylene, 2-butynylene, 3-butynylene, 1-pentynylene, 2-pentynylene, 3-pentynylene, or 4-pentynylene.

In the present invention, "halogen" is fluorine, chlorine, bromine, or iodine.

In the present invention, "C1-4 alkoxy" is, for example, methoxy, ethoxy, propoxy, isopropoxy, butoxy, 1-methylpropoxy, tert-butoxy, or isobutoxy.

In the present invention, "C1-4 alkylthio" is, for example, methylthio, ethylthio, propylthio, isopropylthio, butylthio, 1-methylpropylthio, tert-butylthio, or isobutylthio.

In the present invention, "C2-4 alkenyl" is, for example, ethenyl, 1-propenyl, 2-propenyl, 1-butenyl, 2-butenyl, or 3-butenyl.

In the present invention, "C2-4 alkynyl" is, for example, ethynyl, 1-propynyl, 2-propynyl, 1-butynyl, 2-butynyl, or 3-butynyl.

In the present invention, "C1-4 haloalkyl" represents halogen-substituted C1-4 alkyl, and is, for example, monofluoromethyl, difluoromethyl, trifluoromethyl, 2-fluoroethyl, 1-fluoroethyl, 2,2-difluoroethyl, 1,2-difluoroethyl, 1,1-difluoroethyl, 2,2,2-trifluoroethyl, 1,2,2-trifluoroethyl, 1,1,2-trifluoroethyl, 1,2,2,2-tetrafluoroethyl, 1,1,2,2-tetrafluoroethyl, pentafluoroethyl, 1,2-dibromo-1,2,2-trifluoroethyl, 1-chloro-1,2,2,2-tetrafluoroethyl, 3-fluoropropyl, 3-chloropropyl, 2-fluoropropyl, 2-chloropropyl, 1-fluoropropyl, 1-chloropropyl, 3,3-difluoropropyl, 2,3-difluoropropyl, 1,3-difluoropropyl, 1,2-difluoropropyl, 2,2-difluoropropyl, 1,1-difluoropropyl, 3,3,3-trifluoropropyl, 2,3,3-trifluoropropyl, 1,3,3-trifluoropropyl, 1,2,2-trifluoropropyl, 1,1,2-trifluoropropyl, 1,1,3-trifluoropropyl, 1,1,2,2-tetrafluoropropyl, 2,2,3,3,3-pentafluoropropyl, 4-fluorobutyl, 4-chlorobutyl, 3-fluorobutyl, 3-chlorobutyl, 2-fluorobutyl, 2-chlorobutyl, 1-fluorobutyl, 1-chlorobutyl, 3,3-difluorobutyl, 2,3-difluorobutyl, 1,3-difluorobutyl, 1,2-difluorobutyl, 2,2-difluorobutyl, 1,1-difluorobutyl, 3,3,3-trifluorobutyl, 2,3,3-trifluorobutyl, 1,3,3-trifluorobutyl, 1,2,2-trifluorobutyl, 1,1,2-trifluorobutyl, 1,1,3-trifluorobutyl, 1, 1,2,2-tetrafluorobutyl, or 2,2,3,3,3-pentafluorobutyl.

In the present invention, "sulfur that may be oxidized" represents sulfur (S), sulfoxide (S(O)), or sulfone ($SO_2$).

In the present invention, "four- to ten-membered heterocyclic ring" means a four- to ten-membered monocyclic or bicyclic heterocyclic ring containing 1 to 5 heteroatoms selected from an oxygen atom, a nitrogen atom, and a sulfur atom, and is, for example, an oxetane, azetidine, pyrrolidine, pyrrole, imidazole, triazole, tetrazole, pyrazole, pyridine, piperidine, piperazine, pyrazine, pyrimidine, pyridazine, azepine, diazepine, furan, pyran, oxepin, thiophene, thiopyran, thiepine, oxazole, isooxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, indazole, quinoline, isoquinoline, quinolizine, purine, phthalazine, pteridin, naphthyridine, quinoxaline, quinazoline, cinnoline, benzooxazole, benzothiazole, benzoimidazole, benzodioxole, benzooxathiol, chromene, benzofurazan, benzothiadiazole, benzotriazole, pyrroline, pyrrolidine, imidazoline, imidazolidine, triazoline, triazolidine, tetrazoline, tetrazolidine, pyrazoline, pyrazolidine, dihydropyridine, tetrahydropyridine, dihydropyrazine, tetrahydropyrazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepin, tetrahydrooxepin, perhydrooxepin, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisooxazole, tetrahydroisooxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, tetrahydrotriazolopyrazine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzooxathiane, dihydrobenzooxazine, dihydrobenzothiazine, pyrazinomorpholine, dihydrobenzooxazole, perhydrobenzooxazole, dihydrobenzothiazole, perhydrobenzothiazole, dihydrobenzoimidazole, perhydrobenzoimidazole, dioxolan, dioxane, dioxaindan, benzodioxane, thiochromane, dihydrobenzodioxine, dihydrobenzoxathiin, chromane, pyrazolopyrimidine, imidazopyridazine, imidazopyridine, imidazopyrimidine, pyrrolopyridine, pyrrolopyrimidine, pyrrolopyridazine, imidazopyrazine, pyrazolopyridine, pyrazolopyrimidine, triazolopyridine, or dihydropyridooxazine ring.

In the present invention, "three- to ten-membered heterocyclic ring" means a three-to ten-membered monocyclic or bicyclic heterocyclic ring containing 1 to 5 heteroatoms selected from an oxygen atom, a nitrogen atom, and a sulfur atom, and is, for example, aziridine, oxirane, thiirane, or any of the heterocyclic rings exemplified above for the "four- to ten-membered heterocyclic ring.

In the present invention, "five- to ten-membered aromatic heterocyclic ring" means a five- to ten-membered monocyclic or bicyclic aromatic heterocyclic ring containing 1 to 4 heteroatoms selected from an oxygen atom, a nitrogen atom, and a sulfur atom, and is, for example, a pyrrole, imidazole, triazole, tetrazole, pyrazole, furan, thiophene, oxazole, isooxazole, thiazole, isothiazole, furazan, oxadiazole, thiadiazole, pyridine, pyrazine, pyrimidine, pyridazine, indole, isoindole, benzofuran, isobenzofuran, benzothiophene, isobenzothiophene, indazole, purine, benzooxazole, benzothiazole, benzoimidazole, benzofurazan, benzothiadiazole, benzotriazole, quinoline, isoquinoline, phthalazine, pteridin, naphthyridine, quinoxaline, quinazoline, or cinnoline ring.

In the present invention, "five- to six-membered monocyclic aromatic heterocyclic ring" is, for example, a pyrrole, imidazole, triazole, tetrazole, pyrazole, pyridine, pyrazine, pyrimidine, pyridazine, furan, thiophene, oxazole, isooxazole, thiazole, isothiazole, furazan, oxadiazole, or thiadiazole ring.

In the present invention, "C4-10 carbon ring" means a C4 to 10 monocyclic or bicyclic carbon ring, and is, for example, a cyclobutane, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, or perhydronaphthalene ring.

In the present invention, "C3-10 carbon ring" means a C3 to 10 monocyclic or bicyclic carbon ring, and is, for example, cyclopropane, or any of the carbon rings exemplified above for the "C4-10 carbon ring."

In the present invention, "C1-6 alkyl" is, for example, methyl, ethyl, n-propyl, isopropyl, n-butyl, sec-butyl, tert-butyl, isobutyl, pentyl, 1-methylbutyl, 2-methylbutyl, 3-methylbutyl, 1,1-dimethylpropyl, 1,2-dimethylpropyl, 2,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 1-methyl-1-ethylpropyl, 2-methyl-2-ethylpropyl, 1-ethylbutyl, 2-ethylbutyl, or 1,1-dimethylpentyl.

In the present invention, "C3-6 cycloalkyl" is cyclopropyl, cyclobutyl, cyclopentyl, or cyclohexyl.

In the present invention, "four- to six-membered heterocyclic ring" means a four- to six-membered monocyclic heterocyclic ring containing 1 to 4 heteroatoms selected from an oxygen atom, a nitrogen atom, and a sulfur atom, and is, for example, an oxetane, azetidine, pyrrolidine, piperidine, pyrazine, pyran, thiopyran, oxazine, oxadiazine, thiazine, thiadiazine, pyrrole, imidazole, triazole, tetrazole, pyrazole, pyridine, pyrimidine, pyridazine, furan, thiophene, oxazole, isooxazole, thiazole, isothiazole, furazan, oxadiazole, or thiadiazole ring.

In the present invention, $R^1$ is preferably $COOR^8$.

In the present invention, $R^8$ is preferably a hydrogen atom or C1-4 alkyl, more preferably a hydrogen atom.

In the present invention, $R^{8-1}$ is preferably C1-4 alkyl, benzene, or pyridine. The benzene and the pyridine may be substituted with C1-4 alkyl, C1-4 haloalkyl, C1-4 alkoxy, - O(C1-4 haloalkyl), C1-4 alkylthio, -S(C1-4 haloalkyl),

halogen, or nitrile.

In the present invention, $L^1$ is preferably C1-5 alkylene, or C2-5 alkenylene, more preferably C1-5 alkylene, particularly preferably propylene.

In the present invention, $R^2$ is preferably a fluorine atom.

In the present invention, $X^1$ is preferably $CR^6$.

In the present invention, $R^8$ is preferably a hydrogen atom, or C1-4 alkyl, more preferably a hydrogen atom.

In the present invention, $X^2$ is preferably $CR^7$.

In the present invention, $R^7$ is preferably fluorine, nitrile, $-CH_2R^9$, or $-OR^9$, more preferably nitrile.

In the present invention, $R^9$ is preferably a four- to ten-membered heterocyclic ring which may be substituted with methyl or trifluoromethyl. The four- to ten-membered heterocyclic ring is preferably a five- to ten-membered aromatic heterocyclic ring, more preferably a five- to ten-membered nitrogen-containing aromatic heterocyclic ring (for example, pyrazole, imidazole, triazole, pyrrolopyridine, pyrrolopyrimidine, pyrrolopyridazine, imidazopyridazine, imidazopyridine, imidazopyrimidine, imidazopyrazine, pyrazolopyridine, or pyrazolopyrimidine).

In the present invention, $L^2$ is preferably -CH=CH-, -NHCO-, -CONH-, $-NHSO_2-$, or $-SO_2NH-$, more preferably -NHCO-, or -CONH-, particularly preferably -NHCO-.

In the present invention, $R^3$ is preferably a fluorine atom.

In the present invention, $R^4$ is preferably methyl, ethyl, or trifluoromethyl, more preferably methyl.

In the present invention, $X^3$ is preferably methylene, or an oxygen atom, more preferably an oxygen atom.

In the present invention, $R^{10}$ is preferably methyl, ethyl, methylcarbonyl, ethylcarbonyl, methylsulfonyl, ethylsulfonyl, or tert-butoxycarbonyl.

In the present invention, the ring is preferably a benzene, thiophene, or pyrazole ring, more preferably a benzene ring.

In the present invention, $R^5$ is preferably $-CONHR^{11}$, a fluorine atom, methoxy, a benzene ring, or a four- to ten-membered heterocyclic ring. The four- to ten-membered heterocyclic ring is preferably an azetidine, pyrrolidine, piperidine, oxazolidine, oxadiazole, triazole, thiophene, furan, pyrazole, thiazole, oxazole, imidazole, pyridine, pyrazine, pyridazine, pyrimidine, pyrazolopyrimidine, pyrrolopyrimidine, pyrazolopyridine, pyrrolopyridine, or dihydropyridooxazine ring.

In the present invention, $R^{11}$ is preferably C1-6 alkyl, C3-6 cycloalkyl, or a pyran, pyrrolidine, piperidine, pyrazole, thiazole, oxazole, isooxazole, pyridine, pyridazine, or pyrimidine ring, more preferably C1-6 alkyl.

In the present invention, $R^{13}$ is preferably halogen, C1-6 alkyl, C3-6 cycloalkyl, C1-4 alkoxy, a hydroxyl group, $-NR^{20}R^{21}$, or a benzene, oxetane, pyridine, pyrazole, or oxazole ring, more preferably fluorine, methyl, ethyl, n-propyl, isopropyl, n-butyl, sec-butyl, tert-butyl, isobutyl, cyclopentyl, cyclobutane, oxetane, a hydroxyl group, methoxy, ethoxy, propoxy, isopropoxy, dimethylamino, or a benzene, pyridine, pyrazole, or oxazole ring.

In the present invention, $R^{20}$ is preferably a hydrogen atom.

In the present invention, $R^{21}$ is preferably a hydrogen atom or methyl.

In the present invention, $R^{12}$ is preferably C1-3 alkyl, C3-6 cycloalkyl, benzene, or a four- to six-membered heterocyclic ring. The four- to six-membered heterocyclic ring is preferably an oxetane, azetidine, pyrrolidine, piperidine, pyrazine, pyran, thiopyran, oxazine, oxadiazine, thiazine, thiadiazine, pyrrole, imidazole, triazole, tetrazole, pyrazole, pyridine, pyrazine, pyrimidine, pyridazine, furan, thiophene, oxazole, isooxazole, thiazole, isothiazole, furazan, oxadiazole, or thiadiazole ring. The four- to six-membered heterocyclic ring may be substituted with C1-4 alkoxy.

In the present invention, $R^{14}$ is preferably a hydrogen atom, methyl, ethyl, benzene, or benzyl.

In the present invention, $R^{19}$ is preferably methoxy, $-CONHCH_3$, $-CON(CH_3)_2$, or an oxazole, thiazole, pyrazole, or pyridine ring.

In the present invention, $R^{15}$ is preferably methyl, cyclopropyl, or benzene.

In the present invention, $R^{16}$ is preferably a hydroxyl group.

In the present invention, $R^{17}$ is preferably methyl, ethyl or cyclopropyl, and more preferably methyl.

In the present invention, $R^{18}$ is preferably a fluorine atom, methyl, ethyl, n-propyl, isopropyl, n-butyl, sec-butyl, tert-butyl, isobutyl, cyclopropyl, methoxy, ethoxy, n-propoxy, isopropoxy, oxo, nitrile, a hydroxyl group, hydroxymethyl, 1-methyl-1-hydroxyethyl, methylsulfonyl, pyridine, or dimethylamino.

In the present invention, m is preferably an integer of 1 to 2, and more preferably 1.

In the present invention, n is preferably an integer of 0 to 1, and more preferably 1.

In the present invention, p is preferably 0.

In the present invention, q is preferably 0.

In the present invention, r is preferably an integer of 0 to 4, and more preferably an integer of 0 to 2.

In the present invention, s is preferably an integer of 0 to 2, and more preferably 1 or 2.

In the present invention, t is preferably an integer of 0 to 2.

In the present invention, $X^{3a}$ is preferably an oxygen atom.

In the present invention, na is preferably an integer of 0 to 1, and more preferably 1.

In the present invention, qa is preferably 0.

In the present invention, ra is preferably an integer of 0 to 2.

In the present invention, preferred as the compound of general formula (I) is a combination of the preferred definitions of the ring, $R^1$, $R^2$, $R^3$, $R^4$, $R^5$, $R^6$, $R^7$, $R^8$, $R^{8-1}$, $R^9$, $R^{10}$, $R^{11}$, $R^{12}$, $R^{13}$, $R^{14}$ , $R^{15}$, $R^{16}$, $R^{17}$, $R^{18}$, $R^{19}$, $R^{20}$, $R^{21}$, $L^1$, $L^2$, $L^3$, $X^1$, $X^2$, $X^3$, m, n, p, q, r, s and t.

In the present invention, the compound represented by general formula (I) is preferably a compound or a salt thereof represented by the following general formula (I-a):

[Formula 3]

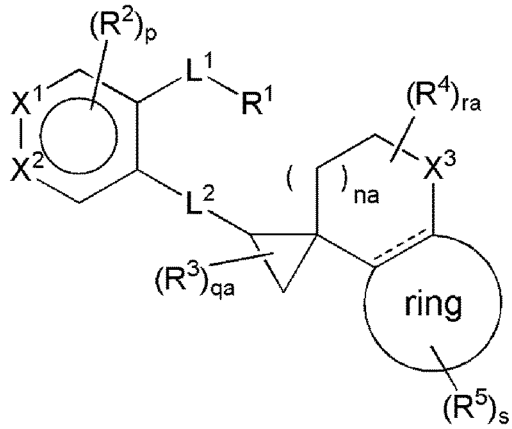

(I-a)

(wherein na represents an integer of 0 to 1, qa represents an integer of 0 to 3, ra represents an integer of 0 to 4, and other symbols are as defined above), more preferably a compound or a salt thereof represented by general formula (I-1) or a salt thereof:

[Formula 4]

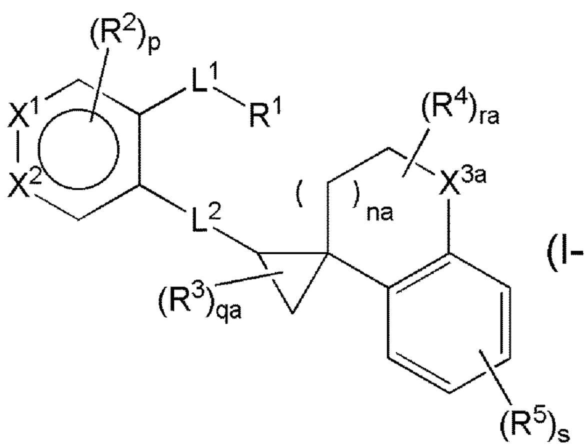

(I-1)

(wherein na represents an integer of 0 to 1, qa represents an integer of 0 to 3, ra represents an integer of 0 to 4, $X^{3a}$ represents methylene or an oxygen atom, and other symbols are as defined above).

In the present invention, another embodiment of the compound represented by general formula (I-) is a compound or a salt thereof represented by general formula (I-b):

[Formula 5]

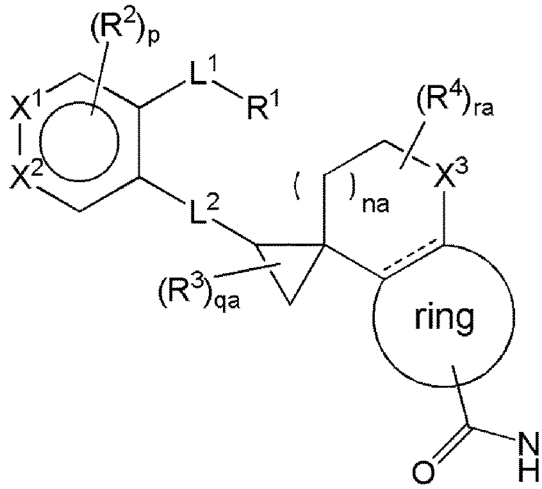

(I-b)

(wherein na represents an integer of 0 to 1, qa represents an integer of 0 to 3, ra represents an integer of 0 to 4, and other symbols are as defined above), more preferably a compound or a salt thereof represented by general formula (I-c):

[Formula 6]

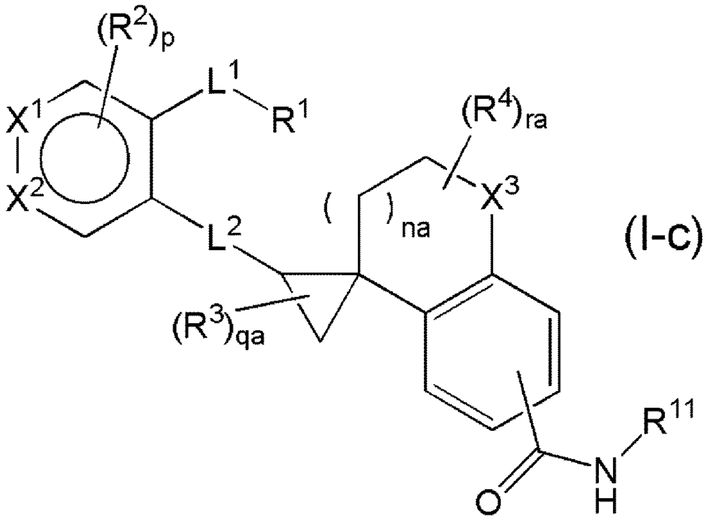

(I-c)

(wherein na represents an integer of 0 to 1, qa represents an integer of 0 to 3, ra represents an integer of 0 to 4, and other symbols are as defined above), still more preferably a compound or a salt thereof represented by general formula (I-d):

[Formula 7]

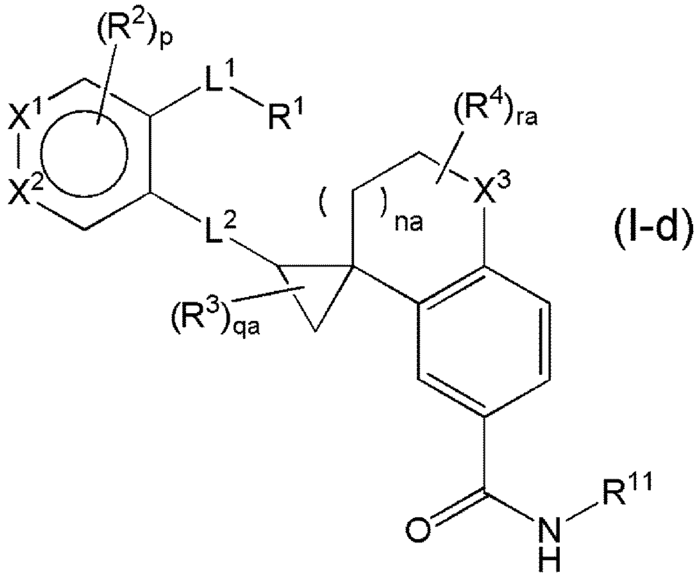

(I-d)

(wherein na represents an integer of 0 to 1, qa represents an integer of 0 to 3, ra represents an integer of 0 to 4, and other symbols are as defined above), furthermore preferably a compound or a salt thereof represented by general formula (I-e):

[Formula 8]

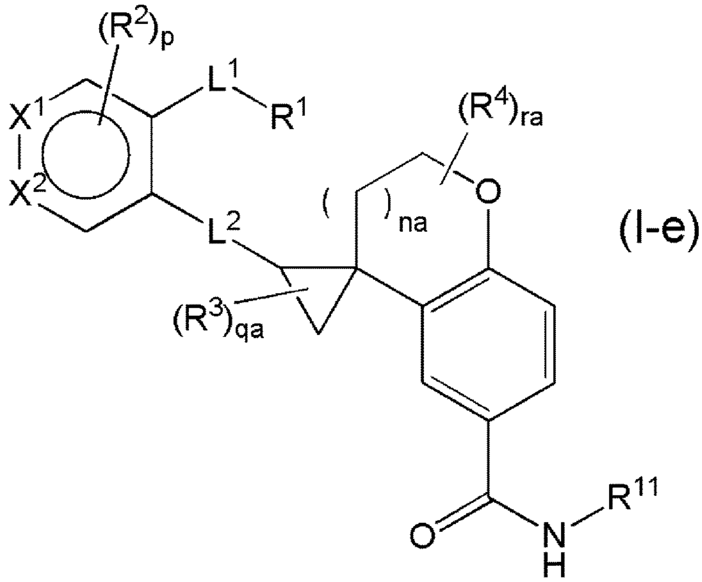

(I-e)

(wherein na represents an integer of 0 to 1, qa represents an integer of 0 to 3, ra represents an integer of 0 to 4, and other symbols are as defined above), particularly preferably a compound or a salt thereof represented by general formula (I-2):

[Formula 9]

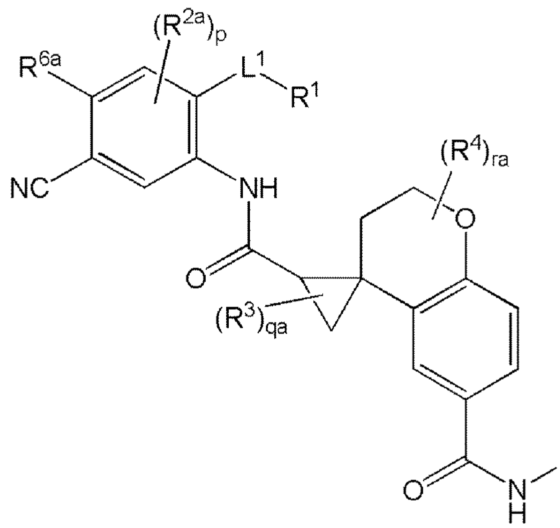

(I-2)

(wherein $R^{2a}$ represents halogen, $R^{6a}$ represents a hydrogen atom or halogen, qa represents an integer of 0 to 3, ra represents an integer of 0 to 4, and other symbols are as defined above), most preferably a compound or a salt thereof represented by general formula (I-4):

[Formula 10]

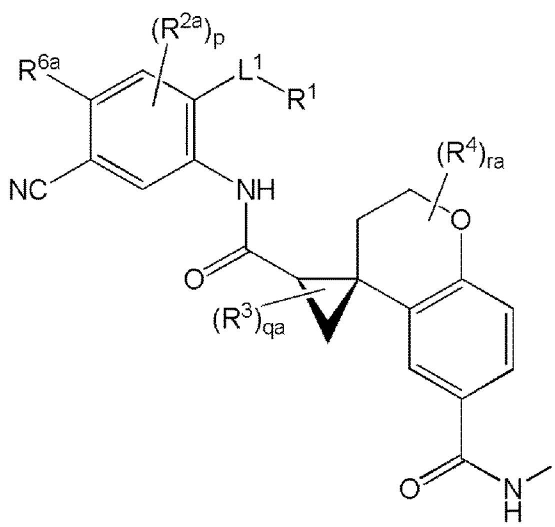

(I-4)

(wherein $R^{2a}$ represents halogen, $R^{6a}$ represents a hydrogen atom or halogen, qa represents an integer of 0 to 3, ra represents an integer of 0 to 4, and other symbols are as defined above).

In the present invention, still another embodiment of the compound represented by general formula (I) is a compound or a salt thereof represented by general formula (I-f):

[Formula 11]

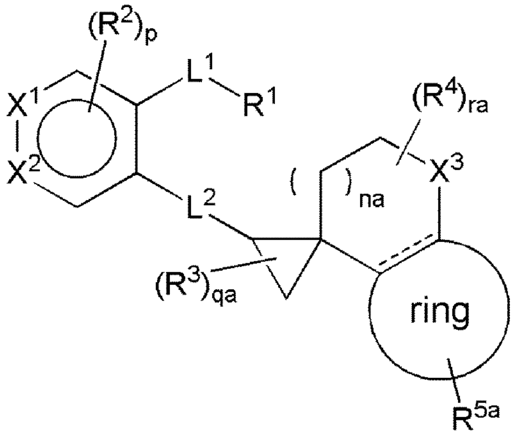

(I-f)

(wherein $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, na represents an integer of 0 to 1, qa represents an integer of 0 to 3, ra represents an integer of 0 to 4, and other symbols are as defined above), still more preferably a compound or a salt thereof represented by general formula (I-g):

[Formula 12]

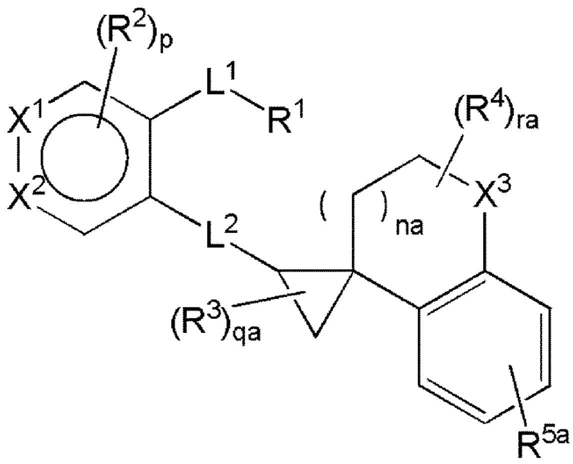

(I-g)

(wherein $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, na represents an integer of 0 to 1, qa represents an integer of 0 to 3, ra represents an integer of 0 to 4, and other symbols are as defined above), still more preferably a compound or a salt thereof represented by general formula (I-h):

[Formula 13]

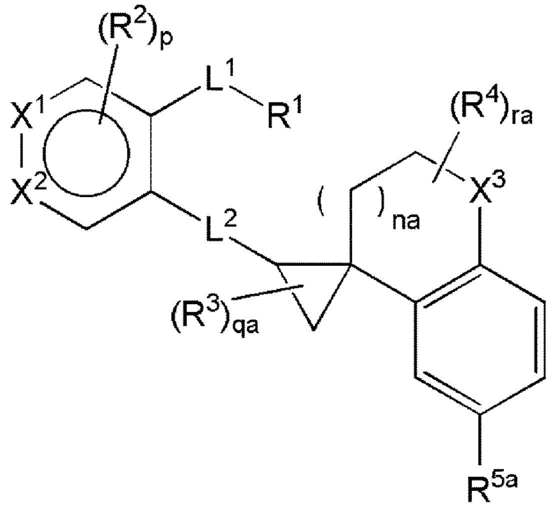

(I-h)

(wherein $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, na represents an integer of 0 to 1, qa represents an integer of 0 to 3, ra represents an integer of 0 to 4, and other symbols are as defined above), furthermore preferably a compound or a salt thereof represented by general formula (I-i):

[Formula 14]

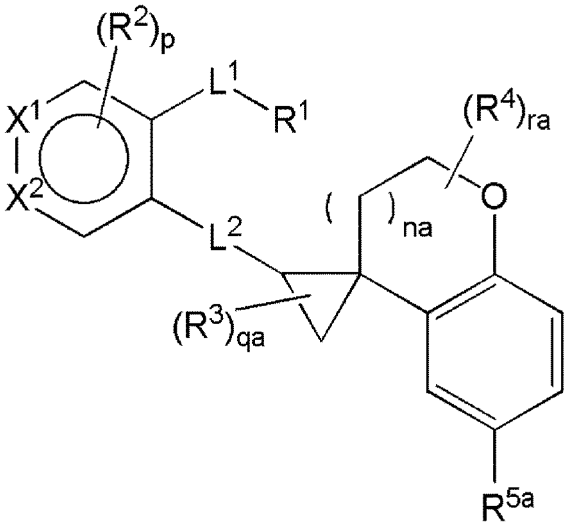

(I-i)

(wherein $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, na represents an integer of 0 to 1, qa represents an integer of 0 to 3, ra represents an integer of 0 to 4, and other symbols are as defined above), particularly preferably a compound or a salt thereof represented by general formula (I-3):

[Formula 15]

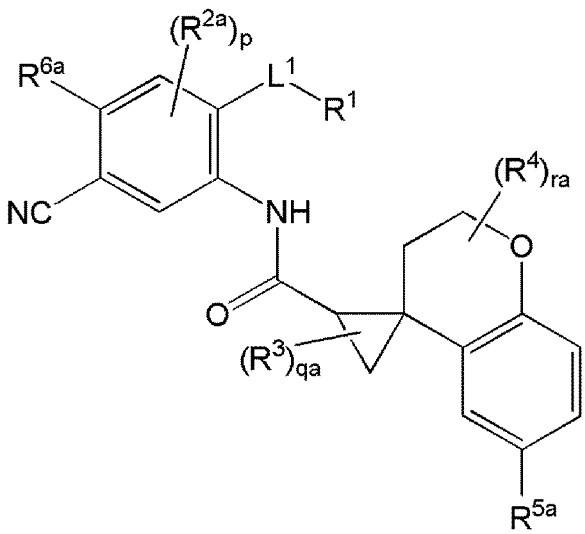

(I-3)

(wherein $R^{2a}$ represents halogen, $R^{6a}$ represents a hydrogen atom or halogen, $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, qa represents an integer of 0 to 3, ra represents an integer of 0 to 4, and other symbols are as defined above), most preferably a compound or a salt thereof represented by general formula (1-5):

[Formula 16]

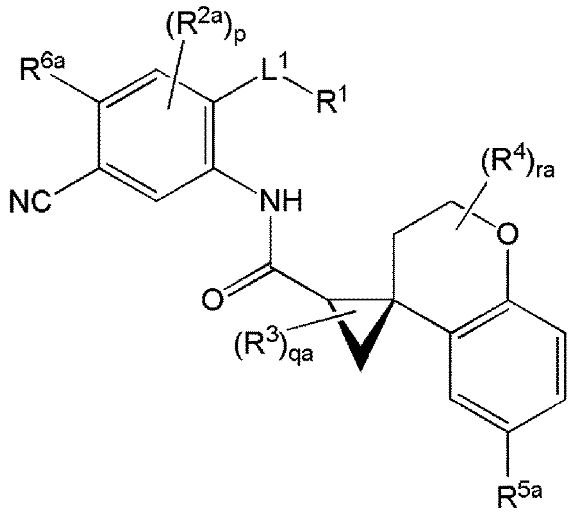

(I-5)

(wherein $R^{2a}$ represents halogen, $R^{6a}$ represents a hydrogen atom or halogen, $R^{5a}$ is a C4-10 carbon ring which may be substituted with one to three $R^{18}$, or a four- to ten-membered heterocyclic ring which may be substituted with one to three $R^{18}$, wherein, when a plurality of $R^{18}$ exists, the plurality of $R^{18}$ each independently may be the same or different, qa represents an integer of 0 to 3, ra represents an integer of 0 to 4, and other symbols are as defined above).

In the present invention, in a general formula selected from the group of general formula (I-a), general formula (I-b), general formula (I-c), general formula (I-d), general formula (I-e), general formula (I-f), general formula (I-g), general formula (I-h), general formula (I-i) and general formula (I-1), it is preferred that $L^1$ is propylene and $L^2$ is -CH = CH-, -NHCO-, -CONH-, $-NHSO_2-$ or $-SO_2NH-$. More preferably, $L^1$ is propylene and $L^2$ is - NHCO- or -CONH-. Still more preferably, $L^1$ is propylene and $L^2$ is -NHCO-.

In the present invention, in general formula selected from the group consisting of general formula (I-2), general formula (I-3), general formula (I-4) and general formula (I-5), it is preferred that $L^1$ is propylene.

In the present invention, the $EP_4$ antagonist is more preferably a compound or a salt thereof described in the

section "Examples" in WO 2016/111347. The $EP_4$ antagonist is still more preferably:

(1) 4-[4-cyano-2-({ [(2'R,4S)-6-(methylcarbamoyl)-2,3-dihydrospiro[chromene-4,1'-cyclopropan]-2'-yl]carbonyl}amino)phenyl]butanoicacid,
(2) 4-{4-cyano-2-[({(2'R,4S)-6-[(cyclopropylmethyl)carbamoyl]-2,3-dihydrospiro[chromene-4,1'-cyclopropan]-2'-yl}carbonyl)amino]phenyl}butanoic acid,
(3) 4-{4-cyano-2-[({(2'R,4S)-6-[(2-methoxyethyl)carbamoyl]-2,3-dihydrospiro[chromene-4,1'-cyclopropan]-2'-yl}carbonyl)amino]phenyl}butanoic acid,
(4) 4-{4-cyano-2-[({(2'R,4S)-6-[(2-methyl-2-propanyl)carbamoyl]-2,3-dihydrospiro[chromene-4,1'-cyclopropan]-2'-yl}carbonyl)amino]phenyl}butanoic acid,
(5) 4-[4-cyano-2-({[(2'R,4S)-6-{[(2S)-1-methoxy-2-propanyl]carbamoyl}-2,3-dihydrospiro[chromene-4,1'-cyclopropan]-2'-yl]carbonyl}amino)phenyl]butanoic acid,
(6) 4-{4-cyano-2-[({(2'R,4S)-6-[(1-methyl-1H-pyrazol-3-yl)carbamoyl]-2,3-dihydrospiro[chromene-4,1'-cyclopropan]-2'-yl}carbonyl)amino]phenyl}butanoic acid,
(7) 4-[4-cyano-2-({ [(2'R,4S)-6-(cyclopropylcarbamoyl)-2,3-dihydrospiro[chromene-4,1'-cyclopropan]-2'-yl]carbonyl}amino)phenyl]butanoic acid,
(8) 4-[4-cyano-2-({ [(2'R,4S)-6-(isopropylcarbamoyl)-2,3-dihydrospiro[chromene-4,1'-cyclopropan]-2'-yl]carbonyl}amino)phenyl]butanoic acid,
(9) 4-[4-cyano-2-({[(2'R,4S)-6-(cyclopentylcarbamoyl)-2,3-dihydrospiro[chromene-4,1'-cyclopropan]-2'-yl]carbonyl}amino)phenyl]butanoic acid,
(10) 4-{2-[({(2'R,4S)-6-[(2S)-2-butanylcarbamoyl]-2,3-dihydrospiro[chromene-4,1'-cyclopropan]-2'-yl}carbonyl)amino]-4-cyanophenyl}butanoic acid,
(11) 4-{4-cyano-2-[({(2'R,4S)-6-[(trans-4-hydroxycyclohexyl)carbamoyl]-2,3-dihydrospiro[chromene-4,1'-cyclopropan]-2'-yl}carbonyl)amino]phenyl}butanoic acid,
(12) 4-{4-cyano-2-[({(2'R,4S)-6-[(cis-4-hydroxycyclohexyl)carbamoyl]-2,3-dihydrospiro[chromene-4,1'-cyclopropan]-2'-yl}carbonyl)amino]phenyl}butanoic acid,
(13) 4-[4-cyano-2-({ [(2'R,4S)-6-(2-pyridinylcarbamoyl)-2,3-dihydrospiro[chromene-4,1'-cyclopropan]-2'-yl]carbonyl}amino)phenyl]butanoic acid,
(14) 4-[4-cyano-2-({ [(2'R,4S)-6-(3-pyridazinylcarbamoyl)-2,3-dihydrospiro[chromene-4,1'-cyclopropan]-2'-yl]carbonyl}amino)phenyl]butanoic acid,
(15) 4-[4-cyano-2-({ [(2'R,4S)-6-(cyclobutylcarbamoyl)-2,3-dihydrospiro[chromene-4,1'-cyclopropan]-2'-yl]carbonyl}amino)phenyl]butanoic acid,
(16) 4-[4-cyano-2-({[(2'R,4S)-6-{[1-(2-methyl-2-propanyl)-1H-pyrazol-4-yl]carbamoyl}-2,3-dihydrospiro[chromene-4,1'-cyclopropan]-2'-yl]carbonyl}amino)phenyl]butanoic acid,
(17) 4-[4-cyano-2-({ [(2'R,4S)-6-(tetrahydro-2H-pyran-4-ylcarbamoyl)-2,3-dihydrospiro[chromene-4,1'-cyclopropan]-2'-yl]carbonyl}amino)phenyl]butanoic acid,
(18) 4-[4-cyano-2-({ [(2'R,4S)-6-(propylcarbamoyl)-2,3-dihydrospiro[chromene-4,1'-cyclopropan]-2'-yl]carbonyl}amino)phenyl]butanoic acid,
(19) 4-{4-cyano-2-[({(2'R,4S)-6-[(2-ethoxyethyl)carbamoyl]-2,3-dihydrospiro[chromene-4,1'-cyclopropan]-2'-yl}carbonyl)amino]phenyl}butanoic acid,
(20) 4-[4-cyano-2-({[(2'R,4S)-6-(ethylcarbamoyl)-2,3-dihydrospiro[chromene-4,1'-cyclopropan]-2'-yl]carbonyl}amino)phenyl]butanoic acid,
(21) 4-[4-cyano-2-({ [(1R,2R)-6'-(methylcarbamoyl)-2',3'-dihydrospiro[cyclopropane-1,1'-inden]-2-yl]carbonyl}amino)phenyl]butanoic acid,
(22) 4-{4-cyano-2-[({(lR,2R)-6'-[(2-methoxyethyl)carbamoyl]-2',3'-dihydrospiro[cyclopropane-1,1 '-inden]-2-yl}carbonyl)amino]phenyl}butanoic acid,
(23) 4-{4-cyano-2-[({(1R,2R)-6'-[(1-methyl-1H-pyrazol-4-yl)carbamoyl]-2',3'-dihydrospiro[cyclopropane-1,1'-inden]-2-yl}carbonyl)amino]phenyl}butanoic acid,
(24) 4-[4-cyano-2-({ [(2'R,4S)-7-fluoro-6-(methylcarbamoyl)-2,3-dihydrospiro[chromene-4,1'-cyclopropan]-2'-yl]carbonyl}amino)phenyl]butanoic acid,
(25) 4-{4-cyano-2-[({(2'R,4S)-7-fluoro-6-[(2-methoxyethyl)carbamoyl]-2,3-dihydrospiro[chromene-4,1'-cyclopropan]-2'-yl}carbonyl)amino]phenyl}butanoic acid,
(26) 4-[4-cyano-2-({[(2'R,4S)-7-fluoro-6-(isopropylcarbamoyl)-2,3-dihydrospiro[chromene-4,1'-cyclopropan]-2'-yl]carbonyl}amino)phenyl]butanoic acid,
(27) 4-[4-cyano-2-({ [(2'R,4S)-7-(methylcarbamoyl)-2,3-dihydrospiro[chromene-4,1'-cyclopropan]-2'-yl]carbonyl}amino)phenyl]butanoic acid,
(28) 4-{4-cyano-2-[({(2'R,4S)-7-[(2-methoxyethyl)carbamoyl]-2,3-dihydrospiro[chromene-4,1'-cyclopropan]-2'-yl}carbonyl)amino]phenyl}butanoic acid,

(29) 4-[4-cyano-2-({ [(2'R,4S)-7-methoxy-6-(methylcarbamoyl)-2,3-dihydrospiro[chromene-4,1'-cyclopropan]-2'-yl]carbonyl}amino)phenyl]butanoic acid,
(30) 4-{4-cyano-2-[({(2'R,4S)-7-methoxy-6-[(2-methoxyethyl)carbamoyl]-2,3-dihydrospiro[chromene-4,1'-cyclopropan]-2'-yl}carbonyl)amino]phenyl}butanoic acid,
(31) 4-[4-cyano-2-({[(2'R,3S)-5-(methylcarbamoyl)spiro[1-benzofuran-3,1'-cyclopropan]-2'-yl]carbonyl}amino)phenyl]butanoic acid,
(32) 4-{4-cyano-2-[({(2'R,3S)-5-[(2-methoxyethyl)carbamoyl]spiro[1-benzofuran-3,1'-cyclopropan]-2'-yl}carbonyl)amino]phenyl}butanoic acid,
(33) 4-[4-cyano-2-({[(1S,2R)-6'-[(2-methoxyethyl)carbamoyl]-3',3'-dimethyl-2',3'-dihydrospiro[cyclopropane-1,1'-inden]-2-yl]carbonyl}amino)phenyl]butanoic acid, and
(34) 4-[4-cyano-2-({ [(1S,2R)-3',3'-dimethyl-6'-(methylcarbamoyl)-2',3'-dihydrospiro[cyclopropane-1,1'-inden]-2-yl]carbonyl}amino)phenyl]butanoic acid.

In the present invention, the $EP_4$ antagonist is preferably:

(1) 4-[4-cyano-2-({ [(2'R,4S)-6-(5-methyl-1,3,4-oxadiazol-2-yl)-2,3-dihydrospiro[chromene-4,1'-cyclopropan]-2'-yl]carbonyl}amino)phenyl]butanoic acid,
(2) 4-[4-cyano-2-({ [(2'R,4S)-6-(5-cyclopropyl-1,3,4-oxadiazol-2-yl)-2,3-dihydrospiro[chromene-4,1'-cyclopropan]-2'-yl]carbonyl}amino)phenyl]butanoic acid,
(3) 4-[4-cyano-2-({ [(2'R,4S)-6-(3-methyl-1,2,4-oxadiazol-5-yl)-2,3-dihydrospiro[chromene-4,1'-cyclopropan]-2'-yl]carbonyl}amino)phenyl]butanoic acid,
(4) 4-[4-cyano-2-({[(2'R,4S)-6-(3-pyridinyl)-2,3-dihydrospiro[chromene-4,1'-cyclopropan]-2'-yl]carbonyl}amino)phenyl]butanoic acid,
(5) 4-[4-cyano-2-({ [(2'R,4S)-6-(1H-pyrazol-1-yl)-2,3-dihydrospiro[chromene-4,1'-cyclopropan]-2'-yl]carbonyl}amino)phenyl]butanoic acid,
(6) 4-[4-cyano-2-({[(2'R,4S)-6-(1H-pyrazol-5-yl)-2,3-dihydrospiro[chromene-4,1'-cyclopropan]-2'-yl]carbonyl}amino)phenyl]butanoic acid,
(7) 4-[4-cyano-2-({[(2'R,4S)-6-(4-pyridazinyl)-2,3-dihydrospiro[chromene-4,1'-cyclopropan]-2'-yl]carbonyl}amino)phenyl]butanoic acid,
(8) 4-[4-cyano-2-({[(2'R,4S)-6-(2-oxo-1-pyrrolidinyl)-2,3-dihydrospiro[chromene-4,1'-cyclopropan]-2'-yl]carbonyl}amino)phenyl]butanoic acid,
(9) 4-[4-cyano-2-({[(2'R,4S)-6-(6-methoxy-3-pyridinyl)-2,3-dihydrospiro[chromene-4,1'-cyclopropan]-2'-yl]carbonyl}amino)phenyl]butanoic acid,
(10) 4-{4-cyano-2-[({(2'R,4S)-6-[6-(1H-pyrazol-1-yl)-3-pyridinyl]-2,3-dihydrospiro[chromene-4,1'-cyclopropan]-2'-yl}carbonyl)amino]phenyl}butanoic acid,
(11) 4-{4-cyano-2-[({(2'R,4S)-6-[6-(dimethylamino)-3-pyridinyl]-2,3-dihydrospiro[chromene-4,1'-cyclopropan]-2'-yl}carbonyl)amino]phenyl}butanoic acid,
(12) 4-[4-cyano-2-({ [(2'R,4S)-6-(6-methyl-3-pyridinyl)-2,3-dihydrospiro[chromene-4,1'-cyclopropan]-2'-yl]carbonyl}amino)phenyl]butanoic acid,
(13) 4-{4-cyano-2-[({(2'R,4S)-6-[6-(methylamino)-3-pyridinyl]-2,3-dihydrospiro[chromene-4,1'-cyclopropan]-2'-yl}carbonyl)amino]phenyl}butanoic acid,
(14) 4-[4-cyano-2-({[(2'R,4S)-6-(2-pyridinyl)-2,3-dihydrospiro[chromene-4,1'-cyclopropan]-2'-yl]carbonyl}amino)phenyl]butanoic acid,
(15) 4-[4-cyano-2-({ [(2'R,4S)-6-(1,3-thiazol-2-yl)-2,3-dihydrospiro[chromene-4,1'-cyclopropan]-2'-yl]carbonyl}amino)phenyl]butanoic acid,
(16) 4-[4-cyano-2-({ [(2'R,4S)-6-(1,3-oxazol-2-yl)-2,3-dihydrospiro[chromene-4,1'-cyclopropan]-2'-yl]carbonyl}amino)phenyl]butanoic acid,
(17) 4-[4-cyano-2-({[(2'R,4S)-6-(1-methyl-1H-1,2,3-triazol-4-yl)-2,3-dihydrospiro[chromene-4,1'-cyclopropan]-2'-yl]carbonyl}amino)phenyl]butanoic acid,
(18) 4-[4-cyano-2-({[(2'R,4S)-6-(3-pyridazinyl)-2,3-dihydrospiro[chromene-4,1'-cyclopropan]-2'-yl]carbonyl}amino)phenyl]butanoic acid,
(19) 4-[4-cyano-2-({[(2'R,3S)-5-(3-pyridinyl)spiro[1-benzofuran-3,1'-cyclopropan]-2'-yl]carbonyl}amino)phenyl]butanoic acid, and
(20) 4-[4-cyano-2-({[(1S,2R)-3',3'-dimethyl-6'-(3-pyridinyl)-2',3'-dihydrospiro[cyclopropane-1,1'-inden]-2-yl]carbonyl}amino)phenyl]butanoic acid.

Particularly preferably, the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid (also abbreviated as "compound

A", hereinafter) or a salt thereof represented by the following structural formula:

[Formula 17]

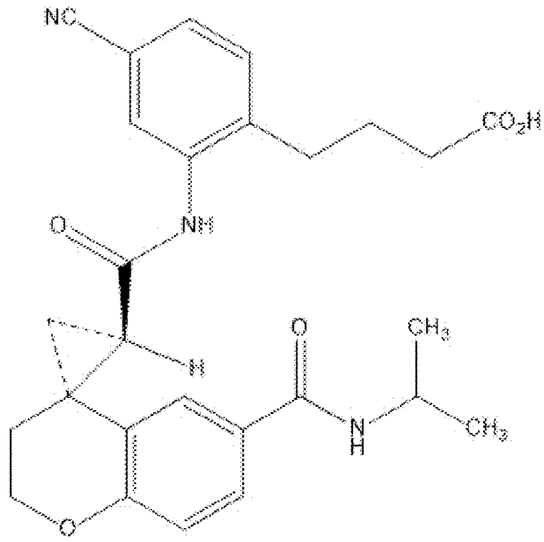

4-[4-Cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid is also named as 4-[4-cyano-2-({[(2'R,4S)-6-(isopropylcarbamoyl)-2,3-dihydrospiro[chromene-4,1'-cyclopropane]-2'-yl]carbonyl}amino)phenyl]butanoic acid. Compound A can be produced according to a known method, for example, a method described in Example 2-13 in WO 2016/111347.

Particularly preferably, the $EP_4$ antagonist is 4-{4-cyano-2-[({(2'R,4S)-6-[(2-methoxyethyl)carbamoyl]-2,3-dihydrospiro[chromene-4,1'-cyclopropan]-2'-yl}carbonyl)amino]phenyl}butanoic acid (also abbreviated as "compound B", hereinafter) or a salt thereof represented by the following structural formula:

[Formula 18]

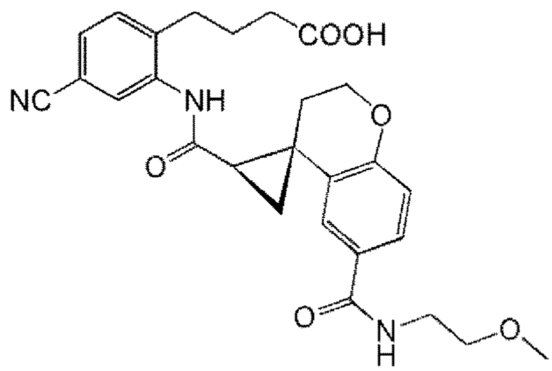

Compound B can be produced according to a known method, for example, a method described in Example 2-2 in WO 2016/111347.

It is to be understood that all isomeric forms of the compounds fall within the scope of the present invention, unless otherwise specifically stated. For example, the alkyl, alkoxy, and alkylene include linear and branched alkyl, alkoxy, and alkylene. The present invention also includes all of the following: isomers due to a double bond, a ring, and a fused ring (E, Z, cis, and trans isomers), isomers due to the presence of an asymmetric carbon (R and S isomers, α and β isomers, enantiomers, diastereomers), optical isomers involving optical rotation (D, L, d, 1 isomers), polar compounds separated by chromatography (high-polarity, and low-polarity compounds), equilibrium compounds, rotational isomers, mixtures of any proportions of these compounds, and racemic mixtures. The present invention also includes all isomers due to tautomerism.

As is clear for a skilled person, the following symbols as used herein have the following meaning, unless otherwise specifically stated.

[Formula 19]

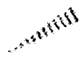

represents a bond into the plane of the paper (i.e., the α configuration).

[Formula 20]

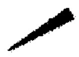

represents a bond out of the plane of the paper (i.e., the β configuration).

[Formula 21]

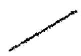

represents a mix of α and β configurations.

[Salts]

The compound represented by general formula (I) is converted into a salt using a known method.
The salt is preferably a pharmaceutically acceptable salt.
Preferably, the salt is water soluble.
Examples of the pharmaceutically acceptable salt include acid addition salts, alkali metal salts, alkali-earth metal salts, ammonium salts, and amine salts.
Examples of the pharmaceutically acceptable salt include acid addition salts, alkali metal salts, alkali-earth metal salts, ammonium salts, and amine salts.
The acid addition salts may be inorganic acid salts, for example, such as hydrochloride, hydrobromate, hydroiodide, sulfates, phosphates, and nitrates, or organic acid salts, for example, such as acetates, lactates, tartrates, benzoates, citrates, methanesulfonate, ethanesulfonate, trifluoroacetate, benzenesulfonate, toluenesulfonate, isethionates, glucuronates, and gluconates. Examples of the alkali metal salts include potassium salts and sodium salts.
Examples of the alkaline earth metal salts include calcium salts and magnesium salts.
An example of the ammonium salts is tetramethylammonium salts.
Examples of the amine salts include triethylamine salts, methylamine salts, dimethylamine salts, cyclopentylamine salts, benzylamine salts, phenethylamine salts, piperidine salts, monoethanolamine salts, diethanolamine salts, tris(hydroxymethyl)aminomethane salts, lysine salts, arginine salts and N-methyl-D-glucamine salts.
The compound of the present invention may be transformed into an N-oxide using any method. As used herein, N-oxide refers to compounds of general formula (I) with oxidized nitrogen atoms.
The compound represented by general formula (I) or a salt thereof may be present in a non-solvated form or a form solvated with a pharmaceutically acceptable solvent such as water and ethanol. The solvate is preferably a hydrate.
The compound represented by the general formula (I) can form co-crystals with a suitable co-crystal forming agent. The co-crystal forming agent is preferably one that is pharmaceutically acceptable. A co-crystal is typically defined as a crystal in which two or more different molecules are formed by intermolecular interactions that differ from ionic bonds. Further, the co-crystal may be a complex of a neutral molecule and a salt. The co-crystal can be adjusted by known methods, such as by melt crystallization, by recrystallization from a solvent, or by physically pulverizing a component together. Suitable co-crystal forming agent includes those described in WO 2006/007448 A.
In the present invention, all references to the compounds represented by general formula (I) include the compounds represented by general formula (I), or salts thereof, N-oxides thereof, solvates (e.g., hydrate) thereof or co-crystals thereof, orN-oxides, solvates (e.g., hydrates) or co-crystals of salts of the compounds represented by general formula (I).
In the present invention, the compounds or salts thereof represented by general formula (I)include the compounds represented by general formula (I), or salts thereof, N-oxides thereof, solvates (e.g., hydrates) thereof or co-crystals thereof, or an N-oxides, solvates (e.g., hydrates) or co-crystals of salts of the compounds represented by general formula (I).

[Prodrug]

The compound represented by the general formula (I) can be administered in the form of a prodrug. As used herein, a prodrug of the compound represented by general formula (I) refers to a compound that is transformed into the compound of general formula (I) in the body through reaction with, for example, an enzyme, and stomach acid. The following are examples of prodrugs of the compounds represented by general formula (I): A compound of general formula (I) with an amino group that is acylated, alkylated, or phosphorylated (for example, a compound of general formula (I) with an amino group that is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, acetoxymethylated, or tert-butylated); a compound of general formula (I) with a hydroxyl group that is acylated, alkylated, phosphorylated, or borated (for example, a compound of general formula (I) with a hydroxyl group that is acetylated, palmitoylated, propanoylated, pivaloylated, succinylated, fumarylated, alanylated, or dimethylaminomethylcarbonylated; and a compound of general formula (I) with a carboxy group that is esterificated or amidated (for example, a compound of general formula (I) with a carboxy group that is ethylesterificated, phenylesterificated, carboxymethylesterificated, dimethylaminomethylesterificated, pivaloyloxymethylesterificated, 1-{ (ethoxycarbonyl)oxy} ethylesterificated, phthalidylesterificated, (5-methyl-2-oxo-1,3-dioxolen-4-yl)methylesterificated, 1-{ [(cyclohexyloxy)carbonyl]oxy}ethylesterificated, or methylamidated). These compounds may be produced by a method known per se. The prodrug of the compounds represented by general formula (I) may be a hydrate or a nonhydrate. The prodrug of the compounds represented by general formula (I) may be one that transforms into the compound of general formula (I) under physiological conditions, such as described in Development of Drugs, Vol. 7, Molecular Design, pp. 163 - 198, 1990, Hirokawa Publishing Company.

Furthermore, each of atoms constituting the compound represented by general formula (I) may be substituted by an isotope thereof (e.g., $^{2}H$, $^{3}H$, $^{13}C$, $^{14}C$, $^{15}N$, $^{16}N$, $^{17}O$, $^{18}O$, $^{18}F$, $^{35}S$, $^{36}Cl$, $^{77}Br$, $^{125}I$) or the like.

[Method for producing compound of present invention]

In the present invention, the $EP_4$ antagonist can be produced by a known method, for example, and the compound represented by the general formula (I) can be produced according to the method described in WO 2016/111347.

[Preparation and Administration Method]

In the present invention, the $EP_4$ antagonist is generally formulated into a preparation together with various additives or pharmaceutically acceptable excipients such as solvents and is administered as oral or parenteral preparation systemically or locally. The term "pharmaceutically acceptable carrier" as used herein refers to a substance which is materials except active substances which are generally used for preparations. The pharmaceutically acceptable excipients are preferably excipients which are harmlessness, and do not show any pharmacological effect and inhibit treatment effect of the active substances at the dosage of the drug products. In addition, the pharmaceutically acceptable excipients can be used to enhance effectiveness of the active substances, make production of the drugs easy, stabilize quality and improve usability. Specifically, the material described in "*Iyakuhintenkabutujiten*" (yakujinippousha, 2016), (edited by nihonniyakuhinntennkazai kyokai)", etc. may be selected according to intentions.

Dosage forms for administration includes, for example, oral preparation (e.g.: tablets, capsules, granules, powders, oral solutions, syrups, oral jelly agents, etc.), oro-mucosal preparation (e.g.: tablets for oro-mucosal application, sprays for oro-mucosal application, semi-solid preparations for oro-mucosal application, gargles, etc.), preparations for injection (e.g.: injections, etc.), preparations for dialysis (e.g.: dialysis agents, etc.), preparation for inhalation (e.g.: inhalations, etc.), preparation for ophthalmic application (e.g.: ophthalmic liquids and solutions, ophthalmic ointments, etc.), preparation for otic application (e.g.: ear preparation, etc.), preparations for nasal application (nasal preparations, etc.), preparation for recta (e.g.: suppositories, semi-solid preparations for rectal application, enemas for rectal application, etc.), preparations for vaginal application (e.g.: tablets for vaginal use, suppositories for vaginal use, etc.) and preparation for cutaneous application (e.g.: solid preparations for cutaneous application, liquids and solutions for cutaneous application, sprays, ointment, creams, gels, patches, etc.).

A dose of the $EP_4$ antagonist of the present invention may vary depending on an age, a weight, symptom, a therapeutic effect, an administration method, a treatment time, and the like. However, the compound of the present invention or a concomitant drug of the compound of the present invention and another drug is administered orally, usually, in a range of 1 ng to 1000 mg per once per adult, once to several times a day, or is administered parenterally, in a range of 0.1 ng to 100 mg per once per adult, once to several times a day, or continuously administered intravenously, in a range of 1 to 24 hours a day. As described above, since the dose varies depending on various conditions, a dose smaller

than the above dose may be sufficient, or administration beyond the range may be necessary.

As one aspect, when Compound A is used, Compound A is administered orally at a dose of about 1 to 100 mg per administration one to three times per day, preferably at a dose of 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg or 100 mg per administration one to three times per day, still more preferably at a dose of 1 mg, 5 mg, 10 mg, 15 mg, 20 mg, 25 mg, 30 mg, 35 mg, 40 mg, 45 mg or 50 mg per administration one to three times per day, still more preferably at a dose of 5 mg, 10 mg, 20 mg or 40 mg per administration once per day, furthermore preferably at a dose of 20 mg or 40 mg per administration once per day.

(2) Immune checkpoint inhibitors

In the present invention, the term "immune checkpoint molecule" refers to a molecule that exerts an immunosuppressive function by transmitting a suppressive co-signal. As the immune checkpoint molecule, the followings are known: CTLA-4, PD-1, PD-L1 (programmed cell death-ligand 1), PD-L2 (programmed cell death-ligand 2), LAG-3 (Lymphocyte activation gene 3), TIM3 (T cell immunoglobulin and mucin -3), BTLA (B and T lymphocyte attenuator), B7H3, B7H4, CD 160, CD 39, CD 73, A2aR (adenosine A2a receptor), KIR (killer inhibitory receptor), VISTA (V-domain Ig-containing suppressor of T cell activation), IDO1 (Indoleamine 2,3-dioxygenase), ArginaseI, TIGIT (T cell immunoglobulin and ITIM domain), CD 115, and the like (See Nature Reviews Cancer, 12, p. 252-264, 2012; Cancer Cell, 27, p.450-461, 2015). However, the immune checkpoint molecule is not particularly limited as long as they are molecules having a function conforming to the definition.

In the present invention, the immune checkpoint inhibitor is a substance that inhibits the function of an immune checkpoint molecule. The immune checkpoint inhibitor is not particularly limited as long as it is a substance capable of suppressing the function (signal) of the immune checkpoint molecule.

Examples of the immune checkpoint inhibitor include an anti-PD-1 antibody (e.g., Nivolumab, Cemiplimab (REGN-2810), Pembrolizumab (MK-3475), Spartalizumab (PDR-001), Tislelizumab (BGB-A317), AMP-514 (MEDI0680), Dostarlimab (ANB011/TSR-042), Toripalimab (JS001), Camrelizumab (SHR-1210), Genolimzumab (CBT-501), Sintilimab (IBI308), STI-A1110, ENUM 388 D4, ENUM 244C8, GLS010, Retifanlimab (MGA012), Balstilimab (AGEN2034), CS1003, Serplulimab (HLX10), BAT-1306, AK105, AK103, BI 754091, LZM009, CMAB819, Sym021, Geptanolimab(GB226), SSI-361, JY034, HX008, ISU106, Budigalimab(ABBV181), Prolgolimab(BCD-100), Sasanlimab(PF-06801591), CX-188, Cetrelimab(JNJ-63723283), Zimberelimab(AB122) and the like), an anti-PD-L1 antibody (e.g., Atezolizumab(RG7446/MPDL3280A), Avelumab(PF-06834635/MSB0010718C), Durvalumab(MEDI4736), BMS-936559, STI-1014, Envafolimab(KN035), Lodapolimab(LY3300054), HLX20, SHR-1316, CS1001(WBP3155), MSB2311, BGB-A333, KL-A167, CK-301, AK106, AK104, ZKAB001, FAZ053, CBT-502(TQB2450), JS003, CX-072 and the like) or an anti-CTLA-4 antibody (e.g., Ipilimumab (MDX-010), Zalifrelimab (AGEN1884), Tremelimumab, and the like), an anti-PD-L2 antibody, a PD-L1 fusion protein, a PD-L2 fusion protein (e.g., AMP-224), an anti-Tim-3 antibody (e.g., MBG453), an anti-LAG-3 antibody (e.g., BMS-986016, LAG525), an anti-KIR antibody (e.g., Lirilumab), and the like. An antibody containing a heavy chain and light chain complementarity determining region (CDRs) or a variable region (VR) of the known antibody is also an embodiment of the immune checkpoint inhibitor. For example, a further embodiment of an anti-PD-1 antibody includes, for example, an antibody including heavy and light chain complementarity-determining regions (CDRs) or variable regions (VR) of Nivolumab.

In the present invention, the immune checkpoint inhibitor is preferably an anti-PD-1 antibody, an anti-PD-L1 antibody, or an anti-CTLA-4 antibody, and more preferably an anti-PD-1 antibody or an anti-PD-L1 antibody. The anti-PD-1 antibody is preferably Nivolumab, Cemiplimab, Pembrolizumab, Spartalizumab, Tislelizumab, Toripalimab, Sintilimab, and Camrelizumab, and the anti-PD-L1 antibody is preferably Atezolizumab, Avelumab, Durvalumab, and BMS-936559, and the anti-CTLA-4 antibody is preferably Ipilimumab and Tremelimumab. Furthermore, the anti-PD-1 antibody is more preferably Nivolumab, Cemiplimab, and Pembrolizumab, and still more preferably Nivolumab. In the present invention, the immune checkpoint inhibitor is preferably an anti-PD-1 antibody, and more preferably Nivolumab.

In the present invention, the immune checkpoint inhibitor can be produced by a known method. Nivolumab can be produced according to the method described in WO 2006/121168, Pembrolizumab can be produced according to the method described in WO 2008/156712, BMS-936559 can be produced according to the method described in WO 2007/005874, and Ipilimumab can be produced according to the method described in WO 2001/014424.

In the present invention, any one or any plurality of these immune checkpoint inhibitors can be used in combination with the administration of an $EP_4$ antagonist and a standard therapy.

The dose of the immune checkpoint inhibitor used for the combination of the present invention varies with age, body weight, symptom, therapeutic effect, route of administration, duration of treatment, and the like but is adjusted in a manner that the optimal desired effects are obtained.

For example, the immune checkpoint inhibitor can be administered as an active ingredient intravenously (e.g., intravenous drip infusion) at a dose of about 1 to10 mg/kg (body weight) per administration or at a dose of about 200 to

1200 mg per administration over about 30 to 60 minutes or over about 60 minutes or longer at 2- to 4-week intervals. In this regard, the dose per administration per body weight is, for example, 1 mg/kg, 2 mg/kg, 3 mg/kg, 4 mg/kg, 5 mg/kg, 6 mg/kg, 7 mg/kg, 8 mg/kg, 9 mg/kg or 10 mg/kg, and the dose per administration is, for example, 200 mg, 240 mg, 250 mg, 280 mg, 300 mg, 320 mg, 350 mg, 360 mg, 400 mg, 420 mg, 450 mg, 480 mg, 500 mg, 540 mg, 560 mg, 600 mg, 640 mg, 700 mg, 720 mg, 750 mg, 800 mg, 840 mg, 900 mg, 1000 mg, 1080 mg, 1100 mg, 1120 mg or 1200 mg. Examples of the intervals of the administration include 2-week intervals, 3-week intervals, or 4-week intervals, and the dosing time for a single administration is, for example, about 30 minutes, about 60 minutes, or about 60 minutes or longer.

When the immune checkpoint inhibitor is Nivolumab which is an anti-PD-1 antibody, the administration can be performed by the following administration and dosages. For a malignant melanoma patient, Nivolumab is administered by intravenous drip infusion at a dose of 3 mg/kg (body weight) per administration at 2-week intervals or at a dose of 2 mg/kg (body weight) per administration at 3-week intervals, or is administered by intravenous drip infusion at a dose of 240 mg per administration at 2-week intervals or at a dose of 480 mg per administration at 4-week intervals. For a patient with non-small cell lung cancer, renal cell cancer, classical Hodgkin lymphoma, head and neck cancer, stomach cancer and malignant pleural mesothelioma, Nivolumab is administered by intravenous drip infusion at a dose of 3 mg/kg (body weight) per administration at 2-week intervals. As another administration and dosage, for a patient with malignant melanoma, non-small cell lung cancer, renal cell cancer, urothelial cancer, MSI-H or dMMR-positive colorectal cancer (Also included are pediatric patients over the age of 12), stomach cancer, hepatocellular cancer, small cell lung cancer and malignant pleural mesothelioma, Nivolumab is administered by intravenous drip infusion at a dose of 240 mg per administration at 2-week intervals or at a dose of 480 mg per administration at 4-week intervals. As still another administration and dosage, for a malignant melanoma patient, Nivolumab is administered in combination with Ipilimumab, wherein it may be possible that Nivolumab is administered by an intravenous drip infusion four times at a dose of 1 mg/kg (body weight) per administration at 3-week intervals, and is then administered by an intravenous drip infusion at a dose of 3 mg/kg (body weight) per administration at 2-week intervals or administered four times by an intravenous drip infusion at a dose of 80 mg per administration at 3-week intervals, and then is administered by an intravenous drip infusion at a dose of 240 mg per administration at 2-week intervals or at a dose of 480 mg per administration at 4-week intervals. For example for the administration to a renal cell cancer patient or a colorectal cancer patient in combination with Ipilimumab, it may be possible that Nivolumab is administered four times by an intravenous drip infusion at a dose of 240 mg per administration at 3-week intervals and is then administered by an intravenous drip infusion at a dose of 240 mg per administration at 2-week intervals or at a dose of 480 mg per administration at 4-week intervals.

In addition, when Pembrolizumab, which is also an anti-PD-1 antibody, is to be administered, the administration can be performed by the following administration and dosage. That is, for a patient with malignant melanoma, non-small cell lung cancer, classical Hodgkin's lymphoma, head and neck cancer, MSI-H or dMMR-positive solid cancer or colorectal cancer, urothelial cancer, cervical cancer, primary mediastinal B-cell lymphoma, hepatocellular carcinoma, stomach cancer and Merkel cell cancer, Pembrolizumab is administered by an intravenous drip infusion at a dose of 200 mg per administration at 3-week intervals or at a dose of 400 mg per administration at 6-week intervals. As another administration and dosage, for example, for a patient with classical Hodgkin lymphoma, MSI-H or dMMR-positive solid cancer or colorectal cancer and primary mediastinal B-cell lymphoma of a child of 2 years or older, Pembrolizumab is administered by an intravenous drip infusion at a dose of 2 mg/kg (body weight) per administration (up to 200 mg per administration) at 3-week intervals.

When the immune checkpoint inhibitor is Avelumab which is an anti-PD-L1 antibody, for a patient with Merkel cell cancer and urothelial cancer, Avelumab is administered by an intravenous drip infusion at a dose of 10 mg/kg (body weight) per administration at 2-week intervals. When Atezolizumab, which is also a PD-L1 antibody, is to be administered, for a patient with non-small cell lung cancer, urothelial cancer and hepatocellular carcinoma, Atezolizumab is administered by an intravenous drip infusion at a dose of 1200 mg per administration at 3-week intervals. For a patient with triple negative breast cancer, Atezolizumab is administered in combination with paclitaxel, wherein Atezolizumab is administered by an intravenous drip infusion at a dose of 840 mg per administration at 2-week intervals. When Durvalumab, which is also a PD-L1 antibody, is to be administered, for a patient with non-small cell lung cancer and urothelial cancer, Durvalumab is administered by an intravenous drip infusion at a dose of 10 mg/kg (body weight) per administration at 2-week intervals. For a patient with advanced small cell lung cancer, Durvalumab is administered by an intravenous drip infusion at a dose of 1500 mg per administration at 4-week intervals.

When Ipilimumab, which is an anti-CTLA-4 antibody, is to be administered, for a patient with malignant melanoma, Ipilimumab is administered alone or in combination with Nivolumab, wherein Ipilimumab is administered by an intravenous drip infusion four times at a frequency of once a day at a dose of 3 mg/kg (body weight) per administration at 3-week intervals. For a patient with renal cell cancer and MSI-H or dMMR-positive colorectal cancer, Ipilimumab is administered in combination with Nivolumab, wherein Ipilimumab is administered by an intravenous drip infusion four times at a frequency of once a day at a dose of 1 mg/kg (body weight) per administration at 3-week intervals. For a patient with non-small cell lung cancer, Ipilimumab is administered by an intravenous drip infusion at a dose of 1 mg/kg (body weight) per administration at 6-week intervals.

The dosage form of the immune checkpoint inhibitor in the present invention is preferably parenteral administration including subcutaneous administration, intradermal administration, intraperitoneal administration, intramuscular administration, and intravenous administration. Among these, subcutaneous administration or intravenous administration is preferred. Intravenous administration is more preferred. As the dosage form for intravenous administration, intravenous drip infusion is preferred.

In the present invention, the dosage and administration can also be employed in the therapeutic method of the present invention.

(3) Combination of XELOX plus Bevacizumab therapy, $EP_4$ antagonists and immune checkpoint inhibitor (also abbreviated as "combination-1", hereinafter)

The term "XELOX therapy" as used herein refers to a method for treating cancer using the combination of capecitabine and oxaliplatin (L-OHP).

The term "XELOX plus Bevacizumab therapy" as used herein refers to a method for treating cancer using the combination of three components, i.e., Capecitabine, Oxaliplatin and Bevacizumab. In one embodiment, the XELOX plus Bevacizumab therapy is a therapy in which Bevacizumab is administered intravenously at a dose of 7.5 mg/kg over about 30 to 90 minutes and Oxaliplatin is administered intravenously at a dose of 130 mg/$m^2$ (body surface area) over 2 hours, the series of the administrations are performed at 3-week intervals, Capecitabine is administered orally at a frequency of twice a day at a dose of 1000 mg/$m^2$/administration (body surface area of less than 1.36 $m^2$: 1200 mg/administration, body surface area of 1.36 to less than 1.66 $m^2$: 1500 mg/administration, body surface area of 1.66 to less than 1.96 $m^2$: 1800 mg/administration, body surface area of 1.96$m^2$ or more: 2100 mg/administration) for 14 days, followed by rest for 7 days. In one embodiment, in any one of the second and subsequent cycles of administration in the XELOX plus Bevacizumab therapy, the administration of Bevacizumab may be discontinued depending on the degree of onset of adverse side effects in a patient, and the dose of capecitabine may be reduced to 1800 mg/dose, 1500 mg/dose, 1200 mg/dose, 900 mg/dose, or 600 mg/dose or discontinue and the administration of Oxaliplatin may be reduced to 100 mg/$m^2$ (body surface area) or 85 mg/$m^2$ (body surface area) or discontinued depending on the dosage at the start of the administration and a body surface area of a patient. The XELOX plus Bevacizumab therapy can be closed, reduced, and resumed in accordance with the judgment of a physician with reference to the latest package insert.

In one embodiment, Compound A, which is an $EP_4$ antagonist to be used in combination with a XELOX plus Bevacizumab therapy, is administered at a dose of 5 mg orally once a day, at a dose of 10 mg orally once a day, at a dose of 20 mg orally once a day, or at a dose of 40 mg orally once a day. Preferably, Compound A is administered at a dose of 20 mg orally once a day or at a dose of 40 mg orally once a day. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A is administered as an $EP_4$ antagonist at a starting dose of 40 mg, the one-step reduction amount is 20 mg and the two-step reduction amount is 10 mg. When Compound A is administered as an $EP_4$ antagonist at a starting dose of 20 mg, the one-step reduction amount is 10 mg and the two-step reduction amount is 5 mg.

In one embodiment, Nivolumab to be used as an immune checkpoint inhibitor in combination with a XELOX plus Bevacizumab therapy is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 360 mg per administration at 3-week intervals. The administration of Nivolumab itself may be discontinued depending on the degree of the onset of adverse side effects in the patient. When the criteria for resuming are satisfied, the administration of Nivolumab can be resumed.

The type of cancer to which the combination-1 can be applied is not particularly limited as long as the combination-1 can exhibit the effect thereof on the cancer. In one embodiment, the cancer is colorectal cancer, and preferably colonic/rectal cancer. More preferably, the cancer is curatively unresectable advanced or recurrent colonic/rectal cancer.

In one embodiment, the combination-1 is administered to a patient who has not been treated for colorectal cancer.

With respect to the combination-1, in one embodiment, when the $EP_4$ antagonist (preferably Compound A) and the immune checkpoint inhibitor (preferably an anti-PD-1 antibody (preferably Nivolumab)) and a XELOX plus Bevacizumab therapy are administered on the same day, the $EP_4$ antagonist and the immune checkpoint inhibitor are administered first. In one embodiment, Bevacizumab, Oxaliplatin and Capecitabine are administered in this order after the administration of the $EP_4$ antagonist and immune checkpoint inhibitor. In another embodiment, when the $EP_4$ antagonist (preferably Compound A) and the immune checkpoint inhibitor (preferably an anti-PD-1 antibody (preferably Nivolumab)) and the XELOX plus Bevacizumab therapy are administered on the same day, the XELOX plus Bevacizumab therapy

may be performed first, or simultaneously with the administration of the $EP_4$ antagonist and the immune checkpoint inhibitor. In one embodiment, the order of administration of the $EP_4$ antagonist, the immune checkpoint inhibitor, Bevacizumab, Oxaliplatin and Capecitabine may any one unless otherwise defined, and two or more of them may be administered simultaneously.

(4) $EP_4$ antagonist or combination of $EP_4$ antagonist and immune checkpoint inhibitor (hereinafter, abrreviated as "combination-2") as preoperative adjuvant therapy after preoperative chemoradiotherapy

The term "preoperative chemoradiotherapy" as used herein refers to a therapy in which the irradiation with radioactive ray and a chemotherapy with Fluorouracil (5-FU) or Capecitabine are combined. In the present invention, a combination of the irradiation with a radioactive ray and the administration of Capecitabine is preferred.

In one embodiment, the preoperative chemoradiotherapy to be employed in the present invention is 45 Gy/25 times of irradiation to the pelvic cavity and 5.4 Gy/3 times of boost irradiation to the primary lesion, and 825 mg/$m^2$ of Capecitabine (the starting dose is based on the usage and dosage of Xeloda (registered trademark) tablet 300) is administered twice a day, and capecitabine is orally administered in a period corresponding to 75% of 28 times of irradiation of radiation in combination with radiation (for example, in the case of 50.4 Gy/28 of irradiation, oral administration for 21 days or 42 times or more as oral administration in the morning and evening), but the dose can be appropriately reduced depending on the degree of expression of adverse side effects of the patient.

In one embodiment, Compound A that is an $EP_4$ antagonist to be used as a preoperative adjuvant therapy after a preoperative chemoradiotherapy is administered orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. More preferably, Compound A is administered orally once a day at a dose of 40 mg. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A that is an $EP_4$ antagonist is administered at a starting dose of 40 mg, the one-step reduction amount is 20 mg and the two-step reduction amount is 10 mg.

In one embodiment, Nivolumab that is an immune checkpoint inhibitor to be used as a preoperative adjuvant therapy after a preoperative chemoradiotherapy is administered by intravenous drip infusion, at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 240 mg per administration at 2-week intervals. The administration of Nivolumab itself may be discontinued depending on the degree of the onset of adverse side effects in the patient. When the criteria for resuming are satisfied, the administration of Nivolumab can be resumed.

One embodiment of the combination-2 is the administration of an $EP_4$ antagonist, and one embodimentis the combined administration of an $EP_4$ antagonist and an immune checkpoint inhibitor.

The type of cancer to which the combination-2 is applied is not particularly limited as long as it is a cancer for which the present preoperative adjuvant therapy can exert the effect. In one embodiment, the cancer is colorectal cancer, and preferably colonic/rectal cancer. More preferably, it is locally advanced rectal cancer that is curatively resectable.

In one embodiment, the combination-2 is administered to a patient who has no distant metastasis on diagnostic imaging after the completion of preoperative chemoradiotherapy and can undergo curative resection.

In one embodiment, the combination-2 is administered to a patient who can undergo (curative) resection in the present preoperative adjuvant therapy.

(5) FFX therapy, or dose reduction regimen thereof

The term "FFX therapy" as used herein refers to a method for treating cancer using a combination of four agents of Oxaliplatin (L-OHP), Irinotecan hydrochloride hydrate (Irinotecan, CPT-11), levofolinate calcium (levofolinate, I-LV), and Fluorouracil (5-FU). As a recommended dosage, for example, Oxaliplatin is intravenously administered at a dose of 85 mg/$m^2$ (body surface area) over 2 hours, then Levofolinate is intravenously administered at a dose of 200 mg/$m^2$ over 2 hours, irinotecan is intravenously administered at a dose of 180 mg/$m^2$ over 1.5 hours from 30 minutes after the start of the administration of Levofolinate, Fluorouracil is rapidly intravenously administered at a dose of 400 mg/$m^2$ after the completion of the administration of Levofolinate, and Fluorouracil is intravenously administered at a dose of 2400 mg/$m^2$ over 46 hours, and the series of administrations is performed at 2-week intervals.

The "dose reduction regimen" of the FFX therapy is a prescription for reducing the dose of any one of the four agents administered in the FFX therapy from the first administration or discontinuing the administration itself, or reducing the dose in any one of the second and subsequent cycles or discontinuing the administration of any one of the four

agents depending on the degree of onset of adverse side effects observed in any one of the first and subsequent cycles. In that aspect, for example, rapid intravenous administration of Fluorouracil may not be carried out from the initial administration, the dosage of Oxaliplatin may be any dosage of 65 mg/$m^2$, 50 mg/$m^2$ or between 85 to 50 mg/$m^2$, the dosage of Irinotecan may be any dosage of 150 mg/$m^2$, 120 mg/$m^2$, 90 mg/$m^2$ or between 180 to 90 mg/$m^2$, and the dosage of Fluorouracil administered intravenously continuously may be any dosage of 1800 mg/$m^2$, 1200 mg/$m^2$ or between 2400 to 1200 mg/$m^2$.

As another aspect of the dose reduction regimen, in any one of the second and subsequent cycles of administration in the FFX therapy, the rapid intravenous administration of Fluorouracil may be discontinued depending on the degree of onset of adverse side effects in a patient, the dose of Oxaliplatin may be reduced to any dose of 65 mg/$m^2$, 50 mg/$m^2$, or between 85 to 50 mg/$m^2$ or the administration of Oxaliplatin may be discontinued depending on the degree of onset of adverse side effects in a patient, the dose of Irinotecan may be reduced to any dose of 150 mg/$m^2$, 120 mg/$m^2$, 90 mg/$m^2$, or between 180 to 90 mg/$m^2$ or the administration of Irinotecan may be discontinued depending on the degree of development of adverse side effect of a patient, and the dose of Fluorouracil continuously administered intravenously may be changed depending on the degree of onset of adverse side effects in a patient, the dosage may be reduced to any dosage of 1800 mg/$m^2$, 1200 mg/$m^2$ or between 2400 to 1200 mg/$m^2$, or the administration of the Fluorouracil may be discontinued.

Another embodiment of the dose reduction regimen recognized as a modified FOLFIRINOX therapy (mFFX therapy) is a therapeutic method in which, as a recommended dosage, for example, Oxaliplatin is intravenously administered at a dose of 85 mg/$m^2$ (body surface area) over 2 hours, then Levofolinate is intravenously administered at a dose of 200 mg/$m^2$ over 2 hours, Irinotecan is intravenously administered at a dose of 150 mg/$m^2$ over 1.5 hours from 30 minutes after the start of the administration of Levofolinate, and Fluorouracil is intravenously administered at a dose of 2400 mg/$m^2$ over 46 hours after the completion of the administration of Levofolinate, wherein the series of the administrations are performed at 2-week intervals.

As another dose reduction regimen of the mFFX therapy, for example, the dose of Oxaliplatin may be any dose of 65 mg/$m^2$, 50 mg/$m^2$ or between 85 to 50 mg/$m^2$, the dose of Irinotecan may be any dose of 140 mg/$m^2$, 120 mg/$m^2$ or between 140 to 120 mg/$m^2$, and the dose of Fluorouracil to be intravenously administered continuously may be any dose of 2400 mg/$m^2$, 1800 mg/$m^2$, 1200 mg/$m^2$ or between 2400 to 1200 mg/$m^2$, starting from the first dose.

In the administration in any of the second and subsequent cycles in the mFFX therapy, the dose of Oxaliplatin may be reduced to any dose of 65 mg/$m^2$, 50 mg/$m^2$, or between 85 to 50 mg/$m^2$ or the administration of Oxaliplatin may be discontinued depending on the degree of onset of adverse side effects in a patient, the dose of Irinotecan may be reduced to any dose of 120 mg/$m^2$, 90 mg/$m^2$, or between 150 to 90 mg/$m^2$ or the administration of Irinotecan may be discontinued depending on the degree of onset of adverse side effects in a patient, and the dose of Fluorouracil continuously administered intravenously may be reduced to any dose of 1800 mg/$m^2$, 1200 mg/$m^2$, or between 2400 to 1200 mg/$m^2$ or the administration of the Fluorouracil may be discontinued depending on the degree of onset of adverse side effects in a patient.

The interval of the series of the administrations in the FFX therapy or a dose reduction regimen thereof (e.g., mFFX therapy) may be set to three-week intervals or four-week intervals temporarily or continuously depending on the degree of the onset of adverse side effects in a patient. The withdrawal, dose reduction, and resuming of the FFX therapy or a dose reduction regimen thereof (e.g., mFFX therapy) are performed by the judgment of a physician with reference to the latest package insert.

(6) Combination with FFX therapy or dose reduction regimen thereof (hereinafter, also abbreviated as " combination-3")

In one embodiment, the $EP_4$ antagonist to be used in combination with a FFX therapy or a dose reduction regimen thereof (e.g., mFFX therapy) is administered in the dosage regimen set forth in the section "(1) $EP_4$ antagonist". In one embodiment, Compound A that is an $EP_4$ antagonist is administered orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. More preferably, Compound A is orally administered at a dose of 20 mg once a day. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A is administered as an $EP_4$ antagonist at a starting dose of 40 mg, the one-step reduction amount is 20 mg and the two-step reduction amount is 10 mg. When Compound A is administered as an $EP_4$ antagonist at a starting dose of 20 mg, the one-step reduction amount is 10 mg and the two-step reduction amount is 5 mg.

In one embodiment, the immune checkpoint inhibitor in the combination-3 is administered in the dosage and administration described in the section "(2) Immune checkpoint inhibitor". In one embodiment, Nivolumab that is an

immune checkpoint inhibitor is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 480 mg per administration at 4-week intervals. The administration of Nivolumab itself may be discontinued depending on the degree of the onset of adverse side effects in the patient. When the criteria for resuming are satisfied, the administration of Nivolumab can be resumed.

In one embodiment, in the combination-3, when an $EP_4$ antagonist (preferably Compound A) and an immune checkpoint inhibitor (preferably an anti-PD-1 antibody (preferably Nivolumab)) and a FFX therapy or a dose reduction regimen thereof (e.g., mFFX therapy) are administered on the same day, the $EP_4$ antagonist and the immune checkpoint inhibitor are administered first. In one embodiment, the FFX therapy or a dose reduction regimen thereof (e.g., mFFX therapy) is performed after administration of the $EP_4$ antagonist and immune checkpoint inhibitor. As another embodiment, when an $EP_4$ antagonist (preferably Compound A) and an immune checkpoint inhibitor (preferably an anti-PD-1 antibody (preferably Nivolumab)) and an FFX therapy or a dose reduction regimen thereof (e.g., mFFX therapy) are administered on the same day, the FFX therapy or the dose reduction regimen thereof (e.g., mFFX therapy) may be performed first, or may be performed simultaneously with the administration of the $EP_4$ antagonist and the immune checkpoint inhibitor. In one embodiment, the order of administration of the $EP_4$ antagonist, immune checkpoint inhibitor, and the FFX therapy or a dose reduction regimen thereof (e.g., mFFX therapy) may be started with any agent unless otherwise defined, and two or more agents may be administered simultaneously.

(7) GnP therapy

The term "GnP therapy" as used herein refers to a therapy in which Gemcitabine and nab-Paclitaxel are combined. In one embodiment, the GnP therapy is a therapy in which, as a recommended dosage, for example, Gemcitabine is intravenously administered at a dose of 1000 $mg/m^2$ (body surface area) over 30 minutes, nab-Paclitaxel is intravenously administered at a dose of 125 $mg/m^2$ (body surface area) over 30 minutes, wherein the series of the administrations are performed at 1-week intervals, continued for 3 weeks, and then taken off for 1 week. In one embodiment, in the administration in any of the second and subsequent cycles in the GnP therapy, the amount of Gemcitabine may be reduced to 800 $mg/m^2$ or 600 $mg/m^2$, or the amount of nab-Paclitaxel may be reduced to 100 $mg/m^2$ or 75 $mg/m^2$, depending on the degree of the onset of adverse side effects in the patient. Withdrawal, dose reduction, and resuming of GnP therapy are performed by the judgment of a physician with reference to the latest package insert.

(8) Combination with GnP therapy (hereinafter, abbreviated as "combination-4")

In one embodiment in the combination-4, the $EP_4$ antagonist is administered in the dosage described in the section " (1) $EP_4$ antagonist". In one embodiment, Compound A that is an $EP_4$ antagonist is administered orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. More preferably, Compound A is administered orally once a day at a dose of 40 mg. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A that is an $EP_4$ antagonist is administered at a starting dose of 40 mg, the one-step reduction amount is 20 mg and the two-step reduction amount is 10 mg. When Compound A that is an $EP_4$ antagonist is administered at a starting dose of 20 mg, the one-step reduction amount is 10 mg and the two-step reduction amount is 5 mg.

In one embodiment, the immune checkpoint inhibitor in the combination-4 is administered in the dosage and administration described in the section "(2) Immune checkpoint inhibitor". In one embodiment, Nivolumab that is an immune checkpoint inhibitor is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 480 mg per administration at 4-week intervals. The administration of Nivolumab itself may be discontinued depending on the degree of the onset of adverse side effects in the patient. When the criteria for resuming are satisfied, the administration of Nivolumab can be resumed.

(9) DTX plus RAM Therapy

The term "Docetaxel-and-Ramucirumab therapy" as used herein refers to a method for treating cancer using a combination of Docetaxel and Ramucirumab. The recommended dose for the therapy is one in which 60 $mg/m^2$ (body

surface area) of Docetaxel is intravenously administered over 60 minutes or longer and 10 mg/kg of Ramucirumab is intravenously administered over 60 minutes, wherein the series of the administrations are performed at 3-week intervals. In one embodiment, in the administration in any of the second and subsequent cycles in the DTX plus RAM therapy, the amount of Docetaxel may be increased or decreased to 75 mg/m$^2$ or 50 mg/m$^2$, or the administration of Docetaxel may be discontinued, Ramucirumab may be reduced to 8 mg/kg or 6 mg/kg, or the administration of Ramucirumab may be discontinued, or the time for the administration of Ramucirumab may be shortened to 30 to 60 minutes, depending on the degree of onset of adverse side effects in a patient. The withdrawal, dose reduction, and resuming of DTX plus RAM therapy are performed by the judgment of a physician with reference to the latest package insert. In one embodiment, the administration of Docetaxel and Ramucirumab is started on the same day.

(10) Combination with DTX plus RAM therapy (hereinafter, abbreviated as "combination-5")

In one embodiment, the $EP_4$ antagonist in the combination-5 is administered in the dosage described in the section "(1) $EP_4$ antagonist". In one embodiment, Compound A that is an $EP_4$ antagonist is administered orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A is administered as an $EP_4$ antagonist at a starting dose of 40 mg, the one-step reduction amount is 20 mg and the two-step reduction amount is 10 mg. When Compound A is administered as an $EP_4$ antagonist at a starting dose of 20 mg, the one-step reduction amount is 10 mg and the two-step reduction amount is 5 mg.
In one embodiment, the immune checkpoint inhibitor in the combination-5 is administered in the dosage and administration described in the section "(2) Immune checkpoint inhibitor". In one embodiment, Nivolumab that is an immune checkpoint inhibitor is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 360 mg per administration at 3-week intervals. The administration of Nivolumab itself may be discontinued depending on the degree of the onset of adverse side effects in the patient. When the criteria for resuming are satisfied, the administration of Nivolumab can be resumed.
In one embodiment, the administration of DTX plus RAM therapy, the administration of the immune checkpoint inhibitor and the administration of $EP_4$ antagonist are started on the same day.
In one embodiment, in the combination-5, when an $EP_4$ antagonist (preferably Compound A) and an immune checkpoint inhibitor (preferably an anti-PD-1 antibody (preferably Nivolumab)) and DTX plus RAM therapy are administered on the same day, the $EP_4$ antagonist and the immune checkpoint inhibitor are administered first. In one embodiment, DTX plus RAM therapy is performed after administration of an $EP_4$ antagonist and an immune checkpoint inhibitor. As another embodiment, when an $EP_4$ antagonist (preferably Compound A) and an immune checkpoint inhibitor (preferably an anti-PD-1 antibody (preferably Nivolumab)) and DTX plus RAM therapy are administered on the same day, the DTX plus RAM therapy may be performed first, or may be performed simultaneously with the administration of the $EP_4$ antagonist and the immune checkpoint inhibitor. In one embodiment, the order of administration of the $EP_4$ antagonist, immune checkpoint inhibitor, Docetaxel, and Ramucirumab may be started from any agent unless otherwise defined, and two or more agents may be administered simultaneously.

(11) DTX therapy

The term "Docetaxel therapy" as used herein refers to a method for treating cancer using Docetaxel. In one embodiment, as a recommended dosage, for example, Docetaxel is intravenously administered at a dose of 60 mg/m$^2$ (body surface area) over 60 minutes or longer, and the administration is performed at 3-week intervals. In one embodiment, in the administration in any of the second and subsequent cycles in the DTX therapy, the Docetaxel may be increased or decreased to 75 mg/m$^2$ or 50 mg/m$^2$, or the administration of Docetaxel may be discontinued, depending on the condition of a patient. Withdrawal, dose reduction, and resuming of DTX therapy are performed by the judgment of a physician with reference to the latest package insert.

(12) Combination with DTX therapy (hereinafter, abbreviated as "combination-6")

In one embodiment, in this combination-6, the $EP_4$ antagonist is administered in the dosage and administration described in the section "(1) $EP_4$ antagonist". In one embodiment, Compound A that is an $EP_4$ antagonist is administered

orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A that is an $EP_4$ antagonist is administered at a starting dose of 40 mg, the one-step reduction amount is 20 mg and the two-step reduction amount is 10 mg. When Compound A that is an $EP_4$ antagonist is administered at a starting dose of 20 mg, the one-step reduction amount is 10 mg and the two-step reduction amount is 5 mg.

In one embodiment, the immune checkpoint inhibitor in the combination-6 is administered in the dosage and administration described in the section "(2) Immune checkpoint inhibitor". In one embodiment, Nivolumab that is an immune checkpoint inhibitor is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 360 mg per administration at 3-week intervals. The administration of Nivolumab itself may be discontinued depending on the degree of the onset of adverse side effects in the patient. When the criteria for resuming are satisfied, the administration of Nivolumab can be resumed.

In one embodiment, administration of DTX therapy, the administration of an immune checkpoint inhibitors and the administration of an $EP_4$ antagonists are started on the same day.

In one embodiment, in the combination-6, when an $EP_4$ antagonist (preferably Compound A) and an immune checkpoint inhibitor (preferably an anti-PD-1 antibody (preferably Nivolumab)) and DTX therapy are administered on the same day, the $EP_4$ antagonist and the immune checkpoint inhibitor are administered first. In one embodiment, a DTX therapy is performed after administration of an $EP_4$ antagonist and an immune checkpoint inhibitor. As another embodiment, when an $EP_4$ antagonist (preferably Compound A) and an immune checkpoint inhibitor (preferably an anti-PD-1 antibody (preferably Nivolumab)) and DTX therapy are administered on the same day, the DTX therapy may be performed first, or may be performed simultaneously with the administration of the $EP_4$ antagonist and the immune checkpoint inhibitor. In one embodiment, the order of administration of the $EP_4$ antagonist, immune checkpoint inhibitor and the Docetaxel may be started from any agent unless otherwise defined, and two or more agents may be administered simultaneously.

(13) RAM therapy

The term "Ramucirumab therapy (hereinafter, also abbreviated as "RAM therapy")" as used herein refers to a cancer therapy with Ramucirumab. In one embodiment, as a recommended dosage, for example, Ramucirumab is intravenously administered at a dose of 10 mg/kg over 60 minutes, and the series of the administrations are performed at 3-week intervals. In one embodiment, in the administration in any of the second and subsequent cycles in the RAM therapy, the amount of Ramucirumab may be reduced to 8 mg/kg or 6 mg/kg or the administration of Ramucirumab may be discontinued depending on the condition of a patient, and the time for the administration of Ramucirumab may be shortened to 30 to 60 minutes depending on the degree of adverse side effects in a patient.

(14) Combination with RAM therapy (hereinafter, abbreviated as "combination-7")

In one embodiment, in this combination use-7, the $EP_4$ antagonist is administered in the dosage described in the section "(1) $EP_4$ antagonist". In one embodiment, Compound A that is an $EP_4$ antagonist is administered orally at a dose of 5 mg once a day, at a dose of 10 mg once a day, at a dose of 20 mg once a day, or at a dose of 40 mg once a day. Preferably, Compound A is administered at a dose of 20 mg orally once a day or at a dose of 40 mg orally once a day. Depending on the degree of onset of adverse side effects in the patient, the dose of Compound A may be reduced, or the administration of Compound A itself may be discontinued. In addition, if the criteria for resuming are satisfied, the administration of Compound A can be resumed. In the case of resuming, the dose of Compound A can be reduced by step by step and the administration of Compound A can be resumed according to the judgment of a physician. In one embodiment, when Compound A that is an $EP_4$ antagonist is administered at a starting dose of 40 mg, the one-step reduction amount is 20 mg and the two-step reduction amount is 10 mg. When Compound A that is an $EP_4$ antagonist is administered at a starting dose of 20 mg, the one-step reduction amount is 10 mg and the two-step reduction amount is 5 mg.

In one embodiment, the immune checkpoint inhibitor in the combination-7 is administered in the dosage and administration described in the section "(2) Immune checkpoint inhibitor". In one embodiment, Nivolumab that is an immune checkpoint inhibitor is administered by intravenous drip infusion at a dose of 240 mg per administration at 2-week intervals, at a dose of 360 mg per administration at 3-week intervals, or at a dose of 480 mg per administration at 4-week intervals. Preferably, Nivolumab is administered by intravenous drip infusion at a dose of 360 mg per administration at 3-week intervals. The administration of Nivolumab itself may be discontinued depending on the degree of the onset of adverse side effects in the patient. When the criteria for resuming are satisfied, the administration of Nivolumab can be resumed.

In one embodiment, the administration of the RAM therapy, the administration of the immune checkpoint inhibitor, and the administration of the $EP_4$ antagonist are started on the same day.

In one embodiment, in the combination-7, when an $EP_4$ antagonist (preferably Compound A) and an immune checkpoint inhibitor (preferably an anti-PD-1 antibody (preferably Nivolumab)) and a RAM therapy are administered on the same day, the $EP_4$ antagonist and the immune checkpoint inhibitor are administered first. In one embodiment, the RAM therapy is performed after the $EP_4$ antagonist and immune checkpoint inhibitor are administered. As another embodiment, when the $EP_4$ antagonist (preferably Compound A) and the immune checkpoint inhibitor (preferably the anti-PD-1 antibody (preferably Nivolumab)) and the RAM therapy are administered on the same day, the RAM therapy may be performed first, or may be performed simultaneously with the administration of the $EP_4$ antagonist and the immune checkpoint inhibitor. In one embodiment, the order of administration of the $EP_4$ antagonist, the administration of the immune checkpoint inhibitor, and the administration of Ramucirumab may be started with any agent unless otherwise defined, and two or more agents may be administered simultaneously.

[Diseases and patients for application]

The disease to which the therapeutic method of the present invention can be applied is cancer.

More specific examples of the cancer include leukemia (for example, acute myelogenous leukemia, chronic myelogenous leukemia, acute lymphoblastic leukemia, and chronic lymphocytic leukemia)l, malignant lymphoma (Hodgkin lymphoma, non-Hodgkin lymphoma (for example, adult T-cell leukemia, follicular lymphoma, and diffuse large B-cell lymphoma)), multiple myeloma, myelodysplastic syndrome, head and neck cancer, esophageal cancer, esophageal adenocarcinoma, stomach cancer, esophagogastric junction cancer, duodenal cancer, colorectal cancer, colon cancer, rectal cancer, liver cancer (for example, hepatocellular carcinoma), gallbladder/bile duct cancer, biliary tract cancer, pancreatic cancer (for example, pancreatic ductal cancer, insulinoma, intraductal papillary mucinous tumor), thyroid cancer, lung cancer (for example, non-small cell lung cancer (for example, squamous non-small cell lung cancer, non-squamous non-small cell lung cancer), small cell lung cancer), breast cancer, ovarian cancer (for example, serous ovarian cancer), cervical cancer, corpus uteri cancer, endometrial cancer, vaginal cancer, vulvar cancer, renal cancer (for example, renal cell carcinoma), renal pelvis/ureter cancer, urothelial cancer (for example, bladder cancer and upper urinary tract cancer), penile cancer, prostate cancer, testicular tumor ((for example, germ cell tumors), osteosarcoma/soft tissue sarcoma, malignant bone tumor, skin cancer ((for example, uveal malignant melanoma, malignant melanoma, and Merkel cell carcinoma), thymoma, mesothelioma, malignant pleural mesothelioma, glioblastoma, blood cancer, cancer of unknown primary, and the like.

The disease to which the therapeutic method of the present invention is applied is preferably colorectal cancer, pancreatic cancer, or lung cancer, and more preferably colonic/rectal cancer which is curatively unresectable advanced or recurrent, rectal cancer which is curatively resectable locally advanced cancer, pancreatic cancer having distant metastasis, and lung cancer which has undergone a combination therapy including an anti-PD-1 antibody or an anti-PD-L1 antibody and a platinum preparation and has been found to be advanced or recurrent. In addition, the non-small cell lung cancer is more preferably a non-small cell lung cancer that has undergone combination therapy including a curatively unresectable advanced or recurrent colonic/rectal cancer, a locally advanced cancer that can be curatively resected, a pancreatic ductal cancer having distant metastasis, an anti-PD-1 antibody or an anti-PD-L1 antibody, and a platinum preparation, and has been found to have progressed or relapsed unfavorably. As used herein, the term "treatment" of cancer includes, for example, a treatment performed for (i) reducing the proliferation of tumor cells, (ii) reducing symptoms resulting from cancer, (iii) improving the quality of life of a cancer patient, (iv) reducing the dose of other anticancer drugs or cancer therapy adjuvants that have already been administered, and/or (v) prolonging the survival of a cancer patient, and the term "prevention of progression" of cancer means slowing the progression of cancer, stabilizing symptoms resulting from cancer, and reversing the progression of symptoms. In addition, the term "prevention of recurrence" of cancer means to prevent the recurrence of cancer in a patient whose cancer lesion has completely or substantially disappeared or removed by cancer treatment or resection surgery.

The therapeutic method of the present invention may be prescribed for the following cancer patients: (a) patients with an insufficient or insufficient therapeutic effect by an anticancer drug or patients with exacerbations after treatment by an anticancer drug; (b) patients with radically unresectable, metastatic, recurrent, refractory and/or distant metastatic cancer; (c) patients with cancer having a Tumor Proportion Score (abbreviated as "TPS" hereinafter) of 50% or more, 25% or more, 10% or more, 5% or more, or 1% or more; (d) patients with cancer having a Combined Positive Score (abbreviated as "CPS", hereinafter) of 20% or more, 10% or more, 5% or more, or 1% or more; (e) patients with cancer

having mismatch repair defects (abbreviated as "dMMR", hereinafter) and/or high frequency microsatellite instability (abbreviated as "MSI-H", hereinafter); and (f) patients with cancer having a high tumor mutation burden (abbreviated as "TMB", hereinafter). Meanwhile, the therapeutic method of the present invention may also be more sought for prescription to the following cancer patients: (g) patients who have not been treated with anti-cancer drugs; (h) patients with cancers whose TPS is less than 50%, 25%, 10%, 5% or 1%; (i) patients with cancers whose CPS is less than 20%, 10%, 5% or 1%; (j) patients with cancers that do not have dMMR and/or MSI-H or have infrequent microsatellite instability (abbreviated as "MSI-L", hereinafter); or (k) patients with cancers whose TMB is infrequent. In particular, cancer patients for whom the application of the therapeutic method of the present invention is required include (i) patients with curatively unresectable advanced or recurrent cancer, particularly colonic/rectal cancer, (ii) patients with curatively resectable locally advanced cancer, particularly rectal cancer, (iii) patients with cancer, particularly pancreatic cancer, who have not been treated with an anticancer drug and/or who have distant metastases, or (iv) patients with cancer, particularly lung cancer, who have received a combination therapy including an anti-PD-1 antibody or an anti-PD-L1 antibody and a platinum preparation and who have been found to progress or recur refractory.

In one embodiment, a patient with radically unresectable advanced or recurrent colonic/rectal cancer is mentioned. In one embodiment, a patient who has not been treated for colonic/rectal cancer is mentioned. Preferred is a patient who has not been treated with a systemic anti-malignant-tumor agent for colonic/rectal cancer. More preferred is a patient who has not been treated with a systemic anti-malignant-tumor agent for radically unresectable advanced or recurrent colonic/rectal cancer.

In one embodiment, a patient with rectal cancer which is a locally advanced, curatively resectable cancer is mentioned. In one embodiment, a patient who has not been treated for rectal cancer is mentioned.

In one embodiment, a patient with pancreatic cancer who has not been treated with an anti-cancer drug and/or has distant metastases is mentioned.

In one embodiment, a patient who has not been treated for pancreatic cancer is mentioned. Preferred is a patient who has not been treated with a systemic anti-malignant-tumor agent for pancreatic cancer. More preferred is a patient who has not been treated with a systemic anti-malignant-tumor agent for pancreatic cancer having distant metastasis.

In one embodiment, a patient with lung cancer who has received a combination therapy including an anti-PD-1 antibody or an anti-PD-L1 antibody and a platinum preparation, and who has experienced refractory progression or recurrence is mentioned.

In one embodiment, a patient identified as having non-small cell lung cancer is mentioned. Preferred is a patient with stage IV or recurrent non-small cell lung cancer. More preferred is a patient who has received a combination therapy including an anti-PD-1 antibody or an anti-PD-L1 antibody and a platinum preparation as a primary therapy, and who is identified as having progression or recurrence of non-response.

Among these patients, the therapeutic method of the present invention is expected to exert a maximum anti-tumor effect against a patient for which the effect of the treatment with an immune checkpoint inhibitor or an $EP_4$ receptor antagonist alone is insufficient. When the therapeutic method of the present invention is employed, it is possible to administer the drugs at reduced doses, and therefore it is expected that the occurrence of adverse side effects can be reduced.

In one aspect, the therapeutic method of the present invention exhibits a synergistic effect. In one aspect, the therapeutic method of the present invention exhibits a synergistic effect compared with a therapeutic effect achieved by an immune checkpoint inhibitor, an $EP_4$ receptor antagonist or a standard therapy alone or a therapeutic effect achieved by the combination of an immune checkpoint inhibitor and a standard therapy, the combination of an $EP_4$ receptor antagonist and a standard therapy or the combination of an immune checkpoint inhibitor and an $EP_4$ receptor antagonist.

In one aspect, the therapeutic method of the present invention reduces adverse side effects. As one aspect, the therapeutic method of the present invention reduces adverse side effects compared with a therapeutic effect achieved by an immune checkpoint inhibitor, an $EP_4$ receptor antagonist or a standard therapy alone or a therapeutic effect achieved by the combination of an immune checkpoint inhibitor and a standard therapy the combination of an EP4 receptor antagonist and a standard therapy, or the combination of an immune checkpoint inhibitor and an $EP_4$ receptor antagonist.

The timing of the administration of drugs used for the combination administration of the present invention may be simultaneously or separately, unless otherwise defined.

As one aspect, the therapeutic method of the present invention is also applicable to the treatment of metastatic cancer and the prevention of metastasis.

As one aspect, the therapeutic method of the present invention inhibits recurrence.

In the present invention, the term "treatment" means that the reduction in tumor size, the prevention (delay or stop) of tumor growth, the prevention (delay or stop) of tumor metastasis, the prevention (inhibition or delay) of recurrence, and the alleviation of at least one of one or more symptoms associated with cancer occurs.

In the present invention, the therapeutic method of the present invention may be used in combination with other

drugs (e.g., known anti-cancer therapies) for (1) the complementation and/or enhancement of therapeutic effects, (2) the improvement of kinetics and absorption, the reduction of dosage, and/or (3) the reduction of adverse side effects.

In the present description, the term "about" as used herein means that a numerical value may change below or above the displayed numerical value by within 10%. That is the term "about 30 minutes" means 30 minutes $\pm$ 5 minutes, and the term "about 60 minutes" means 60 minutes $\pm$ 5 minutes.

In the present description, the term "standard therapy" means a standard therapy recommended to be performed on a general patient with a certain condition based on scientific evidences, and one example thereof is a chemotherapy. In one aspect, the following therapies are included: (i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) Docetaxel-and-Ramucirumab therapy.

The present invention provides the following embodiments, for example.

[1] An agent for suppressing the progression of, suppressing the recurrence of and/or treating cancer containing an $EP_4$ antagonist, as an active ingredient, which is characterized by being administered in combination with a standard therapy (preferably (i) Bevacizumab-and-XELOX therapy, (iii) FOLFIRINOX therapy or a dose reduction regimen thereof, (iv) Gemcitabine-and-nab-Paclitaxel therapy, or (v) Docetaxel-and/or-Ramucirumab therapy, more preferably (i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) Docetaxel-and-Ramucirumab therapy, still more preferably (i) Bevacizumab-and-XELOX therapy, (iii) FOLFIRINOX therapy or a dose reduction regimen thereof, (iv) Gemcitabine-and-nab-Paclitaxel therapy, or (vi) Docetaxel-and-Ramucirumab therapy) and an administration of an immune checkpoint inhibitor.

[2] An agent for suppressing the progression of, suppressing the recurrence of and/or treating cancer containing an immune checkpoint inhibitor, as an active ingredient, which is characterized by being administered in combination with a standard therapy (preferably (i) Bevacizumab-and-XELOX therapy, (iii) FOLFIRINOX therapy or a dose reduction regimen thereof, (iv) Gemcitabine-and-nab-Paclitaxel therapy, or (v) Docetaxel-and/or-Ramucirumab therapy, more preferably (i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) Docetaxel-and-Ramucirumab therapy, still more preferably (i) Bevacizumab-and-XELOX therapy, (iii) FOLFIRINOX therapy or a dose reduction regimen thereof, (iv) Gemcitabine-and-nab-Paclitaxel therapy, or (vi) Docetaxel-and-Ramucirumab therapy) and an administration of an $EP_4$ antagonist.

[3] The agent according to item [1] or [2] above, wherein the $EP_4$ antagonist is a compound represented by general formula (I):

[Formula 22]

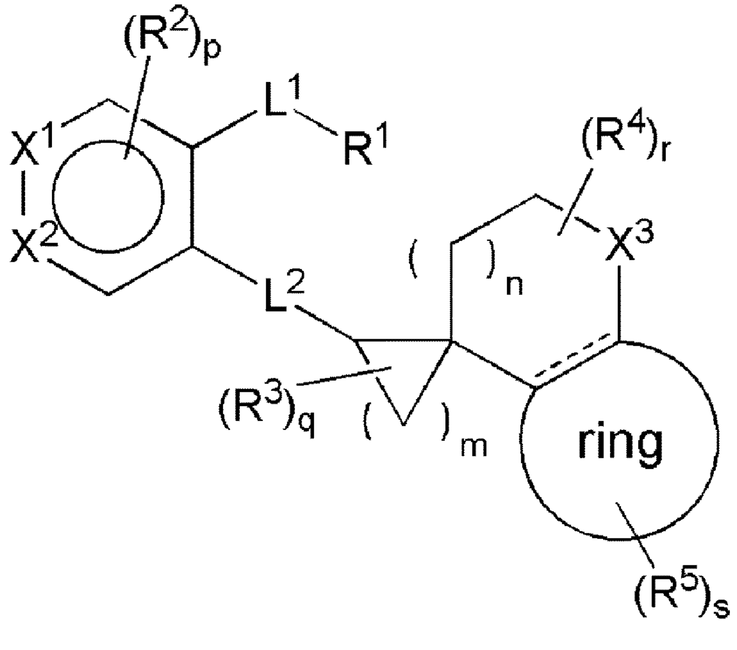

(I)

(wherein $R^1$ represents $COOR^8$, tetrazole, $SO_3H$, $SO_2NH_2$, $SO_2NHR^{8-1}$, $CONHS0_2R^{8-1}$, $SO_2NHCOR^{8-1}$, or hydroxamic acid,

wherein $R^8$ represents a hydrogen atom, C1-4 alkyl, or benzyl, and

$R^{8-1}$ represents C1-4 alkyl, C1-4 haloalkyl, a C3-10 carbon ring, or a three- to ten-membered heterocyclic ring, wherein the C3-10 carbon ring and the three- to ten-membered heterocyclic ring each may be substituted with C1-4 alkyl, C1-4 haloalkyl, C1-4 alkoxy, - O(C1-4 haloalkyl), C1-4 alkylthio, -S(C1-4 haloalkyl), halogen, or nitrile (here and below, "-CN"),

$L^1$ represents C1-5 alkylene, C2-5 alkenylene, or C2-5 alkynylene,

$R^2$ represents halogen, C1-4 alkyl, C1-4 alkoxy, C1-4 alkylthio, C2-4 alkenyl, C2-4 alkynyl, -O(Cl-4 haloalkyl), -S(C1-4 haloalkyl), -C(O)(Cl-4 alkyl), $-SO_2$(Cl-4 alkyl), - CONH(C1-4 alkyl), -CON(C1-4 alkyl)$_2$, -NHC(O)(C1-4 alkyl), -N(C1-4 alkyl)C(O)(C1-4 alkyl), $-NHSO_2$(Cl-4 alkyl), -N(C1-4 alkyl)$SO_2$(Cl-4 alkyl), $-SO_2NH$(Cl-4 alkyl), - $SO_2N$(Cl-4 alkyl)$_2$, $-NR^{17}R^{17}$, nitro, nitrile, a hydroxyl group, aldehyde (here and below, formyl), or carboxyl, wherein the C1-4 alkyl groups each may be substituted with halogen, and

the (C1-4 alkyl)$_2$ in $R^2$ represents two independent C1-4 alkyl groups which may be the same or different,

$X^1$ represents $CR^6$ or a nitrogen atom, wherein $R^6$ represents a hydrogen atom or $R^2$,

$X^2$ represents $CR^7$ or a nitrogen atom, wherein $R^7$ represents a hydrogen atom, $R^2$, or $-L^3-R^9$, wherein $L^3$ represents methylene, an oxygen atom, or a sulfur atom which may be oxidized, and $R^9$ represents a four- to ten-membered heterocyclic ring which may be substituted with a substituent selected from the group consisting of halogen, C1-4 alkyl, and C1-4 haloalkyl,

$L^2$ represents $-CH_2CH_2-$, -CH=CH-, $-CH_2O-$, $-OCH_2-$, $-CH_2S-$, $-SCH_2-$, $-CH_2S(O)-$, $-S(O)CH_2-$, $-CH_2SO_2-$, $-SO_2CH_2-$, $-CH_2NH-$, $-NHCH_2-$, -NHCO-, -CONH-, $-NHSO_2-$, or - $SO_2NH-$,

$R^3$ represents C1-4 alkyl or halogen,

$R^4$ represents halogen, C1-4 alkyl, or C1-4 haloalkyl,

$X^3$ represents methylene, an oxygen atom, a sulfur atom which may be oxidized, or $NR^{10}$, wherein $R^{10}$ represents C1-4 alkyl, -C(O)(C1-4 alkyl), -C(O)O(C1-4 alkyl), or - $SO_2$(Cl-4 alkyl), wherein the C1-4 alkyl groups each may be substituted with halogen,

the ring represents a benzene ring or a five- or six-membered monocyclic aromatic heterocyclic ring;

[Formula 23]

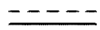

represents a single bond or a double bond;

$R^5$ represents (1) halogen, (2) C1-4 alkyl, (3) carboxyl, (4) nitrile, (5) $-CONHR^{11}$, (6) - $C(O)R^{12}$, (7) $-OR^{14}$, (8) $-S(O)_tR^{15}$, (9) $-CH_2R^{16}$, (10) $-NR^{17}R^{17}$, (11) $-NHCOR^{11}$, (12) a C4-10 carbon ring, or (13) a four- to ten-membered heterocyclic ring, wherein the C4-10 carbon ring or the four- to ten-membered heterocyclic ring may be substituted with one to three $R^{18}$, wherein, when a plurality of $R^{18}$ exists, the plurality of $R^{18}$ each independently may be the same or different,

$R^{11}$ represents C1-6 alkyl, C3-6 cycloalkyl, phenyl, or a four- to six-membered heterocyclic ring and may be substituted with one to three $R^{13}$, wherein, when a plurality of $R^{13}$ exists, the plurality of $R^{13}$ each independently may be the same or different, and

$R^{13}$ represents halogen, C1-6 alkyl, C3-6 cycloalkyl, C1-4 alkoxy, a hydroxyl group, $-NR^{20}R^{21}$, benzene, or a four- to six-membered heterocyclic ring,

wherein $R^{20}$ and $R^{21}$ each independently represent a hydrogen atom or C1-4 alkyl,

$R^{12}$ represents C1-6 alkyl, C3-6 cycloalkyl, benzene, or a four- to six-membered heterocyclic ring, wherein the C3-6 cycloalkyl, the benzene, and the four- to six-membered heterocyclic ring each independently may be substituted with halogen, C1-4 alkyl, or C1-4 alkoxy,

$R^{14}$ represents a hydrogen atom, C1-6 alkyl, C3-6 cycloalkyl, benzene, or benzyl, wherein the C1-6 alkyl may be substituted with one to three $R^{19}$, wherein, when a plurality of $R^{19}$ exists, the plurality of $R^{19}$ each independently may be the same or different, and

$R^{19}$ represents C1-4 alkoxy, -CONH(C1-4 alkyl), -CON(C1-4 alkyl)$_2$, or a five- or six-membered monocyclic aromatic heterocyclic ring which may be substituted with a substituent selected from the group consisting of C1-4 alkyl and C1-4 haloalkyl,

wherein the (C1-4 alkyl)$_2$ in $R^{19}$ represents two independent C1-4 alkyl groups which may be the same or different,

$R^{15}$ represents C1-6 alkyl, C3-6 cycloalkyl, benzene, or benzyl,

$R^{16}$ represents a hydroxyl group or C1-4 alkoxy,

each $R^{17}$ independently represents a hydrogen atom, C1-6 alkyl, or C3-6 cycloalkyl, and

$R^{18}$ represents halogen, C1-6 alkyl, C3-6 cycloalkyl, C1-4 alkoxy, oxo, nitrile, a hydroxyl group, hydroxymethyl, 1-methyl-1-hydroxyethyl, (C1-4 alkyl)$SO_2-$, a four- to six-membered heterocyclic ring, (C1-4 alkyl)NH-, or (C1-4

alkyl)$_2$N-,
wherein the (C1-4 alkyl)$_2$ in $R^{18}$ represents two independent C1-4 alkyl groups which may be the same or different,
m represents an integer of 1 to 4,
n represents an integer of 0 to 4,
p represents an integer of 0 to 2,
q represents an integer of 0 to 6,
r represents an integer of 0 to 6,
s represents an integer of 0 to 4,
t represents an integer of 0 to 2, and
$R^2$, $R^3$, $R^4$, and $R^5$ each independently may be the same or different when p, q, r, and s are each an integer of 2 or more) or a salt thereof.

[4] The agent according to any one of items [1] to [3] above, wherein the standard therapy is Bevacizumab-and-XELOX therapy.
[5] The agent according to any one of items [1] to [4] above, wherein the cancer is colorectal cancer.
[6] The agent according to item [5] above, wherein the colorectal cancer is colonic/rectal cancer.
[7] The agent according to item [6] above, wherein the colonic/rectal cancer is curatively unresectable advanced or recurrent colonic/rectal cancer (preferably, previously untreated curatively unresectable advanced or recurrent colonic/rectal cancer).
[8] The agent according to any one of items [1] to [7] above, wherein the $EP_4$ antagonist is a compound of represented by general formula (I-2):

[Formula 24]

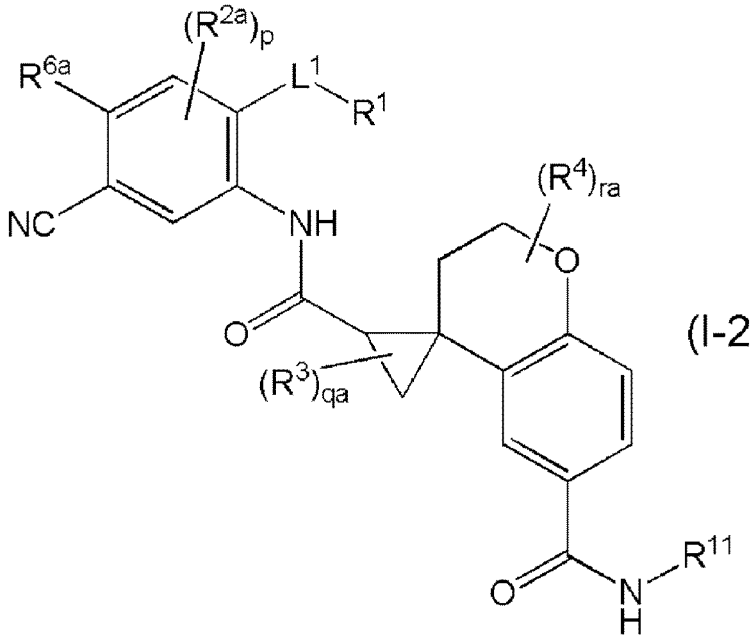

(I-2)

(wherein $R^{2a}$ represents halogen, $R^{6a}$ represents a hydrogen atom or halogen, qa represents an integer of 0 to 3, ra represents an integer of 0 to 4, and other symbols are as defined in claim 1) or a salt thereof.
[9] The agent according to any one of items [1] to [8] above, wherein the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof.
[10] The agent according to item [9] above, wherein the $EP_4$ antagonist is orally administered at a dose of 5 mg to 40 mg per administration once a day.
[11] The agent according to item [9] above, wherein the $EP_4$ antagonist is orally administered at a dose of 20 mg or 40 mg per administration once a day.
[12] The agent according to any one of items [1] to [11] above, wherein the immune checkpoint inhibitor is an anti-PD-1 antibody, an anti-PD-L1 antibody, or an anti-CTLA-4 antibody.

[13] The agent according to item [12] above, wherein the immune checkpoint inhibitor is an anti-PD-1 antibody.

[14] The agent according to item [13] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012 (Retifanlimab), AGEN2034 (Balstilimab), CS1003, HLX10 (Serplulimab), BAT-1306, AK105, AK103, BI 754091, LZM009, CMAB819, Sym021, GB226 (Geptanolimab), SSI-361, JY034, HX008, ISU106, ABBV181 (Budigalimab), BCD-100 (Prolgolimab), PF-06801591 (Sasanlimab), CX-188, JNJ-63723283 (Cetrelimab) or AB122 (Zimberelimab).

[15] The agent according to item [12] above, wherein the immune checkpoint inhibitor is an anti-PD-L1 antibody, and the anti-PD-L1 antibody is Atezolizumab, Avelumab, Durvalumab, BMS-936559, STI-1014, KN035 (Envafolimab), LY3300054 (Lodapolimab), HLX20, SHR-1316, CS1001, MSB2311, BGB-A333, KL-A167, CK-301, AK106, AK104, ZKAB001, FAZ053, CBT-502, JS003 or CX-072.

[16] The agent according to item [12] above, wherein the immune checkpoint inhibitor is an anti-CTLA-4 antibody, and the anti-CTLA-4 antibody is Ipilimumab, AGEN1884, or Tremelimumab.

[17] The agent according to item [13] above, wherein the anti-PD-1 antibody is Nivolumab.

[18] The agent according to item [13] above, wherein the anti-PD-1 antibody is Pembrolizumab.

[19] The agent according to item [13] above, wherein the anti-PD-1 antibody is Cemiplimab.

[20] The agent according to item [15] above, wherein the anti-PD-L1 antibody is Avelumab.

[21] The agent according to item [15] above, wherein the anti-PD-L1 antibody is Atezolizumab.

[22] The agent according to item [15] above, wherein the anti-PD-L1 antibody is Durvalumab.

[23] The agent according to item [16] above, wherein the anti-CTLA-4 antibody is Ipilimumab.

[24] The agent according to item [17] above, wherein Nivolumab is administered at a dose of 3 mg/kg (body weight) or 240 mg per administration at 2-week intervals, at a dose of 360 mg per administration at 3-week intervals, or at a dose of 480 mg per administration at 4-week intervals (preferably, 240 mg per administration at 2-week intervals, 360 mg per administration at 3-week intervals or 480 mg per administration at 4-week intervals).

[25] The agent according to item [17] above, wherein Nivolumab is intravenously administered at a dose of 360 mg per administration at 3-week intervals.

[26] The agent according to item [17] above, wherein Nivolumab is intravenously administered at a dose of 360 mg per administration over about 30 minutes at 3-week intervals.

[27] The agent according to item [18] above, wherein Pembrolizumab is administered at a dose of 2 mg/kg (body weight) per administration or 200 mg per administration at 3-week intervals or at a dose of 400 mg per administration at 6-week intervals.

[28] The agent according to item [19] above, wherein Cemiplimab is administered at a dose of 350 mg per administration at 3-week intervals.

[29] The agent according to item [20] above, wherein Avelumab is administered at a dose of 10 mg/kg (body weight) per administration at 2-week intervals.

[30] The agent according to item [21] above, wherein Atezolizumab is administered at a dose of 1200 mg per administration at 3-week intervals.

[31] The agent according to item [22] above, wherein Durvalumab is administered at a dose of 10 mg/kg (body weight) per administration at 2-week intervals.

[32] The agent according to item [23] above, wherein Ipilimumab is administered at a dose of 3 mg/kg (body weight) or 1 mg/kg (body weight) per administration intravenously four times at 3-week intervals.

[33] The agent according to any one of items [1] to [32] above, wherein the Bevacizumab-and-XELOX therapy includes:

(i) administering Bevacizumab at a dose of 7.5 mg/kg per administration at 3-week intervals;
(ii) administering Oxaliplatin at a dose of130 mg/$m^2$ at 3-week intervals; and
(iii) administering Capecitabine orally at a dose of 1000 mg/$m^2$ twice a day for 14 days, with a 7-day rest period.

[34] The agent according to any one of items [1] to [33] above, wherein the administration of Bevacizumab, the administration of Oxaliplatin and the administration of Capecitabine are started on the same day in the therapy.

[35] The agent according to any one of [1] to [34] above, wherein the administration of Bevacizumab, the administration of Oxaliplatin, the administration of Capecitabine, the administration of the immune checkpoint inhibitor, and the administration of the $EP_4$ antagonist are started on the same day in the therapy.

[36] An agent for suppressing the progression of, suppressing the recurrence of and/or treating cancer containing an $EP_4$ antagonist, as an active ingredient, which is characterized by being administered in combination with Bevacizumab-and-XELOX therapy and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg per administration (preferably 20 mg or 40 mg per administration);
(ii) the immune checkpoint inhibitor is Nivolumab, and Nivolumab is administered at a dose of 240 mg per administration at 2-week intervals, at a dose of 360 mg per administration at 3-week intervals, or at a dose of 480 mg per administration at 4-week intervals (preferably at a dose of 360 mg per administration at 3-week intervals), and
(iii) the Bevacizumab-and-XELOX therapy includes:

administering Bevacizumab at a dose of 7.5 mg/kg per administration at 3-week intervals;
administering Oxaliplatin at a dose of 130 $mg/m^2$ at 3-week intervals;
and administering Capecitabine at a dose of 1000 $mg/m^2$ twice a day for 14 days, with a 7-day rest period.

[37] An agent for suppressing the progression of, suppressing the recurrence of and/or treating cancer containing an immune checkpoint inhibitor, as an active ingredient, which is characterized by being administered in combination with Bevacizumab-and-XELOX therapy and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably at a dose of 360 mg per administration at 3-week intervals), and
(iii) the Bevacizumab-and-XELOX therapy includes: administering Bevacizumab at a dose of 7.5 mg/kg per administration at 3-week intervals; administering Oxaliplatin at a dose of 130 $mg/m^2$ at 3-week intervals;

and administering Capecitabine at a dose of 1000 $mg/m^2$ orally twice a day for 14 days, with a 7-day rest period.
[38] The agent according to item [36] or [37] above, wherein the administration of Bevacizumab, the administration of Oxaliplatin, the administration of Capecitabine, the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the administration of Nivolumab are started on the same day in the therapy.
[39] The agent according to any one of items [36] to [38] above, wherein the cancer is colorectal cancer.
[40] The agent according to item [39] above, wherein the colorectal cancer is colonic/rectal cancer.
[41] The agent according to item [40] above, wherein the colonic/rectal cancer is curatively unresectable advanced or recurrent colonic/rectal cancer (preferably, previously untreated curatively unresectable advanced or recurrent colonic/rectal cancer).

[2-1] An agent for suppressing the progression of, suppressing the recurrence of and/or treating cancer containing an $EP_4$ antagonist, as an active ingredient, which is characterized by being administered to a cancer patient who has undergone preoperative chemoradiotherapy.
[2-2] The agent according to item [2-1] above, wherein the agent is administered in further combination with an immune checkpoint inhibitor.
[2-3] An agent for suppressing the progression of, suppressing the recurrence of and/or treating cancer containing an immune checkpoint inhibitor, as an active ingredient, which is characterized by being administered to a cancer patient who has undergonepreoperative chemoradiotherapy, in combination with an $EP_4$ antagonist.
[2-4] The agent according to any one of items [2-1] to [2-3] above, wherein the $EP_4$ antagonist is a compound represented by general formula (I) as defined in item [3] above or a salt thereof.
[2-5] The agent according to any one of items [2-1] to [2-4] above, wherein the cancer is colorectal cancer (preferably colonic/rectal cancer).
[2-6] The agent according to item [2-5] above, wherein colorectal cancer is curatively respectable locally advanced rectal cancer.
[2-7] The agent according to any one of items [2-1] to [2-6] above, wherein the immune checkpoint inhibitor and/or the $EP_4$ antagonist is used as preoperative adjuvant therapy after preoperative chemoradiotherapy (CRT).
[2-8] The agent according to any one of items [2-1] to [2-7] above, wherein the $EP_4$ antagonist is a compound represented by general formula (I-2) as defined in item [8] above or a salt thereof.

[2-9] The agent according to any one of items [2-1] to [2-8] above, wherein the $EP_4$ antagonist is 4-[4-cyano-2-({(2'R,4S)-6-[(propane-2-yl)carbamoyl]-2,3-dihydrospiro[1-benzopyran-4,l'-cyclopropane]-2'-carbonyl}amino)phenyl]butanoic acid or a salt thereof.

[2-10] The agent according to item [2-9] above, wherein the $EP_4$ antagonist is orally administered at a dose of 5 mg to 40 mg per administration once a day.

[2-11] The agent according to item [2-9] above, wherein the $EP_4$ antagonist is orally administered at a dose of 20 mg or 40 mg per administration (preferably 40 mg per administration) once a day.

[2-12] The agent according to any one of items [2-2] to [2-11] above, wherein the immune checkpoint inhibitor is an anti-PD-1 antibody, an anti-PD-L1 antibody, or an anti-CTLA-4 antibody.

[2-13] The agent according to item [2-12] above, wherein the immune checkpoint inhibitor is an anti-PD-1 antibody.

[2-14] The agent according to item [2-13] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012 (Retifanlimab), AGEN2034 (Balstilimab), CS1003, HLX10 (Serplulimab), BAT-1306, AK105, AK103, BI 754091, LZM009, CMAB819, Sym021, GB226 (Geptanolimab), SSI-361, JY034, HX008, ISU106, ABBV181 (Budigalimab), BCD-100 (Prolgolimab), PF-06801591 (Sasanlimab), CX-188, JNJ-63723283 (Cetrelimab) or AB122 (Zimberelimab).

[2-15] The agent according to item [2-12] above, wherein the immune checkpoint inhibitor is an anti-PD-L1 antibody, and the anti-PD-L1 antibody is Atezolizumab, Avelumab, Durvalumab, BMS-936559, STI-1014, KN035 (Envafolimab), LY3300054 (Lodapolimab), HLX20, SHR-1316, CS1001, MSB2311, BGB-A333, KL-A167, CK-301, AK106, AK104, ZKAB001, FAZ053, CBT-502, JS003, or CX-072.

[2-16] The agent according to item [2-12] above, wherein the immune checkpoint inhibitor is an anti-CTLA-4 antibody, and the anti-CTLA-4 antibody is Ipilimumab, AGEN1884, or Tremelimumab.

[2-17] The agent according to item [2-13] above, wherein the anti-PD-1 antibody is Nivolumab.

[2-18] The agent according to item [2-13] above, wherein the anti-PD-1 antibody is Pembrolizumab.

[2-19] The agent according to item [2-13] above, wherein the anti-PD-1 antibody is Cemiplimab.

[2-20] The agent according to item [2-15] above, wherein the anti-PD-L1 antibody is Avelumab.

[2-21] The agent according to item [2-15] above, wherein the anti-PD-L1 antibody is Atezolizumab.

[2-22] The agent according to item [2-15] above, wherein the anti-PD-L1 antibody is Durvalumab.

[2-23] The agent according to item [2-16] above, wherein the anti-CTLA-4 antibody is Ipilimumab.

[2-24] The agent according to item [2-17] above, wherein Nivolumab is administered at a dose of 3 mg/kg (body weight) or 240 mg per administration at 2-week intervals, at a dose of 360 mg per administration at 3-week intervals, or at a dose of 480 mg per administration at 4-week intervals (preferably, 240 mg per administration at 2-week intervals, at a dose of 360 mg per administration at 3-week intervals or at a dose of 480 mg per administration at 4-week intervals).

[2-25] The agent according to item [2-17] above, wherein Nivolumab is intravenously administered at a dose of 240 mg per administration at 2-week intervals.

[2-26] The agent according to item [2-17] above, wherein Nivolumab is intravenously administered at a dose of 240 mg per administration over about 30 minutes at 2-week intervals.

[2-27] The agent according to item [2-18] above, wherein Pembrolizumab is administered at a dose of 2 mg/kg (body weight) or 200 mg per administration at 3-week intervals.

[2-28] The agent according to item [2-19] above, wherein Cemiplimab is administered at a dose of 350 mg per administration at 3-week intervals.

[2-29] The agent according to item [2-20] above, wherein Avelumab is administered at a dose of 10 mg/kg (body weight) per administration at 2-week intervals.

[2-30] The agent according to item [2-21] above, wherein Atezolizumab is administered at a dose of 1200 mg per administration as at 3-week intervals.

[2-31] The agent according to item [2-22] above, wherein Durvalumab is administered at a dose of 10 mg/kg (body weight) per administration at 2-week intervals.

[2-32] The agent according to item [2-23] above, wherein Ipilimumab is administered at a dose of 3 mg/kg (body weight) or 1 mg/kg (body weight) per administration intravenously four times at 3-week intervals.

[2-33] The agent according to any one of items [2-1] to [2-32] above, wherein the administration is started within 14 days after the completion of the preoperative chemoradiotherapy.

[2-34] The agent according to any one of [2-2] to [2-33] above, wherein surgery is performed on or after 7 days from the last day of administration of the $EP_4$ antagonist and on or after 14 days from the last day of administration of the immune checkpoint inhibitor, and within 14 weeks after the completion of the preoperative chemoradiotherapy.

[2-35] The agent according to any one of items [2-1] to [2-34] above, wherein the agent is administered to a patient who has not observed as having distant metastasis by diagnostic imaging after the completion of the preoperative chemoradiotherapy.

[2-36] The agent according to any one of items [2-1] to [2-35] above, wherein the agent is administered to a patient who is not observed as having distant metastasis by diagnostic imaging after the completion of preoperative chemoradiotherapy and can be subjected to curative resection.
[2-37] The agent according to any one of items [2-1] to [2-36] above, wherein the preoperative chemoradiotherapy is a combination of radiation therapy and Capecitabine.
[2-38] The agent according to any one of items [2-1] to [2-37] above, wherein the preoperative chemoradiotherapy is a therapy in which 45 Gy/25 times of irradiation to the pelvic cavity and 5.4 Gy/3 times of boost irradiation to the primary lesion, and 825 mg/$m^2$ of Capecitabine is administered twice a day for 21 days or 42 times or more.
[2-39] An agent for suppressing the progression of, suppressing the recurrence of and/or treating cancer (preferably colorectal cancer, more preferably colonic/rectal cancer (preferably curatively respectable locally advanced rectal cancer)) containing an $EP_4$ antagonist, as an active ingredient, which is characterized by being administered to a cancer patient who has undergone preoperative chemoradiotherapy, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg, more preferably 40 mg) per administration once a day; and
(ii) the preoperative chemoradiotherapy is a combination of radiation therapy and Capecitabine (preferably, 45 Gy/25 times of irradiation to the pelvic cavity and 5.4 Gy/3 times of boost irradiation to the primary lesion, and the administration of 825 mg/$m^2$ of Capecitabine twice a day for 21 days or 42 times or more are performed in the therapy).

[2-40] The agent according to item [2-39] above, wherein the agent is administered in combination with an immune checkpoint inhibitor, wherein the immune checkpoint inhibitor is Nivolumab, and anti-Nivolumab is administered at a dose of 240 mg per administration at 2-week intervals, at a dose of 360 mg per administration at 3-week intervals, or at a dose of 480 mg per administration at 4-week intervals (preferably 240 mg per administration at 2-week intervals).
[2-41] An agent for suppressing the progression of, suppressing the recurrence of and/or treating colorectal cancer containing an immune checkpoint inhibitor, as an active ingredient, which is characterized by being administered to a cancer patient who has undergone preoperative chemoradiotherapy in combination with an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg, more preferably 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals, or at a dose of 480 mg per administration at 4-week intervals (preferably 240 mg per administration at 2-week intervals); and
(iii) the preoperative chemoradiotherapy is a combination of radiation therapy and Capecitabine (preferably, 45 Gy/25 times of irradiation to the pelvic cavity and 5.4 Gy/3 times of boost irradiation to the primary lesion, and the administration of 825 mg/$m^2$ of Capecitabine twice a day for 21 days or 42 times or more are performed in the therapy).

[3-1] The agent according to item [1] or [2] above, wherein the standard therapy is FOLFIRINOX therapy or a dose reduction regimen thereof.
[3-2] The agent according to item [3-1] above, wherein the $EP_4$ antagonist is a compound represented by general formula (I) as defined in item [3] above or a salt thereof.
[3-3] The agent according to item [3-1] or [3-2] above, wherein the cancer is pancreatic cancer (preferably pancreatic duct cancer, more preferably invasive pancreatic duct cancer).
[3-4] The agent according to item [3-3] above, wherein the pancreatic cancer is pancreatic cancer having distant metastasis.
[3-5] The agent according to any one of items [3-1] to [3-4] above, wherein the agent is administered to a patient who has not been treated with a systemic antineoplastic agent for pancreatic cancer having distant metastasis.
[3-6] The agent according to any one of items [3-1] to [3-5] above, wherein the $EP_4$ antagonist is a compound represented by general formula (I-2) as defined in item [8] above or a salt thereof.
[3-7] The agent according to any one of items [3-1] to [3-6] above, wherein the $EP_4$ antagonist is 4-[4-cyano-2-({(2'R,4S)-6-[(propane-2-yl)carbamoyl]-2,3-dihydrospiro[1-benzopyran-4,l'-cyclopropane]-2'-carbonyl}amino)phenyl]butanoic acid or a salt thereof.

[3-8] The agent according to item [3-7] above, wherein the $EP_4$ antagonist is orally administered at a dose of 5 mg to 40 mg per administration once a day.

[3-9] The agent according to item [3-7] above, wherein the $EP_4$ antagonist is orally administered at a dose of 20 mg or 40 mg per administration once a day.

[3-10] The agent according to any one of items [3-1] to [3-9] above, wherein the immune checkpoint inhibitor is an anti-PD-1 antibody, an anti-PD-L1 antibody, or an anti-CTLA-4 antibody.

[3-11] The agent according to item [3-10] above, wherein the immune checkpoint inhibitor is an anti-PD-1 antibody.

[3-12] The agent according to item [3-11] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012 (Retifanlimab), AGEN2034 (Balstilimab), CS1003, HLX10 (Serplulimab), BAT-1306, AK105, AK103, BI 754091, LZM009, CMAB819, Sym021, GB226 (Geptanolimab), SSI- 361, JY034, HX008, ISU106, ABBV181 (Budigalimab), BCD-100 (Prolgolimab), PF-06801591 (Sasanlimab), CX-188, JNJ-63723283 (Cetrelimab) or AB122 (Zimberelimab).

[3-13] The agent according to item [3-10] above, wherein the immune checkpoint inhibitor is an anti-PD-L1 antibody, and the anti-PD-L1 antibody is Atezolizumab, Avelumab, Durvalumab, BMS-936559, STI-1014, KN035 (Envafolimab), LY3300054 (Lodapolimab), HLX20, SHR-1316, CS1001, MSB2311, BGB-A333, KL-A167, CK-301, AK106, AK104, ZKAB001, FAZ053, CBT-502, JS003 and CX-072.

[3-14] The agent according to item [3-10] above, wherein the immune checkpoint inhibitor is an anti-CTLA-4 antibody, and the anti-CTLA-4 antibody is Ipilimumab, AGEN1884, or Tremelimumab.

[3-15] The agent according to item [3-11] above, wherein the anti-PD-1 antibody is Nivolumab.

[3-16] The agent according to item [3-11] above, wherein the anti-PD-1 antibody is Pembrolizumab.

[3-17] The agent according to item [3-11] above, wherein the anti-PD-1 antibody is Cemiplimab.

[3-18] The agent according to item [3-13] above, wherein the anti-PD-L1 antibody is Avelumab.

[3-19] The agent according to item [3-13] above, wherein the anti-PD-L1 antibody is Atezolizumab.

[3-20] The agent according to item [3-13] above, wherein the anti-PD-L1 antibody is Durvalumab.

[3-21] The agent according to item [3-14] above, wherein the anti-CTLA-4 antibody is Ipilimumab.

[3-22] The agent according to item [3-15] above, wherein Nivolumab is administered at a dose of 3 mg/kg (body weight) or 240 mg per administration at 2-week intervals, at a dose of 360 mg per administration at 3-week intervals, or at a dose of 480 mg per administration at 4-week intervals (preferably, 240 mg per administration at 2-week intervals, 360 mg per administration at 3-week intervals or 480 mg per administration at 4-week intervals).

[3-23] The agent according to item [3-15] above, wherein Nivolumab is intravenously administered at a dose of 480 mg per administration at 4-week intervals.

[3-24] The agent according to item [3-15] above, wherein Nivolumab is intravenously administered at a dose of 480 mg per administration over about 30 minutes at 4-week intervals.

[3-25] The agent according to item [3-16] above, wherein Pembrolizumab is administered at a dose of 2 mg/kg (body weight) or 200 mg per administration at 3-week intervals or at a dose of 400 mg per administration at 6-week intervals.

[3-26] The agent according to item [3-17] above, wherein Cemiplimab is administered at a dose of 350 mg per administration at 3-week intervals.

[3-27] The agent according to item [3-18] above, wherein Avelumab is administered at a dose of 10 mg/kg (body weight) per administration at 2-week intervals.

[3-28] The agent according to item [3-19] above, wherein Atezolizumab is administered at a dose of 1200 mg per administration at 3-week intervals.

[3-29] The agent according to item [3-20] above, wherein Durvalumab is intravenously administered at a dose of 10 mg/kg (body weight) per administration at 2-week intervals or at a dose of 1500 mg per administration four times at 4-week intervals.

[3-30] The agent according to item [3-21] above, wherein Ipilimumab is intravenously administered at a dose of 3 mg/kg (body weight) or 1 mg/kg (body weight) per administration four times at 3-week intervals or at a dose of 1 mg/kg (body weight) per administration at 6-week intervals.

[3-31] The agent according to any one of items [3-1] to [3-30] above, wherein the FOLFIRINOX therapy or a dose reduction regimen thereof is a therapy in which (i) Oxaliplatin, (ii) Levofolinate calcium, (iii) Irinotecan hydrochloride hydrate and (iv) Fluorouracil are administered in combination.

[3-32] The agent according to any one of items [3-1] to [3-31] above, wherein the FOLFIRINOX therapy includes:

(i) administering Oxaliplatin at a dose of 50 to 85 mg/$m^2$ (preferably 50 mg/$m^2$, 65 mg/$m^2$ or 85 mg/$m^2$, more preferably 85 mg/$m^2$) intravenously;

(ii) administering Levofolinate calcium at a dose of 200 mg/$m^2$ intravenously;

(iii) administering Irinotecan hydrochloride hydrate at a dose of 90 to 180 mg/$m^2$ (preferably 90 mg/$m^2$, 120

mg/$m^2$, 150 mg/$m^2$ or 180 mg/$m^2$, more preferably 180 mg/$m^2$) intravenously,
(iv) administering Fluorouracil at a dose of 400 mg/$m^2$ rapidly intravenously; and
(v) further administering Fluorouracil continuously intravenously at a dose of 1200 to 2400 mg/$m^2$ (preferably 1200 mg/$m^2$, 1800 mg/$m^2$ or 2400 mg/$m^2$, more preferably 2400 mg/$m^2$).

[3-33] The agent according to any one of items [3-1] to [3-31] above, wherein the dose reduction regimen of the FOLFIRINOX therapy includes:

(i) administering Oxaliplatin at a dose of 50 to 85 mg/$m^2$ (preferably 50 mg/$m^2$, 65 mg/$m^2$ or 85 mg/$m^2$, more preferably 85 mg/$m^2$) intravenously;
(ii) administering Levofolinate calcium at a dose of 200 mg/$m^2$ intravenously;
(iii) administering Irinotecan hydrochloride hydrate at a dose of 120 to 140 mg/$m^2$ (preferably 140 mg/$m^2$) intravenously; and
(iv) administering Fluorouracil continuously intravenously at a dose of 1200 to 2400 mg/$m^2$ (preferably 1200 mg/$m^2$, 1800 mg/$m^2$ or 2400 mg/$m^2$, more preferably 2400 mg/$m^2$).

[3-34] The agent according to any one of items [3-1] to [3-33] above, wherein the FOLFIRINOX therapy or a dose reduction regimen thereof is a therapy in which the series of the administrations are performed at 2- to 4-week intervals (preferably 2-week intervals, 3-week intervals or 4-week intervals, more preferably 2-week intervals). [3-35] The agent according to any one of items [3-1] to [3-32] and [3-34] above, wherein the FOLFIRINOX therapy includes:

(i) administering Oxaliplatin at a dose of 85 mg/$m^2$ intravenously over 2 hours;
(ii) administering Levofolinate calcium at a dose of 200 mg/$m^2$ intravenously over 2 hours after the completion of the administration of Oxaliplatin;
(iii) administering Irinotecan hydrochloride hydrate at a dose of 180 mg/$m^2$ intravenously over 1.5 hours from 30 minutes after the start of the administration of Levofolinate calcium;
(iv) further administrating, after the completion of the administration of Levofolinate calcium, Fluorouracil at a dose of 400 mg/$m^2$ rapidly intravenously; and
(v) further administering Fluorouracil at a dose of 2400 mg/$m^2$ continuously intravenously over 46 hours,

wherein the series of the administrations are performed at 2-week intervals.
[3-36] The agent according to any one of items [3-1] to [3-31], [3-33] and [3-34] above, wherein the dose reduction regimen of the FOLFIRINOX therapy includes:

(i) administering Oxaliplatin at a dose of 85 mg/$m^2$ intravenously over 2 hours;
(ii) administering Levofolinate calcium at a dose of 200 mg/$m^2$ intravenously over 2 hours;
(iii) administering Irinotecan hydrochloride hydrate at a dose of 150 mg/$m^2$ intravenously over 1.5 hours from 30 minutes after the start of the administration of Levofolinate calcium; and
(iv) further administering Fluorouracil at a dose of 2400 mg/$m^2$ continuously intravenously over 46 hours after the completion of the administration of the Levofolinate calcium,

wherein the series of the administrations are performed at 2-week intervals.
[3-37] The agent according to any one of items [3-1] to [3-36] above, wherein the FOLFIRINOX therapy or a dose reduction regimen thereof and the administration of the immune checkpoint inhibitor and the administration of the $EP_4$ antagonist are started on the same day in the therapy.
[3-38] An agent for suppressing the progression of, suppressing the recurrence of and/or treating cancer containing an $EP_4$ antagonist, as an active ingredient, which is characterized by being administered in combination with FOLFIRINOX therapy or a dose reduction regimen thereof and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 480 mg per administration at 4-week intervals);
(iii) the FOLFIRINOX therapy includes:

(a) administering Oxaliplatin at a dose of 85 mg/$m^2$ intravenously over 2 hours;

(b) administering Levofolinate calcium at a dose of 200 mg/$m^2$ intravenously over 2 hours;
(c) administering Irinotecan hydrochloride hydrate at a dose of 180 mg/$m^2$ intravenously over 1.5 hours from 30 minutes after the start of the administration of Levofolinate calcium;
(d) further administering, after the completion of the administration of the Levofolinate calcium, Fluorouracil at a dose of 400 mg/$m^2$ rapidly intravenously; and
(e) further administering Fluorouracil at a dose of 2400 mg/$m^2$ continuously intravenously over 46 hours, wherein the series of the administrations are performed at 2-week intervals, and wherein the dose reduction regimen of the FOLFIRINOX therapy includes

(a) administering Oxaliplatin 85 mg/$m^2$ intravenously over 2 hours,
(b) administering Levofolinate calcium 200 mg/$m^2$ intravenously over 2 hours,
(c) administering Irinotecan hydrochloride hydrate at 150 mg/$m^2$ intravenously over 1.5 from 30 minutes after the start of Levofolinate calcium administration, and
(d) further intravenously administering 2400 mg/$m^2$ of Fluorouracil over 46 hours after the completion of the administration of the Levofolinate calcium,

wherein the series of the administrations are performed at 2-week intervals.
[3-39] An agent for suppressing the progression of, suppressing the recurrence of and/or treating cancer containing an immune checkpoint inhibitor, as an active ingredient, which is characterized by being administered in combination with a FOLFIRINOX therapy or a dose reduction regimen thereof and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is administered at a dose of 5 mg to 40 mg (preferably 20 mg or 40 mg) per administration once a day orally;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 480 mg per administration at 4-week intervals), and
(iii) the FOLFIRINOX therapy includes:

(a) administering Oxaliplatin at a dose of 85 mg/$m^2$ intravenously over 2 hours;
(b) administering Levofolinate calcium at a dose of 200 mg/$m^2$ intravenously over 2 hours;
(c) administering Irinotecan hydrochloride hydrate at a dose of 180 mg/$m^2$ intravenously over 1.5 hours from 30 minutes after the start of the administration of Levofolinate calcium;
(d) further administering, after the completion of the administration of Levofolinate calcium, Fluorouracil at a dose of 400 mg/$m^2$ rapidly intravenously; and
(e) further administering Fluorouracil at a dose of 2400 mg/$m^2$ continuously intravenously over 46 hours, wherein the series of the administrations are performed at 2-week intervals, and wherein the dose reduction regimen of the FOLFIRINOX therapy includes

(a) administering Oxaliplatin at a dose of 85 mg/$m^2$ intravenously over 2 hours,
(b) administering Levofolinate calcium at a dose of 200 mg/$m^2$ intravenously over 2 hours,
(c) administering Irinotecan hydrochloride hydrate at a dose of 150 mg/$m^2$ intravenously over 1.5 from 30 minutes after the start of Levofolinate calcium administration and
(d) further administering Fluorouracil at a dose of 2400 mg/$m^2$ intravenously over 46 hours after the completion of the administration of Levofolinate calcium,

wherein the series of the administrations are performed at 2-week intervals.

[3-40] The agent according to item [3-38] or [3-39] above, wherein the FOLFIRINOX therapy or a dose reduction regimen thereof, the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the administration of Nivolumab are started on the same day in the therapy.
[3-41] The agent according to any one of items [3-38] to [3-40] above, wherein the cancer is pancreatic cancer (preferably pancreatic duct cancer, more preferably invasive pancreatic duct cancer).
[3-42] The agent according to item [3-41] above, wherein the pancreatic cancer is pancreatic cancer having distant metastasis.
[3-43] The agent according to any one of items [3-38] to [3-42] above, wherein the agent is administered to a patient who has not been treated with a systemic antineoplastic agent for pancreatic cancer having distant metastasis.

[4-1] The agent according to item [1] or [2] above, wherein the standard therapy is Gemcitabine-and-nab-Paclitaxel therapy.
[4-2] The agent according to item [4-1] above, wherein the $EP_4$ antagonist is a compound represented by general formula (I) as defined in item [3] above or a salt thereof.
[4-3] The agent according to item [4-1] or [4-2] above, wherein the cancer is pancreatic cancer (preferably pancreatic duct cancer, more preferably invasive pancreatic duct cancer).
[4-4] The agent according to item [4-3] above, wherein the pancreatic cancer is pancreatic cancer having distant metastasis.
[4-5] The agent according to any one of items [4-1] to [4-4] above, wherein the agent is administered to a patient who has not been treated with a systemic antineoplastic agent for pancreatic cancer having distant metastasis.
[4-6] The agent according to any one of items [4-1] to [4-5] above, wherein the $EP_4$ antagonist is a compound represented by general formula (I-2) as defined in item [8] above or a salt thereof.
[4-7] The agent according to any one of items [4-1] to [4-6] above, wherein the $EP_4$ antagonist is 4-[4-cyano-2-({(2'R,4S)-6-[(propane-2-yl)carbamoyl]-2,3-dihydrospiro[1-benzopyran-4,l'-cyclopropane]-2'-carbonyl}amino)phenyl]butanoic acid or a salt thereof.
[4-8] The agent according to item [4-7] above, wherein the $EP_4$ antagonist is orally administered at a dose of 5 mg to 40 mg per administration once a day.
[4-9] The agent according to item [4-7] above, wherein the $EP_4$ antagonist is orally administered at a dose of 20 mg or 40 mg (preferably 40 mg) per administration once a day.
[4-10] The agent according to any one of items [4-1] to [4-9] above, wherein the immune checkpoint inhibitor is an anti-PD-1 antibody, an anti-PD-L1 antibody, or an anti-CTLA-4 antibody.
[4-11] The agent according to item [4-10] above, wherein the immune checkpoint inhibitor is an anti-PD-1 antibody.
[4-12] The agent according to item [4-11] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012 (Retifanlimab), AGEN2034 (Balstilimab), CS1003, HLX10 (Serplulimab), BAT-1306, AK105, AK103, BI 754091, LZM009, CMAB819, Sym021, GB226 (Geptanolimab), SSI-361, JY034, HX008, ISU106, ABBV181 (Budigalimab), BCD-100(Prolgolimab), PF-06801591 (Sasanlimab), CX-188, JNJ-63723283 (Cetrelimab) or AB122 (Zimberelimab).
[4-13] The agent according to item [4-10] above, wherein the immune checkpoint inhibitor is an anti-PD-L1 antibody, and the anti-PD-L1 antibody is Atezolizumab, Avelumab, Durvalumab, BMS-936559, STI-1014, KN035 (Envafolimab), LY3300054 (Lodapolimab), HLX20, SHR-1316, CS1001, MSB2311, BGB-A333, KL-A167, CK-301, AK106, AK104, ZKAB001, FAZ053, CBT-502, JS003 and CX-072.
[4-14] The agent according to item [4-10] above, wherein the immune checkpoint inhibitor is an anti-CTLA-4 antibody, and the anti-CTLA-4 antibody is Ipilimumab, AGEN1884, or Tremelimumab.
[4-15] The agent according to item [4-11] above, wherein the anti-PD-1 antibody is Nivolumab.
[4-16] The agent according to item [4-11] above, wherein the anti-PD-1 antibody is Pembrolizumab.
[4-17] The agent according to item [4-11] above, wherein the anti-PD-1 antibody is Cemiplimab.
[4-18] The agent according to item [4-13] above, wherein the anti-PD-L1 antibody is Avelumab.
[4-19] The agent according to item [4-13] above, wherein the anti-PD-L1 antibody is Atezolizumab.
[4-20] The agent according to item [4-13] above, wherein the anti-PD-L1 antibody is Durvalumab.
[4-21] The agent according to item [4-14] above, wherein the anti-CTLA-4 antibody is Ipilimumab.
[4-22] The agent according to item [4-15] above, wherein Nivolumab is administered at a dose of 3 mg/kg (body weight) or 240 mg per administration at 2-week intervals, at a dose of 360 mg per administration at 3-week intervals, or at a dose of 480 mg per administration at 4-week intervals (preferably, 240 mg per administration at 2-week intervals, 360 mg per administration at 3-week intervals or 480 mg per administration at 4-week intervals).
[4-23] The agent according to item [4-15] above, wherein Nivolumab is intravenously administered at a dose of 480 mg per administration at 4-week intervals.
[4-24] The agent according to item [4-15] above, wherein Nivolumab is intravenously administered at a dose of 480 mg per administration over about 30 minutes at 4-week intervals.
[4-25] The agent according to item [4-16] above, wherein Pembrolizumab is administered at a dose of 2 mg/kg (body weight) or 200 mg per administration at 3-week intervals.
[4-26] The agent according to item [4-17] above, wherein Cemiplimab is administered at a dose of 350 mg per administration at 3-week intervals.
[4-27] The agent according to item [4-18] above, wherein Avelumab is administered at a dose of 10 mg/kg (body weight) per administration at 2-week intervals.
[4-28] The agent according to item [4-19] above, wherein Atezolizumab is administered at a dose of 1200 mg per administration at 3-week intervals.
[4-29] The agent according to item [4-20] above, wherein Durvalumab is administered at a dose of 10 mg/kg (body

weight) per administration at 2-week intervals.
[4-30] The agent according to item [4-21] above, wherein Ipilimumab is administered at a dose of 3 mg/kg (body weight) or 1 mg/kg (body weight) per administration intravenously four times at 3-week intervals.
[4-31] The agent according to any one of items [4-1] to [4-30] above, wherein the Gemcitabine-and-nab-Paclitaxel therapy includes:

(i) administering Gemcitabine at a dose of 600 to 1000 mg/$m^2$ (preferably 600 mg/$m^2$, 800 mg/$m^2$ or 1000 mg/$m^2$, more preferably 1000 mg/$m^2$) intravenously; and
(ii) administering nab-Paclitaxel at a dose of 75 to 125 mg/$m^2$ (preferably 75 mg/$m^2$, 100 mg/$m^2$ or 125 mg/$m^2$, more preferably 125 mg/$m^2$) intravenously.

[4-32] The agent according to any one of items [4-1] to [4-31] above, wherein the Gemcitabine-and-nab-Paclitaxel therapy is a therapy in which the series of administrations are performed at 1-week intervals and are continued for 3 weeks, with a 1-week rest period.
[4-33] The agent according to any one of items [4-1] to [4-32] above, wherein the Gemcitabine-and-nab-Paclitaxel therapy includes:

(i) administering Gemcitabine at a dose of 1000 mg/$m^2$ intravenously over 30 minutes; and
(ii) administering nab-Paclitaxel at a dose of 125 mg/$m^2$ intravenously over 30 minutes,

wherein the series of the administrations are performed at 1-week intervals and are continued for 3 weeks, with a 1-week rest period.
[4-34] The agent according to any one of items [4-1] to [4-33] above, wherein the administration of Gemcitabine and the administration of nab-Paclitaxel are started on the same day in the therapy.
[4-35] The agent according to any one of items [4-1] to [4-34] above, wherein the administration of Gemcitabine, the administration of nab-Paclitaxel, the administration of an immune checkpoint inhibitor, and the administration of an $EP_4$ antagonist are started on the same day in the therapy.
[4-36] An agent for suppressing the progression of, suppressing the recurrence of and/or treating cancer containing an $EP_4$ antagonist, as an active ingredient, which is characterized by being administered in combination with a Gemcitabine-and-nab-Paclitaxel therapy and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 480 mg per administration at 4-week intervals), and
(iii) the Gemcitabine-and-nab-Paclitaxel therapy includes:
(a) administering Gemcitabine at a dose of 1000 mg/$m^2$ intravenously over 30 minutes; and (b) administering nab-Paclitaxel at a dose of 125 mg/$m^2$ intravenously over 30 minutes, wherein the series of the administrations are performed at 1-week intervals and continued for 3 weeks, with a 1-week rest period.

[4-37] An agent for suppressing the progression of, suppressing the recurrence of and/or treating cancer containing an immune checkpoint inhibitor, as an active ingredient, which is characterized by being administered in combination with a Gemcitabine-and-nab-Paclitaxel therapy and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 480 mg per administration at 4-week intervals), and
(iii) the Gemcitabine-and-nab-Paclitaxel therapy is a therapy in which (a) Gemcitabine is administered at a dose of 1000 mg/$m^2$ intravenously over 30 minutes, and (b) nab-Paclitaxel is administered at a dose of 125 mg/$m^2$ intravenously over 30 minutes,

wherein the series of the administrations are performed at 1-week intervals and continued for 3 weeks, with a 1-week rest period.

[4-38] The agent according to item [4-36] or [4-37] above, wherein the cancer is pancreatic cancer (preferably pancreatic duct cancer, more preferably invasive pancreatic duct cancer).
[4-39] The agent according to item [4-38] above, wherein the pancreatic cancer is pancreatic cancer having distant metastasis.
[4-40] The agent according to any one of items [4-36] to [4-39] above, wherein the agent is administered to a patient who has not been treated with a systemic antineoplastic agent for pancreatic cancer having distant metastasis.
[5-1] The agent according to [1] or [2] above, wherein the standard therapy is a Docetaxel-and/or-Ramucirumab therapy.
[5-2] The agent according to item [5-1] above, wherein the standard therapy is a Docetaxel therapy.
[5-3] The agent according to item [5-1] above, wherein the standard therapy is a Ramucirumab therapy.
[5-4] The agent according to item [5-1] above, wherein the standard therapy is a Docetaxel-and-Ramucirumab therapy.
[5-5] The agent according to any one of items [5-1] to [5-4] above, wherein the $EP_4$ antagonist is a compound represented by general formula (I) as defined in item [3] above or a salt thereof.
[5-6] The agent according to any one of items [5-1] to [5-5] above, wherein the cancer is lung cancer (preferably, non-small cell lung cancer).
[5-7] The agent according to item [5-6] above, wherein the lung cancer is stage-IV or recurrent non-small cell lung cancer (preferably advanced or recurrent non-small cell lung cancer refractory to a combination therapy including an anti-PD-1 antibody or an anti-PD-L1 antibody, and a platinum preparation).
[5-8] The agent according to any one of items [5-1] to [5-7] above, wherein the agent is administered to a patient who has undergone a combination therapy including an anti-PD-1 antibody or an anti-PD-L1 antibody and a platinum preparation, and has been diagnosed as having refractory progression or recurrence.
[5-9] The agent according to any one of items [5-1] to [5-8] above, wherein the $EP_4$ antagonist is a compound represented by general formula (I-2) as defined in item [8] above or a salt thereof.
[5-10] The agent according to any one of items [5-1] to [5-9] above, wherein the $EP_4$ antagonist is 4-[4-cyano-2-({(2'R,4S)-6-[(propane-2-yl)carbamoyl]-2,3-dihydrospiro[1-benzopyran-4,l'-cyclopropane]-2'-carbonyl}amino)phenyl]butanoic acid or a salt thereof.
[5-11] The agent according to item [5-10] above, wherein the $EP_4$ antagonist is orally administered at a dose of 5 mg to 40 mg per administration once a day.
[5-12] The agent according to item [5-10] above, wherein the $EP_4$ antagonist is orally administered at a dose of 20 mg or 40 mg per administration once a day.
[5-13] The agent according to any one of items [5-1] to [5-12] above, wherein the immune checkpoint inhibitor is an anti-PD-1 antibody, an anti-PD-L1 antibody, or an anti-CTLA-4 antibody.
[5-14] The agent according to item [5-13] above, wherein the immune checkpoint inhibitor is an anti-PD-1 antibody.
[5-15] The agent according to item [5-14] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012 (Retifanlimab), AGEN2034 (Balstilimab), CS1003, HLX10 (Serplulimab), BAT-1306, AK105, AK103, BI 754091, LZM009, CMAB819, Sym021, GB226 (Geptanolimab), SSI-361, JY034, HX008, ISU106, ABBV181 (Budigalimab), BCD-100 (Prolgolimab), PF-06801591 (Sasanlimab), CX-188, JNJ-63723283 (Cetrelimab) or AB 122 (Zimberelimab).
[5-16] The agent according to item [5-13] above, wherein the immune checkpoint inhibitor is an anti-PD-L1 antibody, and the anti-PD-L1 antibody is Atezolizumab, Avelumab, Durvalumab, BMS-936559, STI-1014, KN035 (Envafolimab), LY3300054 (Lodapolimab), HLX20, SHR-1316, CS1001, MSB2311, BGB-A333, KL-A167, CK-301, AK106, AK104, ZKAB001, FAZ053, CBT-502, JS003 and CX-072.
[5-17] The agent according to item [5-13] above, wherein the immune checkpoint inhibitor is an anti-CTLA-4 antibody, and the anti-CTLA-4 antibody is Ipilimumab, AGEN1884, or Tremelimumab.
[5-18] The agent according to item [5-14] above, wherein the anti-PD-1 antibody is Nivolumab.
[5-19] The agent according to item [5-14] above, wherein the anti-PD-1 antibody is Pembrolizumab.
[5-20] The agent according to item [5-14] above, wherein the anti-PD-1 antibody is Cemiplimab.
[5-21] The agent according to item [5-16] above, wherein the anti-PD-L1 antibody is Avelumab.
[5-22] The agent according to item [5-16] above, wherein the anti-PD-L1 antibody is Atezolizumab.
[5-23] The agent according to item [5-16] above, wherein the anti-PD-L1 antibody is Durvalumab.
[5-24] The agent according to items [5-17] above, wherein the anti-CTLA-4 antibody is Ipilimumab.
[5-25] The agent according to item [5-18] above, wherein Nivolumab is administered at a dose of 3 mg/kg (body weight) or 240 mg per administration at 2-week intervals, at a dose of 360 mg per administration at 3-week intervals, or at a dose of 480 mg per administration at 4-week intervals (preferably, 240 mg per administration at 2-week intervals, 360 mg per administration at 3-week intervals, or 480 mg per administration at 4-week intervals).
[5-26] The agent according to item [5-18] above, wherein Nivolumab is intravenously administered at a dose of 360

mg per administration at 3-week intervals.

[5-27] The agent according to item [5-18] above, wherein Nivolumab is intravenously administered at a dose of 360 mg per administration over about 30 minutes at 3-week intervals.

[5-28] The agent according to item [5-19] above, wherein Pembrolizumab is administered at a dose of 2 mg/kg (body weight) per administration or at a dose of 200 mg per administration at 3-week intervals, or at a dose of 400 mg per administration at 6-week intervals.

[5-29] The agent according to item [5-20] above, wherein Cemiplimab is administered at a dose of 350 mg per administration at 3-week intervals.

[5-30] The agent according to item [5-21] above, wherein Avelumab is administered at a dose of 10 mg/kg (body weight) per administration at 2-week intervals.

[5-31] The agent according to item [5-22] above, wherein Atezolizumab is administered at a dose of 1200 mg per administration at 3-week intervals.

[5-32] The agent according to item [5-23] above, wherein Durvalumab is administered at a dose of 10 mg/kg (body weight) per administration at 2-week intervals or at a dose of 1500 mg per administration intravenously 4 times at 4-week intervals.

[5-33] The agent according to item [5-24] above, wherein Ipilimumab is administered at a dose of 3 mg/kg (body weight) or 1 mg/kg (body weight) per administration intravenously four times at 3-week intervals or at a dose of 1 mg/kg (body weight) per administration at 6-week intervals.

[5-34] The agent according to any one of items [5-1], [5-2], and [5-4] to [5-33] above, wherein the Docetaxel therapy is a therapy in which 50 to 75 $mg/m^2$ (preferably 50 $mg/m^2$, 60 $mg/m^2$ or 75 $mg/m^2$, more preferably 60 $mg/m^2$) of Docetaxel is intravenously administered.

[5-35] The agent according to any one of items [5-1], [5-2], and [5-4] to [5-34] above, wherein the Docetaxel therapy is a therapy in which 50 to 75 $mg/m^2$ (preferably 50 $mg/m^2$, 60 $mg/m^2$ or 75 $mg/m^2$, more preferably 60 $mg/m^2$) of Docetaxel is intravenously administered over 60 minutes or longer, and the administration is performed at 3-week intervals.

[5-36] The agent according to any one of items [5-1], [5-3], and [5-4] to [5-33] above, wherein the Ramucirumab therapy is a therapy in which 6 to 10 mg/kg (preferably 6 mg/kg, 8 mg/kg or 10 mg/kg, more preferably 10 mg/kg) of Ramucirumab is intravenously administered.

[5-37] The agent according to any one of items [5-1], [5-3], [5-4] to [5-33], and [5-36] above, wherein the Ramucirumab therapy is a therapy in which 6 to 10 mg/kg (preferably 6 mg/kg, 8 mg/kg or 10 mg/kg, more preferably 10 mg/kg) of Ramucirumab is intravenously administered over 30 to 60 minutes (preferably 60 minutes), and the series of the administrations are performed at 3-week intervals.

[5-38] The agent according to any one of items [5-1] and [5-4] to [5-33] above, wherein the Docetaxel-and-Ramucirumab therapy is a therapy in which:

(i) Docetaxel is administered at a dose of 50 to 75 $mg/m^2$ (preferably 50 $mg/m^2$, 60 $mg/m^2$ or 75 $mg/m^2$, more preferably 60 $mg/m^2$) intravenously, and
(ii) Ramucirumab is administered at a dose of 6 to 10 mg/kg (preferably 6 mg/kg, 8 mg/kg or 10 mg/kg, more preferably 10 mg/kg) intravenously.

[5-39] The agent according to any one of items [5-1], [5-4] to [5-33], and [5-38] above, wherein the Docetaxel-and-Ramucirumab therapy is a therapy in which:

(i) Docetaxel is administered at a dose of 50 to 75 $mg/m^2$ (preferably 50 $mg/m^2$, 60 $mg/m^2$ or 75 $mg/m^2$, more preferably 60 $mg/m^2$) intravenously over 60 minutes or longer, and
(ii) Ramucirumab is administered at a dose of 6 to 10 mg/kg (preferably 6 mg/kg, 8 mg/kg or 10 mg/kg, more preferably 10 mg/kg) intravenously over 30 to 60 minutes (preferably 60 minutes),

wherein the series of the administrations are performed at 3-week intervals.

[5-40] The agent according to any one of items [5-1] to [5-39] above, wherein the Docetaxel-and/or-Ramucirumab therapy, the administration of the immune checkpoint inhibitor and the administration of the $EP_4$ antagonist are started on the same day in the therapy.

[5-41] An agent for suppressing the progression of, suppressing the recurrence of and/or treating cancer containing an $EP_4$ antagonist, as an active ingredient, which is characterized by being administered in combination with a Docetaxel therapy and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is admin-

istered at a dose of 5 mg to 40 mg (preferably 20 mg or 40 mg) per administration orally once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Docetaxel therapy is a therapy in which 50 to 75 mg/$m^2$ (preferably 50 mg/$m^2$, 60 mg/$m^2$ or 75 mg/$m^2$, more preferably 60 mg/$m^2$) of Docetaxel is intravenously administered over 60 minutes or longer, and the administration is performed at 3-week intervals.

[5-42] An agent for suppressing the progression of, suppressing the recurrence of and/or treating cancer containing an immune checkpoint inhibitor, as an active ingredient, which is characterized by being administered in combination with a Docetaxel therapy and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is administered orally at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Docetaxel therapy is a therapy in which 50 to 75 mg/$m^2$ (preferably 50 mg/$m^2$, 60 mg/$m^2$ or 75 mg/$m^2$, more preferably 60 mg/$m^2$) of Docetaxel is intravenously administered over 60 minutes or longer, and the administration is performed at 3-week intervals.

[5-43] The agent according to item [5-41] or [5-42] above, wherein the therapy is a therapy in which the administration of Docetaxel, the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the administration of Nivolumab are started on the same day (wherein, when the administrations are performed on the same day, it is preferred that Docetaxel is administered after the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof and Nivolumab.
[5-44] An agent for suppressing the progression of, suppressing the recurrence of and/or treating cancer containing an $EP_4$ antagonist, as an active ingredient, which is characterized by being administered in combination with a Ramucirumab therapy and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is administered orally at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Ramucirumab therapy is a therapy in which 6 to 10 mg/kg (preferably 6 mg/kg, 8 mg/kg or 10 mg/kg, more preferably 10 mg/kg) of Ramucirumab is administered intravenously over 30 to 60 minutes (preferably 60 minutes), and the series of the administrations are performed at 3-week intervals.

[5-45] An agent for suppressing the progression of, suppressing the recurrence of and/or treating cancer containing an immune checkpoint inhibitor, as an active ingredient, which is characterized by being administered in combination with a Ramucirumab therapy and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Ramucirumab therapy is a therapy in which 6 to 10 mg/kg (preferably 6 mg/kg, 8 mg/kg or 10 mg/kg, more preferably 10 mg/kg) of Ramucirumab is administered intravenously over 30 to 60 minutes (preferably 60 minutes), and the series of the administrations are performed at 3-week intervals.

[5-46] The agent according to item [5-44] or [5-45] above, wherein the therapy is a therapy in which the administration

of Ramucirumab, the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the administration of Nivolumab are started on the same day (wherein, when the administrations are performed on the same day, it is preferred that Ramucirumab is administered after the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof and Nivolumab).

[5-47] An agent for suppressing the progression of, suppressing the recurrence of and/or treating cancer containing an $EP_4$ antagonist, as an active ingredient, which is characterized by being administered in combination with a Docetaxel-and-Ramucirumab therapy and an immune checkpoint inhibitor, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Docetaxel-and-Ramucirumab therapy includes:

(a) administering Docetaxel at a dose of 50 to 75 $mg/m^2$ (preferably 50 $mg/m^2$, 60 $mg/m^2$ or 75 $mg/m^2$, more preferably 60 $mg/m^2$) intravenously over 60 minutes or longer; and
(b) administering Ramucirumab at a dose of 6 to 10 mg/kg (preferably 6 mg/kg, 8 mg/kg or 10 mg/kg, more preferably 10 mg/kg) intravenously over 30 to 60 minutes (preferably 60 minutes), and the series of the administrations are performed at 3-week intervals.

[5-48] An agent for suppressing the progression of, suppressing the recurrence of and/or treating cancer containing an immune checkpoint inhibitor, as an active ingredient, which is characterized by being administered in combination with a Docetaxel-and-Ramucirumab therapy and an $EP_4$ antagonist, wherein:

(i) the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the $EP_4$ antagonist is orally administered at a dose of 5 mg to 40 mg (preferably 20 mg or 40 mg) per administration once a day;
(ii) the immune checkpoint inhibitor is Nivolumab, and Nivolumab is administered at a dose of 240 mg per administration at 2-week intervals, at a dose of 360 mg per administration at 3-week intervals or at a dose of 480 mg per administration at 4-week intervals (preferably 360 mg per administration at 3-week intervals), and
(iii) the Docetaxel-and-Ramucirumab therapy includes:

(a) administering Docetaxel at a dose of 50 to 75 $mg/m^2$ (preferably 50 $mg/m^2$, 60 $mg/m^2$ or 75 $mg/m^2$, more preferably 60 $mg/m^2$) intravenously over 60 minutes or longer; and
(b) administering Ramucirumab at a dose of 6 to 10 mg/kg (preferably 6 mg/kg, 8 mg/kg or 10 mg/kg, more preferably 10 mg/kg) intravenously over 30 to 60 minutes (preferably 60 minutes), and the series of the administrations are performed at 3-week intervals.

[5-49] The agent according to item [5-47] or [5-48] above, wherein the therapy is a therapy in which the administration of Docetaxel, the administration of Ramucirumab, the administration of 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof, and the administration of Nivolumab are started on the same day (wherein, when the administrations are performed on the same day, it is preferred that Docetaxel and Ramucirumab are administered after the administration of 4-[4-cyano-2-({ (2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof and Nivolumab).

[5-50] The agent according to any one of items [5-41] to [5-49] above, wherein the cancer is lung cancer (preferably, non-small cell lung cancer).

[5-51] The agent according to item [5-50] above, wherein the lung cancer is stage-IV or recurrent non-small cell lung cancer (preferably advanced or recurrent non-small cell lung cancer refractory to a combination therapy including an anti-PD-1 antibody or an anti-PD-L1 antibody, and a platinum preparation).

[5-52] The agent according to any one of items [5-41] to [5-51] above, wherein the agent is administered to a patient who has undergone a combination therapy including an anti-PD-1 antibody or an anti-PD-L1 antibody and a platinum preparation, and has been diagnosed as having refractory progression or recurrence.

[6-1] An $EP_4$ antagonist for use in the suppression of the progression of, the suppression of the recurrence of and/or

the treatment of cancer, wherein the $EP_4$ antagonist is administered in combination with a standard therapy (preferably, (i) a Bevacizumab-and-XELOX therapy, (iii) a FOLFIRINOX therapy or a dose reduction regimen thereof, (iv) a Gemcitabine-and-nab-Paclitaxel therapy, or (v) Docetaxel-and/or-Ramucirumab therapy, more preferably (i) Bevacizumab-and-XELOX therapy, (iii) FOLFIRINOX therapy or a dose reduction regimen thereof, (iv) Gemcitabine-and-nab-Paclitaxel therapy, (vii) Docetaxel therapy, or (vi) a Docetaxel-and-Ramucirumab therapy, even more preferably (i) a Bevacizumab-and-XELOX therapy, (iii) a FOLFIRINOX therapy or a dose reduction regimen thereof, (iv) a Gemcitabine-and-nab-Paclitaxel therapy, or (vi) a Docetaxel-and-Ramucirumab therapy) and the administration of an immune checkpoint inhibitor.

[6-2] An immune checkpoint inhibitor for use in the suppression of the progression of, the suppression of the recurrence of and/or the treatment of cancer, wherein the immune checkpoint inhibitor is administered in combination with a standard therapy (preferably, (i) a Bevacizumab-and-XELOX therapy, (iii) a FOLFIRINOX therapy or a dose reduction regimen thereof, (iv) a Gemcitabine-and-nab-Paclitaxel therapy, or (v) a Docetaxel-and/or-Ramucirumab therapy, more preferably (i) a Bevacizumab-and-XELOX therapy, (iii) a FOLFIRINOX therapy or a dose reduction regimen thereof, (iv) a Gemcitabine-and-nab-Paclitaxel therapy, (vii) a Docetaxel therapy, or (vi) a Docetaxel-and-Ramucirumab therapy, even more preferably (i) a Bevacizumab-and-XELOX therapy, (iii) a FOLFIRINOX therapy or a dose reduction regimen thereof, (iv) a Gemcitabine-and-nab-Paclitaxel therapy, or (vi) a Docetaxel-and-Ramucirumab therapy) and the administration of an $EP_4$ antagonist.

[6-3] Use of an $EP_4$ antagonist for the manufacture of an agent for suppressing the progression of, suppressing the recurrence of and/or treating cancer, which is administered in combination with a standard therapy (preferably, (i) a Bevacizumab-and-XELOX therapy, (iii) a FOLFIRINOX therapy or a dose reduction regimen thereof, (iv) a Gemcitabine-and-nab-Paclitaxel therapy, or (v) a Docetaxel-and/or-Ramucirumab therapy, more preferably (i) a Bevacizumab-and-XELOX therapy, (iii) a FOLFIRINOX therapy or a dose reduction regimen thereof, (iv) a Gemcitabine-and-nab-Paclitaxel therapy, (vii) a Docetaxel therapy, or (vi) a Docetaxel-and-Ramucirumab therapy, even more preferably (i) a Bevacizumab-and-XELOX therapy, (iii) a FOLFIRINOX therapy or a dose reduction regimen thereof, (iv) a Gemcitabine-and-nab-Paclitaxel therapy, or (vi) a Docetaxel-and-Ramucirumab therapy) and the administration of an immune checkpoint inhibitor.

[6-4] Use of an immune checkpoint inhibitor for the manufacture of an agent for suppressing the progression of, suppressing the recurrence of and/or treating cancer, which is administered in combination with a standard therapy (preferably, (i) a Bevacizumab-and-XELOX therapy, (iii) a FOLFIRINOX therapy or a dose reduction regimen thereof, (iv) a Gemcitabine-and-nab-Paclitaxel therapy, or (v) a Docetaxel-and/or-Ramucirumab therapy, more preferably (i) a Bevacizumab-and-XELOX therapy, (iii) a FOLFIRINOX therapy or a dose reduction regimen thereof, (iv) a Gemcitabine-and-nab-Paclitaxel therapy, (vii) a Docetaxel therapy, or (vi) a Docetaxel-and-Ramucirumab therapy, even more preferably (i) a Bevacizumab-and-XELOX therapy, (iii) a FOLFIRINOX therapy or a dose reduction regimen thereof, (iv) a Gemcitabine-and-nab-Paclitaxel therapy, or (vi) a Docetaxel-and-Ramucirumab therapy) and the administration of an $EP_4$ antagonist.

[6-5] A method for suppressing the progression of, suppressing the recurrence of and/or treating cancer including administering an effective amount of a standard therapy (preferably, (i) a Bevacizumab-and-XELOX therapy, (iii) a FOLFIRINOX therapy or a dose reduction regimen thereof, (iv) a Gemcitabine-and-nab-Paclitaxel therapy, or (v) a Docetaxel-and/or-Ramucirumab therapy, more preferably (i) a Bevacizumab-and-XELOX therapy, (iii) a FOLFIRINOX therapy or a dose reduction regimen thereof, (iv) a Gemcitabine-and-nab-Paclitaxel therapy, (vii) a Docetaxel therapy, or (vi) a Docetaxel-and-Ramucirumab therapy, even more preferably (i) a Bevacizumab-and-XELOX therapy, (iii) a FOLFIRINOX therapy or a dose reduction regimen thereof, (iv) a Gemcitabine-and-nab-Paclitaxel therapy, or (vi) a Docetaxel-and-Ramucirumab therapy), an effective amount of an immune checkpoint inhibitor and an effective amount of an $EP_4$ antagonist to a patient in need of cancer treatment separately or simultaneously.

Unless otherwise defined, all the technical and scientific terms and all the abbreviations used in this specification have the meaning as normally understood by a person skilled in the art of the present invention.

The contents of all the patent documents and the non-patent documents and the contents of the reference documents explicitly cited in this specification are incorporated herein as a part of the specification.

## EXAMPLE

The present invention is described below in detail by way of Examples, but the present invention is not limited by the following descriptions.

As the $EP_4$ receptor antagonist represented by general formula (I), 4-[4cyano-2-({(2'R,4S)-6-[(propan-2-yl)carbamoyl]-2,3-dihydrospiro[1-benzopyran-4,1'-cyclopropane]-2'carbonyl}amino)phenyl]butanoic acid (Compound A) was used. Compound A can be produced according to a known method, for example, a method described in Example 2-13 in WO 2016/111347.

Example 1: An open-label, uncontrolled study in which combination of Compound A as primary treatment, Nivolumab, and XELOX plus Bevacizumab therapy as standard therapy was applied to patients with radically unresectable advanced or recurrent colonic/rectal cancer

The purpose of this study is to examine the tolerability, safety, and efficacy of a combination of Compound A, Nivolumab, and XELOX plus Bevacizumab therapy as a primary treatment in patients with radically unresectable advanced or recurrent colonic/rectal cancer. This trial can evaluate the effect of the combination of Compound A, Nivolumab, and a XELOX plus Bevacizumab therapy.

(1) Patients

Patients with radically unresectable advanced or recurrent colonic/rectal cancer.

(2) Patient selection criteria

At the time of registration, patients were selected, who had not been treated with a systemic antineoplastic agent for radically unresectable advanced or recurrent colonic/rectal cancer and who met all pre-determined patient selection criteria determined in view of the patient selection criteria in each trial performed so far for Compound A, Nivolumab and XELOX plus Bevacizumab therapy. When it became clear from the registration that the criteria were not satisfied before the first administration of the investigational new drug, the administration of the investigational new drug was not started and the study was discontinued.

(3) Patient Exclusion Criteria

At the time of registration, patients were excluded, who were considered to meet either patient exclusion criteria in each trial for Compound A, Nivolumab, and XELOX plus Bevacizumab therapy, or pre-determined patient exclusion criteria determined in consideration of patient selection precautions in each proper use guide or proper use information for Compound A, Nivolumab, and XELOX plus Bevacizumab therapy. When it became clear from the registration that the criteria were not satisfied before the first administration of the investigational new drug, the administration of the investigational new drug was not started and the study was discontinued.

(4) Dosage and Administration and Duration of Administration

[Compound A]

Compound A was orally administered once a day at a dose of 20 mg or 40 mg per administration, and the administration was continued until a predetermined criteria for discontinuation of administration related to Compound A was met. When Compound A, Nivolumab, and XELOX plus Bevacizumab therapy were administered on the same day, Compound A and Nivolumab were administered first, and Compound A and Nivolumab were then administered, and then XELOX plus Bevacizumab therapy was started.

[Nivolumab]

Nivolumab was intravenously administered at a dose of 360 mg over 30 minutes at 3-week intervals, and the administration was continued until predetermined administration discontinuation criteria for Nivolumab were met. The administration of Nivolumab was performed at least 14 days apart from the previous administration and on or after Day 15.

[XELOX plus Bevacizumab therapy]

Bevacizumab was administered intravenously at 7.5 mg/kg over 90 minutes. When tolerated well, Bevacizumab was administered intravenously over 60 minutes for the second time and over 30 minutes for the subsequent cycles. Oxaliplatin (130 mg/$m^2$ (body surface area)) was intravenously administered over 2 hours. Capecitabine (1000 mg/$m^2$) was orally administered twice a day after breakfast and after dinner, followed by oral administration for 14 days and rest for 7 days. Commercially available products were used as Bevacizumab, Oxaliplatin, and Capecitabine.

[Clinical Trial Schedule]

The study consisted of a screening phase, a treatment phase and a post-observation phase. The outline of the study schedule is shown in Fig. 1.

The screening period was within 28 days prior to the investigational new drug administration, and a principal investigator or a sub-investigator included patients who met the above inclusion criteria and who did not meet the above exclusion criteria and were determined to be eligible for the study.

The treatment phase was set to 1 cycle of 21 days, and the first day of administration of the investigational new drug was set to Day 1 of Cycle 1. The first day of each cycle after the second cycle was set to [21 X (the number of cycles) - 1) plus 1] days. Administration was started according to the dosage and administration described above for Compound A, Nivolumab, and XELOX plus Bevacizumab therapies, respectively, and continued according to the administration criteria, dose reduction criteria, and dosage at dose reduction for Compound A, Nivolumab, and XELOX plus Bevacizumab therapies. The time point at which the evaluation at the completion (discontinuation) of the administration of Compound A, Nivolumab, and XELOX plus Bevacizumab therapy was completed was defined as the end of the treatment phase. Among all patients who received the investigational new drug, patients who discontinued or terminated the administration of Compound A, Nivolumab, and XELOX plus Bevacizumab therapy performed evaluation at the completion (discontinuation) of the administration and shifted to the post-observation period. Follow-up investigation was performed after the end of the follow-up period.

[Criteria for Administration of Compound A and Nivolumab]

Patients had to be met all of the predefined administration criteria determined at the start of each dose in view of the administration criteria in the trials carried out to date for Compound A and Nivolumab. When any of these criteria was not met, the scheduled administration of Compound A and Nivolumab was withdrawn. However, even in a case where any of the criteria was not met, it was possible to continue the administration of Compound A and Nivolumab only when it was determined that the clinical symptoms determined to be caused by the progression of the disease were not deteriorated, the clinical benefit by continuing the administration was expected, and the administration of Compound A and Nivolumab was able to be safely continued in consideration of the baseline of the patient and the influence of the concomitant drug.

[Criteria for Discontinuing Administration of Compound A]

In the treatment phase, patients falling under any of the predetermined administration discontinuation criteria determined in consideration of the administration discontinuation criteria in the trials carried out so far for Compound A discontinue the administration of Compound A.

[Criteria for Discontinuing Administration of Nivolumab]

In the treatment phase, patients falling under any of the predetermined administration discontinuation criteria determined in consideration of the administration discontinuation criteria in the trials carried out so far for Nivolumab discontinue the administration of Nivolumab.

[Criteria for administration of XELOX plus Bevacizumab therapy]

Patients had to be met all predetermined administration criteria determined at the start of each dose in view of the administration criteria in previous trials conducted for XELOX plus Bevacizumab therapy. The administration was postponed until the clinical laboratory test value on the administration scheduled date was recovered to a state satisfying the conditions of the criteria, and the administration was performed after confirming that the administration did not correspond to the contraindication of each drug.

[XELOX plus Bevacizumab therapy dose reduction and dose reduction criteria]

The dose reduction was performed according to the latest package insert.

[Criteria for discontinuation of XELOX plus Bevacizumab therapy]

In the treatment phase, for patients who met any of the predetermined discontinuation criteria determined in consideration of the discontinuation criteria in each trial conducted to date for XELOX plus Bevacizumab therapy, the administration of the XELOX plus Bevacizumab therapy was discontinued.

[Evaluation Criteria of Efficacy]

(Diagnostic imaging)

CT/nuclear magnetic resonance imaging (MRI) and the like of the chest, abdomen and pelvis were performed. In addition, in the screening period (within 28 days before the administration of the investigational new drug), when brain metastasis was suspected due to clinical symptoms or the like, CT/MRI imaging, fluorodeoxyglucose-positron emission tomography (FDG-PET) (or bone scintigraphy) or the like in the head was performed to confirm the presence or absence of brain metastasis or bone metastasis.
A principal investigator or a sub-investigator measured a tumor diameter of a target lesion according to RECIST Guideline 1.1 to determine the antitumor effect. Baseline assessment was performed using a latest imaging test within 28 days prior to the administration of the investigational new drug and confirmed about the presence of one or more measurable lesions as defined in RECIST Guideline 1.1. Diagnostic imaging in the treatment phase was measured at 6-week intervals from Day 1 of the first cycle to Week 6 (plus 7 days) to Week 54 (plus 7 days), and thereafter at 12-week intervals (± 7 days).
The total effect and the best total effect were diagnostic imaging results evaluated based on the RECIST Guideline 1.1.

(Evaluation items)

(1) Objective response rate (ORR), (2) Disease control rate (DCR), (3) Overall survival period (OS), (4) Progression-free survival period (PFS), (5) Duration of response (DOR), (6) Time to response (TTR), (7) Best overall response (BOR), (8) Changing rate in the sum of tumor diameter in target lesion, (9) Maximum changing rate in the sum of tumor diameter in target lesion, and (10) Transition of tumor markers (CEA and CA 19-9)

(1) Objective response rate

The term "objective response rate" refers to the percentage of patients whose best overall response was determined to be a complete response (hereinafter abbreviated as "CR") or a partial response (hereinafter abbreviated as "PR").

(2) Disease control rate

The term "disease control rate" refers to the percentage of patients whose best overall response was determined to be CR, PR or stable (stable disease, hereinafter abbreviated as "SD").

(3) Overall survival period

The overall survival period was calculated in accordance with the following formula.

Overall survival period (days) = (the date of death due to any cause) - (the date of starting administration of investigational new drug) + 1

Note that, for patients who became untraceable or who did not die by the data cutoff date, the final survival confirmation date was defined as a termination date.

(4) Progression-free survival period

The progression-free survival period was calculated in accordance with the following formula.
Progression-free survival period (days) = (the date when the overall effect was determined as PD or the earlier of the date of death due to any cause) - (the date of start of administration of investigational new drug) + 1
Note that, for a patient whose overall effect has not been determined to have progressed (progressive disease, hereinafter abbreviated as "PD") and who has not died, the date on which the last evaluable diagnostic imaging was performed was defined as a termination date. For a patient who had not undergone evaluable diagnostic imaging and who had not died, the date on which the administration of the investigational new drug was started was defined as a termination date. For patient who received post-treatment for cancer before the overall effect was determined to be PD

or died, the day on which the last evaluable imaging diagnosis before the start of post-treatment for cancer was performed was defined as a termination date.

(5) Duration of response

The duration of response was calculated in accordance with the following formula.
Duration of response (days) = (the day when the first total response was determined to be PD after the confirmation of response or the earlier day of death due to any cause) - (the date of first determination of confirmed CR or PR) plus 1
A patient to be evaluated was a patient who showed CR or PR determined through the clinical trial.

(6) Time to response

The time to response was calculated in accordance with the following equation.

Time to response (days) = (the date of first determination of confirmed CR or PR) – (the date of start of administration of investigational new drug) + 1

(7) Changing rate in the sum of tumor diameter in target lesion

For a patient having a target lesion, changing rate in the sum of tumor diameter in target lesion was calculated using the following calculation formula. However, the changing rate in the sum of tumor diameter in target lesion after the post-treatment was performed was not calculated.

[Mathematical formula 1]

Changing rate (%) = ([the sum of tumor diameters at each evaluation time point] - [the sum of tumor diameters before administration])/ (the sum of tumor diameters before administration) $\times$ 100

(8) maximum changing rate in the sum of tumor diameter in target lesion

The maximum changing rate in the sum of tumor diameter in target lesion was defined as a changing rate at the time point when the sum of tumor diameter of the target lesion was the smallest. However, the sum of the tumor diameter of the target lesion after the total effect was determined to be PD or after the post-treatment was performed was not used for calculating the maximum changing rate.

[Mathematical formula 2]

Maximum changing rate (%) = ([the sum of smallest tumor diameter after administration] - [the sum of tumor diameter before administration])/ (the sum of tumor diameter before administration) $\times$ 100

[Evaluation items of safety]

The following items were measured, examined and investigated by the principal investigator and the like at a predetermined time.

(1) Dose limiting toxicity, (2) Adverse events, (3) Clinical examination (hematological tests, blood biochemical tests, hormonal tests, blood coagulation system tests, urine qualitative tests, immunological tests), (4) Vital signs (systolic blood pressure /diastolic blood pressure, pulse rate, body temperature), transcutaneous oxygen saturation ($SpO_2$), body weight, (5) 12 lead electrocardiogram, (6) ECOG Performance Status, (7) Chest X-ray, (8) Ophthalmologic

examination (visual acuity test, tonometry, slit lamp microscopy, and (9) CT examination

[Results of Efficacy Evaluation]

At the time when 10 months have elapsed since the first patientwas registered, 2 cases were determined to be PR.

Example 2: An open-label, uncontrolled study in which Compound A or a combination of Compound A and Nivolumab was used as a preoperative adjuvant therapy after a preoperative chemoradiotherapy for curatively resectable locally advanced rectal cancer

The purpose of this study was to examine the efficacy, pharmacokinetics and safety of Compound A administered alone and/or Compound A and Nivolumab administered in combination as a preoperative adjuvant therapy after a preoperative chemoradiotherapy (CRT) for curatively resectable locally advanced rectal cancer. The trial could evaluate the effect of Compound A and/or a combination of Compound A and Nivolumab as a preoperative adjuvant therapy.

(1) Target

Curatively Resectable Locally Advanced Rectal Cancer

(2) Patient selection criteria

At the time of registration, patients were selected who met all of the pre-determined patient selection criteria determined in consideration of the patient selection criteria in each trial performed to date for Nivolumab and Compound A. When it was found that the criteria were not satisfied before the first administration of the investigational new drug from the registration, the administration of the investigational new drug was not started and the trial was discontinued.
As the preoperative CRT, the investigational new drug was administered to a patient who was treated and satisfied the following conditions.

- 45 Gy/25 times of irradiation to the pelvic cavity and 5.4 Gy/3 times of boost irradiation to the primary lesion were performed.
- the administration of Capecitabine was started at a dose of 1,650 mg/$m^2$ (825 mg/$m^2$ twice a day) per day. The starting dose was determined based on Method D of dosage and administration of Xeloda (registered trademark) tablet 300.
- The oral administration of Capecitabine was performed in a period corresponding to 75% of 28 times which was the number of times of irradiation of radioactive ray in combination with the irradiation of radioactive ray (for example, in the case of 50.4 Gy/28 times of irradiation, oral administration for 21 days or 42 times or more as oral administration in the morning and evening). The presence or absence of dose reduction did not matter.

The investigational new drug was administered to patients who could start investigational new drug administration within 14 days after the end of treatment with Capecitabine and radiation as preoperative CRT.
The investigational new drug was administered to a patient who was not found to have distant metastasis by diagnostic imaging after the end of preoperative CRT and could be curatively resected. Note that the diagnostic imaging before registration was performed on the basis of an image captured within a range from 14 days before the end of the preoperative CRT to the registration date.

(3) Patient Exclusion Criteria

At the time of registration, patients were excluded who were considered to meet any of the patient exclusion criteria in each trial for Compound A and Nivolumab or the predetermined patient exclusion criteria determined in consideration of patient selection precautions in each proper use guide or proper use information for Compound A and Nivolumab. When it became clear from the registration that the criteria were not satisfied before the first administration of the investigational new drug, the administration of the investigational new drug was not started and the trial was discontinued.

(4) Dosage and Administration and Duration of Administration

[Compound A]

Compound A was orally administered at a dose of 40 mg per administration once a day, and the administration was continued until a predetermined criteria for discontinuing administration related to Compound A were satisfied.

[Nivolumab]

Nivolumab (240 mg) was intravenously administered over 30 minutes at 2-week intervals, and the administration was continued until a predetermined administration, discontinuation criteria for Nivolumab were satisfied. Nivolumab administration was performed on day 11 or later, at least 10 days apart from the day following the previous administration.

[Clinical Trial Schedule]

The trial consisted of a screening phase, a treatment phase, a perioperative phase and a post-observation phase. An overview of the trial design is shown in Fig. 2.

In the screening phase, patients who had curatively resectable locally advanced rectal cancer that has undergone preoperative CRT and were determined to be eligible for this trial are registered and transitioned to the treatment phase.

In the treatment phase, the administration of the investigational new drug was started within 14 days after the end of preoperative CRT. A group (cohort 1) in which Compound A (40 mg once a day (QD), oral) was administered alone for a certain period of time and then Compound A (40 mg, QD, oral) and Nivolumab (240 mg, 2-week interval (Q2W), IV) were administered in combination for a certain period of time and a group (cohort 2) in which Compound A (40 mg, QD, oral) and Nivolumab (240 mg, Q2W, IV) were administered in combination for a certain period of time were provided. After completion of incorporation into Cohort 1, incorporation into Cohort 2 is started. After completion of the administration of the investigational new drug, all patients were subjected to tests at the completion (discontinuation) of the administration, and transition from the treatment phase to the perioperative phase was performed.

In the perioperative period, in principle, surgery was performed after 7 days from the last day of administration of Compound A and after 14 days from the last day of administration of Nivolumab, and within 14 weeks after the end of preoperative CRT. Regarding the timing of the surgery, the condition of the patient was taken into consideration as the highest priority, and this does not apply to the case where surgery was urgently required, such as exacerbation of the primary disease, and the case where surgery needed to be postponed, such as treatment for adverse events. In the post-observation period, various final tests were performed with any later time point of 30 days after the end of the surgical treatment or the time point at which the principal investigator or the sub-investigator determined that the complications due to the surgery were ameliorated as the starting date. However, in a case where the post-treatment for the primary disease (rectal cancer) was started due to clinical necessity by the starting date, the final test was performed before starting the post-treatment. Thereafter, a follow-up investigation was performed.

[Criteria for Administration of Compound A and Nivolumab]

Patients had to meet all predetermined administration criteria determined at the start of each dose in view of the administration criteria in previous trials conducted for Compound A and Nivolumab. When any of these criteria was not met, the scheduled administration of Compound A and Nivolumab was withdrawn. However, administration of the investigational new drug might be allowed to continue if the principal investigator or sub-investigator determined that continued administration was expected to be useful in light of the balance of benefit and risk.

[Criteria for Discontinuing Administration of Compound A]

In the treatment period, patients falling under any of the predetermined administration discontinuation criteria determined in consideration of the administration discontinuation criteria in the trials carried out so far for Compound A discontinue the administration of Compound A.

[Criteria for Discontinuing Nivolumab Administration]

In the treatment phase, patients falling under any of the predetermined administration discontinuation criteria determined in consideration of the administration discontinuation criteria in the trials carried out so far for Nivolumab discontinue the administration of Nivolumab.

[Evaluation items of efficacy]

(Diagnostic imaging/endoscopic test)

Diagnostic imaging and endoscopic test determined the presence or absence of tumor exacerbation from colonoscopy, CT or MRI imaging. Timing of implementation was not affected by investigational new drug rest. In addition, in the screening phase (within 14 days before administration of the investigational new drug), when brain metastasis is suspected due to clinical symptoms or the like, CT/MRI imaging, FDG-PET (or bone scintigraphy) or the like in the head was performed to confirm the presence or absence of brain metastasis or bone metastasis.

(Evaluation items)

(1) Pathological complete response (pCR) rate, (2) Major pathological response (MPR) rate, (3) Overall survival period (OS), (4) Relapse-free survival period (RFS), (5) Event-free survival period (EFS), and (6) transition of tumor markers (CEA and CA 19-9) (1) pCR rate

The proportion of patients determined as having AJCC Tumor regression grade 0 by a pathologist at each medical institution was calculated as a pCR rate. Tumor cells in which viable tumor cells were not observed not only in the primary lesion but also in the regional lymph nodes (ypT0N0) were defined as pCR.

(2) MPR rate

The proportion of patients determined as having AJCC Tumor regression grade 0 or 1 by a pathologist of each medical institution was calculated as an MPR rate.

[Evaluation items of surgery]

(1) Curative resection implementation rate, (2) Treatment completion rate until curative resection, (3) Curative resection implementation rate during the period specified in the protocol, and (4) Macroscopic evaluation of resected specimens

[Evaluation items of safety]

The following items were measured, examined and investigated by the principal investigator and the like at a predetermined time.

(1) Adverse events, (2) Perioperative complications, (3) Clinical examination (hematological tests, blood biochemical tests, hormonal tests, blood coagulation system tests, urine qualitative tests, immunological tests), (4) Vital signs (systolic/diastolic blood pressure, pulse rate, body temperature), transcutaneous oxygen saturation ($SpO_2$), body weight, (5) 12 lead electrocardiogram, (6) ECOG Performance Status, (7) Chest X-ray, (8) Ophthalmic examination (visual acuity test, tonometry, slit lamp microscopy, and (9) CT examination

[Results of Efficacy Evaluation]

At the time when 10 months have elapsed since the registration of the first patient, 3 cases were determined to be pCR.

Example 3: An open-label, uncontrolled study in which a patient with pancreatic cancer with distant metastasis was treated with a combination of Compound A as a primary treatment, Nivolumab, and mFFX therapy or GnP therapy as a standard therapy

This study aims to study the tolerability, safety and efficacy of a combination of Compound A as a primary treatment, Nivolumab and mFFX therapy or GnP therapy in pancreatic cancer patients with distant metastases. The trial could evaluate the effect of the combination with Compound A, Nivolumab and mFFX therapy or GnP therapy.

(1) Patients

Pancreatic cancer patients with distant metastases

(2) Patient selection criteria

At the time of registration, patients who had not been treated with systemic antineoplastic agents for pancreatic cancer with distant metastases and who met all pre-determined patient selection criteria determined in view of the patient selection criteria in each trial performed so far for Compound A, Nivolumab and mFFX therapy or GnP therapy were selected. When it was found that the criteria were not satisfied before the first administration of the investigational new drug from the registration, the administration of the investigational new drug was not started and the study was discontinued.

(3) Patient Exclusion Criteria

At the time of registration, patients who were considered to meet either patient exclusion criteria in each trial for Compound A, Nivolumab and mFFX therapy or GnP therapy, or predefined patient exclusion criteria determined in consideration of patient selection precautions in each guide for appropriate use or information on appropriate use for Compound A, Nivolumab and mFFX therapy or GnP therapy were excluded. When it was found that the criteria were not satisfied before the first administration of the investigational new drug from the registration, the administration of the investigational new drug was not started and the study was discontinued.

(4) Dosage and Administration and Duration of Administration

[Compound A]

Compound A was orally administered once a day at a dose of 20 mg or 40 mg per administration, wherein the administration was continued until predetermined criteria for discontinuation of administration related to Compound A was met. Note that when Compound A, Nivolumab and the mFFX therapy or GnP therapy were administered on the same day, Compound A and Nivolumab were administered first, and after Compound A and Nivolumab were administered, followed by starting of the mFFX therapy or GnP therapy.

[Nivolumab]

Nivolumab was administered intravenously at a dose of 480 mg over 30 minutes at 4-week intervals, and the administration was continued until a predetermined administration, discontinuation criteria for Nivolumab was met. The administration of Nivolumab was performed at least 24 days apart from the previous administration on and after Day 25.

[mFFX therapy]

Oxaliplatin was administered intravenously at a dose of 85 mg/$m^2$ (body surface area) over 2 hours. Irinotecan was administered intravenously at a dose of 150 mg/$m^2$ (body surface area) over 90 minutes. Levofolinate was intravenously administered at a dose of 200 mg/$m^2$ (body surface area) over 2 hours. Fluorouracil was intravenously administered at a dose of 2400 mg/$m^2$ (body surface area) over 46 hours. The series of the administrations were performed at 2-week intervals. As Oxaliplatin, Irinotecan, Levofolinate, and Fluorouracil, commercially available products were used.

[GnP therapy]

Gemcitabine was administered intravenously at a dose of 1000 mg/$m^2$ (body surface area) over 30 minutes, and the administration was withdrawn for at least 6 days. Nab-paclitaxel was administered intravenously at a dose of 125 mg/$m^2$ (body surface area) over 30 minutes, and the administration was withdrawn for at least 6 days. The series of the administrations were performed at 1-week intervals, continued for three weeks, and then the administration was withdrawn for one week.

[Clinical Trial Schedule]

The study consisted of a screening phase, a treatment phase and a post-observation phase. The outline of the study schedule is shown in Fig. 3.

The screening phase was within 28 days prior to the investigational new drug administration, and a principal investigator or a sub-investigator included patients who met the above selection criteria and who met the above exclusion criteria and were determined to be eligible for the study.

The treatment phase was set to 1 cycle of 28 days, and the first day of administration of the investigational new drug was set to Day 1 of Cycle 1. The first day of each cycle after the second cycle was [28 × (the number of cycles) - 1) + 1] days. Administration was started according to the dosage and administration described above for Compound A, Nivolumab and the mFFX therapy or GnP therapy, respectively, and continued according to the administration criteria, dose reduction criteria and administration at the dose reduction for Compound A, Nivolumab and the mFFX therapy or GnP therapy. The time point at which the evaluation at the completion (discontinuation) of the administration of Compound A, Nivolumab and mFFX therapy or GnP therapy was completed was defined as the end of the treatment period. Among all patients s who received the investigational new drug, patients for whom the administration of Compound A, Nivolumab, and mFFX therapy or GnP therapy was discontinued or terminated were subjected to the evaluation at the completion (discontinuation) of the administration and shift to the post-observation phase. Follow-up investigation was performed after the end of the post-observation phase.

[Criteria for Administration of Compound A and Nivolumab]

Patients had to meet all predetermined administration criteria determined at the start of each dose in view of the administration criteria in previous trials conducted for Compound A and Nivolumab. When any of these criteria was not met, the scheduled administration of Compound A and Nivolumab was withdrawn. However, even in a case where any of the criteria was not met, it was possible to continue the administration of Compound A and Nivolumab only when it was determined that the clinical symptoms determined to be caused by the progression of the disease were not deteriorated, the clinical benefit by continuing the administration was expected, and the administration of Compound A and Nivolumab was able to be safely continued in consideration of the baseline of the patient and the influence of the concomitant drug.

[Criteria for Discontinuing Administration of Compound A]

In the treatment phase, patients falling under any of the predetermined administration discontinuation criteria determined in consideration of the administration discontinuation criteria in the trials carried out so far for Compound A discontinue the administration of Compound A.

[Criteria for Discontinuing Nivolumab Administration]

In the treatment phase, patients falling under any of the predetermined administration discontinuation criteria determined in consideration of the administration discontinuation criteria in the trials carried out so far for Nivolumab discontinue the administration of Nivolumab.

[Criteria for administration of mFFX Therapy]

Patients had to meet all predetermined administration criteria determined at the start of each dose, in consideration of the administration criteria in previously conducted trials for mFFX therapy. The administration was postponed until the clinical laboratory test value on the administration scheduled date was recovered to a state satisfying the conditions of the criteria, and the administration was performed after confirming that the administration did not correspond to the contraindication of each drug.

[Criteria for Dose Reduction and Dose Reduction of mFFX Therapy]

The dose reduction was performed according to the latest package insert.

[Criteria for Discontinuing mFFX Therapy]

In the treatment phase, patients falling under any of the predetermined administration discontinuation criteria determined in consideration of the administration discontinuation criteria in each clinical trial performed so far for the mFFX therapy discontinued the administration of the mFFX therapy.

[Criteria for Administration of GnP Therapy]

At the start of each administration, patients had to meet all predetermined administration criteria determined in view of the administration criteria in the trials carried out so far for GnP therapy. The administration was postponed until the clinical laboratory test value on the administration scheduled date was recovered to a state satisfying the conditions of the criteria, and the administration was performed after confirming that the administration did not correspond to the contraindication of each drug.

[Criteria for Dose Reduction and Dose Reduction of GnP Therapy]

The dose reduction was performed according to the latest package insert.

[Criteria for discontinuing GnP therapy]

In the treatment phase, patients falling under any of the predetermined administration discontinuation criteria determined in consideration of the administration discontinuation criteria in the trials carried out so far for GnP therapy discontinue the administration of GnP therapy.

[Evaluation Criteria of Efficacy]

(Diagnostic imaging)

CT imaging/nuclear magnetic resonance imaging (MRI) and the like of the chest, abdomen and pelvis were performed. In addition, in the screening phase (within 28 days before the administration of the investigational new drug), when brain metastasis was suspected due to clinical symptoms or the like, CT imaging /MRI, fluorodeoxyglucose-positron emission tomography (FDG-PET) (or bone scintigraphy) or the like in the head was performed to confirm the presence or absence of brain metastasis or bone metastasis.

A principal investigator or a sub-investigator measured a tumor diameter of a target lesion according to RECIST Guideline 1.1 to determine the antitumor effect. Baseline assessment was performed using a current imaging study within 28 days prior to the administration of the investigational new drug and confirmed about the presence of one or more measurable lesions as defined in RECIST Guideline 1.1. Imaging diagnosis in the treatment phase was performed from Day 1 to 8 weeks (plus 7 days) after the first cycle, and the tumor diameter was measured.

The overall response and the best overall response were diagnostic imaging results evaluated based on the RECIST Guideline 1.1.

(Evaluation items)

(1) Response rate (ORR), (2) Disease control rate (DCR), (3) Overall survival period (OS), (4) Progression-free survival period (PFS), (5) Duration of response (DOR), (6) Time to response (TTR), (7) Best overall response (BOR), (8) Changing rate in the sum of tumor diameter in target lesion, (9) Maximum changing rate in the sum of tumor diameter in target lesion, (10) Transition of tumor markers (CEA and CA 19-9)

(1) Response rate

The term "response rate" refers to the percentage of patients whose best overall response was determined to be CR or PR.

(2) Disease control rate

The term "disease control rate" refers to the percentage of patients whose best overall response was determined to be CR, PR or SD.

(3) Overall survival period

The overall survival period was calculated in accordance with the following formula.

Overall survival period (days) = (the date of death due to any cause) - (the date of starting administration of investigational new drug) + 1

Note that, for patients who became untraceable or who did not die by the data cutoff date, the final survival

confirmation date was defined as a termination date.

(4) Progression-free survival period

The progression-free survival period was calculated in accordance with the following formula.

Progression-free survival period (days) = (the date when the overall effect was determined as PD or the earlier of the date of death due to any cause) - (the date of start of administration of investigational new drug) + 1

Note that, for a patient whose overall effect has not been determined to have progressed (progressive disease, hereinafter abbreviated as "PD") and who has not died, the date on which the last evaluable diagnostic imaging was performed was defined as a termination date. For a patient who had not undergone evaluable diagnostic imaging and who had not died, the date on which the administration of the investigational new drug was started was defined as a termination date. For patient who received post-treatment for cancer before the overall effect was determined to be PD or died, the day on which the last evaluable imaging diagnosis before the start of post-treatment for cancer was performed was defined as a termination date.

(5) Duration of Response

The duration of response was calculated in accordance with the following formula.

Duration of response (days) = (the day on which the first total response was determined to be PD after the confirmation of response or the earlier day of death due to any cause) - (the date of first determination of confirmed CR or PR) + 1

A patient to be evaluated was a patient who showed CR or PR determined through the clinical trial.

(6) Time to Response

The time to response was calculated in accordance with the following formula.

Time to response (days) = (the date of first determination of confirmed CR or PR) - (the date of start of administration of investigational new drug) + 1

(7) Changing rate in the sum of tumor diameter in target lesion

For a patient having a target lesion, changing rate in the sum of tumor diameter in target lesion was calculated using the following calculation formula. However, the changing rate in the sum of tumor diameter in target lesion after the post-treatment was performed was not calculated.

[Mathematical formula 3]

Changing rate (%) = ([the sum of tumor diameters at each evaluation time point] – [the sum of tumor diameters before administration])/ (the sum of tumor diameters before administration) × 100

(8) Maximum changing rate in the sum of tumor diameter in target lesion

The maximum changing rate in the sum of tumor diameter in target lesion was defined as a changing rate at the time point when the sum of tumor diameter of the target lesion was the smallest. However, the sum of the tumor diameter of the target lesion after the total effect was determined to be PD or after the post-treatment was performed was not used for calculating the maximum changing rate.

[Mathematical formula 4]

$$\text{Maximum changing rate (\%)} = ([\text{the sum of smallest tumor diameter after administration}] - [\text{the sum of tumor diameter before administration}]) / (\text{the sum of tumor diameter before administration}) \times 100$$

(9) Transition of changing rate of the tumor markers (CEA and CA 19-9)

The changing rate of the tumor marker is calculated using the following calculation formula. However, the changing rate of the tumor marker after the post-treatment was not calculated.

[Mathematical formula 5]

$$\text{Changing rate (\%)} = ([\text{the tumor marker at each evaluation time point}] - [\text{the tumor marker before administration}]) / (\text{the tumor marker before administration}) \times 100$$

[Evaluation items of safety]

The following items were measured, examined and investigated by the principal investigator and the like at a predetermined time.

(1) Dose-limiting toxicity (DLT), (2) Adverse events, (3) Clinical examination (hematological tests, blood biochemical tests, hormonal tests, blood coagulation system tests, urine qualitative tests, immunological tests), (4) Vital signs (systolic/diastolic blood pressure, pulse rate, body temperature), transcutaneous oxygen saturation ($SpO_2$), body weight, (5) 12 lead electrocardiogram, (6) ECOG Performance Status, (7) Chest X-ray, (8) Ophthalmologic examination (visual acuity test, tonometry, slit lamp microscopy, and (9) CT examination

[Results of Efficacy Evaluation]

Regarding the combination use of Compound A, Nivolumab, and mFFX therapy, one case was determined to be PR at the time point of 9 months after the registration of the first patient.

In addition, for combination of Compound A, Nivolumab, and GnP therapy, there were two cases judged to be PR at the time point of 9 months after registration of the first patient.

Example 4: An open-label, uncontrolled study involving patients who had advanced or recurrent non-small cell lung cancer refractory to a combination therapy including an anti-PD-1 antibody or an anti-PD-L1 antibody, and a platinum preparation, wherein Compound A, Nivolumab, and the standard therapy of Docetaxel and Ramucirumab were used in combination as a secondary therapy.

The purpose of this study is to examine the tolerability, safety, and efficacy of a combination of Compound A, Nivolumab, Docetaxel, and Ramucirumab as a secondary therapy in non-small cell lung cancer patients who had progressed or relapsed refractory to a combination therapy including an anti-PD-1 antibody or an anti-PD-L1 antibody and a platinum preparation. This trial can evaluate the effect of combination of Compound A, Nivolumab, Docetaxel, and Ramucirumab.

(1) Patients

Patients with stage IV or recurrent non-small cell lung cancer

(2) Patient selection criteria

At the time of registration, patients who received a combination therapy including an anti-PD-1 antibody or anti-PD-L1 antibody as a primary therapy and a platinum preparation as a primary treatment, who were found to have advanced or recurrent non-small cell lung cancer refractory to the combination therapy, and who met all pre-determined patient selection criteria determined in consideration of patient selection criteria in each of the previous trials for compounds A, Nivolumab, Docetaxel, and Ramucirumab were selected. When it was found that the criteria were not satisfied before the first administration of the investigational new drug from the registration, the administration of the investigational new drug was not started and the study was discontinued.

(3) Patient Exclusion Criteria

At the time of registration, patients who were considered to meet any of the patient exclusion criteria in each trial for Compound A, Nivolumab, Docetaxel, and Ramucirumab, or the predetermined patient exclusion criteria determined in consideration of patient selection precautions in each guide for appropriate use or information on appropriate use for Compound A, Nivolumab, and Docetaxel and Ramucirumab were excluded. When it was found that the criteria were not satisfied before the first administration of the investigational new drug from the registration, the administration of the investigational new drug was not started and the study was discontinued.

(4) Dosage and Administration and Duration of Administration

[Compound A]

Compound A was orally administered once a day at a dose of 20 mg or 40 mg per administration, and the administration was continued until the predetermined criteria for discontinuation of administration related to Compound A were met. When Compound A, Nivolumab, Docetaxel, and Ramucirumab were administered on the same day, Compound A and Nivolumab were administered first, and then the administration of Docetaxel and Ramucirumab was started.

[Nivolumab]

Nivolumab was intravenously administered at a dose of 360 mg over about 30 minutes at 3-week intervals, and the administration was continued until the predetermined administration discontinuation criteria for Nivolumab were satisfied. The administration of Nivolumab was performed at least 14 days apart from the previous administration and on or after Day 15.

[Docetaxel and Ramucirumab]

The dosage and administration of Docetaxel and Ramucirumab followed the procedure of the implementing medical institution. As for recommended dosage and administration, 60 $mg/m^2$ (body surface area) of Docetaxel was intravenously administered over 60 minutes or longer at 3-week intervals. Ramucirumab was administered intravenously at a dose of 10 mg/kg over 60 minutes at 3-week intervals. The term "at 3-week intervals" means that Docetaxel or Ramucirumab is administered between Day 22 and Day 29 with the previous administration date as Day 1. Note that commercially available products were used as Docetaxel and Ramucirumab, respectively.

[Clinical Trial Schedule]

The clinical trial consisted of a screening phase, a treatment phase and a post-observation phase. The outline of the clinical trial schedule is shown in Fig. 4.

The screening phase was within 28 days prior to the investigational new drug administration, and a principal investigator or a sub-investigator included patients who met the above selection criteria and who met the above exclusion criteria and were determined to be eligible for the clinical trial.

The treatment phase was set to 1 cycle of 21 days, and the first day of administration of the investigational new drug was set to Day 1 of Cycle 1. The first day of each cycle after the second cycle was [21 × ((the number of cycles) - 1) + 1] days. The administration of Compound A, Nivolumab, Docetaxel, and Ramucirumab was started according to the above dosage and administration, and the administration was continued according to the administration criteria, the dose reduction criteria, and the dose at the time of dose reduction for Compound A, Nivolumab, Docetaxel, and Ramucirumab. The time point at which the evaluation at the completion (discontinuation) of the administration of Compound A, Nivolumab, Docetaxel, and Ramucirumab was completed was defined as the end of the treatment phase. Among all patients who received the investigational new drug, patients for whom the administration of Compound A, Nivolumab, Docetaxel, and Ramucirumab was discontinued or terminated are subjected to the evaluation at the completion (discontinuation) of the administration, and shifted to the post-observation phase. Follow-up investigation was performed after the end of the post-observation phase.

[Criteria for Administration of Compound A and Nivolumab]

Patients had to meet all predetermined administration criteria determined at the start of each administration in view of the administration criteria in previous trials conducted for Compound A and Nivolumab. When any of these criteria was not met, the scheduled administration of Compound A and Nivolumab was withdrawn. However, even in a case

where any of the criteria was not met, it was possible to continue the administration of Compound A and Nivolumab only when it was determined that the clinical symptoms determined to be caused by the progression of the disease were not deteriorated, the clinical benefit by continuing the administration was expected, and the administration of Compound A and Nivolumab was able to be safely continued in consideration of the baseline of the patient and the influence of the concomitant drug.

[Criteria for Discontinuing Administration of Compound A]

In the treatment phase, patients falling under any of the predetermined administration discontinuation criteria determined in consideration of the administration discontinuation criteria in the trials carried out so far for Compound A discontinue the administration of Compound A.

[Criteria for Discontinuing Nivolumab Administration]

In the treatment phase, patients falling under any of the predetermined administration discontinuation criteria determined in consideration of the administration discontinuation criteria in the trials carried out so far for Nivolumab discontinue the administration of Nivolumab.

[Administration Criteria of Docetaxel and Ramucirumab]

The administration was performed according to the latest package insert.

[Criteria for dose reduction and reduction of Docetaxel and Ramucirumab]

The dose reduction was performed according to the latest package insert.

[Criteria for Discontinuing Docetaxel and Ramucirumab]

The administration was discontinued according to the latest package insert.

[Evaluation Criteria of Efficacy]

(Diagnostic imaging)

CT imaging/nuclear magnetic resonance imaging (MRI) and the like of the chest, abdomen and pelvis were performed. In addition, in the screening phase (within 28 days before the administration of the investigational new drug), when brain metastasis was suspected due to clinical symptoms or the like, CT imaging /MRI, fluorodeoxyglucose-positron emission tomography (FDG-PET) (or bone scintigraphy) or the like in the head was performed to confirm the presence or absence of brain metastasis or bone metastasis.

A principal investigator or a sub-investigator measured a tumor diameter of a target lesion according to RECIST Guideline 1.1 to determine the antitumor effect. Baseline assessment was performed using a current imaging study within 28 days prior to the administration of the investigational new drug and confirmed about the presence of one or more measurable lesions as defined in RECIST Guideline 1.1. Imaging diagnosis in the treatment phase was performed at 6-week intervals (± 7 days) from Day 1 of the first cycle to Week 54, and thereafter at 12-week intervals (± 7 days), and the tumor diameter was measured.

The overall response and the best overall response were diagnostic imaging results evaluated based on the RECIST Guideline 1.1.

(Evaluation items)

(1) Response rate (ORR), (2) Disease control rate (DCR), (3) Overall survival (OS), (4) Progression-free survival (PFS), (5) Duration of response (DOR), (6) Time to response (TTR), (7) Best overall response (BOR), (8) Changing rate in the sum of tumor diameter in target lesion, (9) Maximum changing rate in the sum of tumor diameter in target lesion, (10) Transition of tumor marker

(1) Response rate

The term "response rate" refers to the percentage of patients whose best overall response was determined to

be CR or PR.

(2) Disease control rate

The term "disease control rate" refers to the percentage of patients whose best overall response was determined to be CR, PR or SD.

(3) Overall survival period

The overall survival period was calculated in accordance with the following formula.

Overall survival period (days) = (the date of death due to any cause) - (the date of starting administration of investigational new drug) + 1

Note that, for patients who became untraceable or who did not die by the data cutoff date, the final survival confirmation date was defined as a termination date.

(4) Progression-free survival period

The progression-free survival period was calculated in accordance with the following formula.

Progression-free survival period (days) = (the date when the overall effect was determined as PD or the earlier of the date of death due to any cause) - (the date of start of administration of investigational new drug) + 1

Note that, for a patient whose overall effect has not been determined to have progressed (progressive disease, hereinafter abbreviated as "PD") and who has not died, the date on which the last evaluable diagnostic imaging was performed was defined as a termination date. For a patient who had not undergone evaluable diagnostic imaging and who had not died, the date on which the administration of the investigational new drug was started was defined as a termination date. For patient who received post-treatment for cancer before the overall effect was determined to be PD or died, the day on which the last evaluable imaging diagnosis before the start of post-treatment for cancer was performed was defined as a termination date.

(5) Duration of Response

The duration of response was calculated in accordance with the following formula.

Duration of response (days) = (the day on which the first total response was determined to be PD after the confirmation of response or the earlier day of death due to any cause) - (the date of first determination of confirmed CR or PR) + 1

A patient to be evaluated was a patientwho showed CR or PR determined through the clinical trial.

(6) Time to Response

The time to response was calculated in accordance with the following formula.

Time to response (days) = (the date of first determination of confirmed CR or PR) – (the date of start of administration of investigational new drug) + 1

(7) Changing rate in the sum of tumor diameter in target lesion

For a patient having a target lesion, changing rate in the sum of tumor diameter in target lesion was calculated using the following calculation formula. However, the changing rate in the sum of tumor diameter in target lesion after the post-treatment was performed was not calculated.

[Mathematical formula 6]

$$\text{Changing rate (\%)} = (\text{[the sum of tumor diameters at each evaluation time point]} - \text{[the sum of tumor diameters before administration]}) / (\text{the sum of tumor diameters before administration}) \times 100$$

(8) Maximum changing rate in the sum of tumor diameter in target lesion

The maximum changing rate in the sum of tumor diameter in target lesion was defined as a changing rate at the time point when the sum of tumor diameter of the target lesion was the smallest. However, the sum of the tumor diameter of the target lesion after the total effect was determined to be PD or after the post-treatment was performed was not used for calculating the maximum changing rate.

[Mathematical formula 7]

$$\text{Maximum changing rate (\%)} = (\text{[the sum of smallest tumor diameter after administration]} - \text{[the sum of tumor diameter before administration]}) / (\text{the sum of tumor diameter before administration}) \times 100$$

(9) Transition of changing rate of the tumor markers

The changing rate of the tumor marker is calculated using the following calculation formula. However, the changing rate of the tumor marker after the post-treatment was not calculated.

[Mathematical formula 8]

$$\text{Changing rate (\%)} = (\text{[the tumor marker at each evaluation time point]} - \text{[the tumor marker before administration]}) / (\text{the tumor marker before administration}) \times 100$$

[Evaluation items of safety]

The following items were measured, examined and investigated by the principal investigator and the like at a predetermined time.

(1) Dose-limiting toxicity (DLT), (2) Adverse events, (3) Clinical examination (hematological tests, blood biochemical tests, hormonal tests, blood coagulation system tests, urine qualitative tests, immunological tests), (4) Vital signs (systolic/diastolic blood pressure, pulse rate, body temperature), transcutaneous oxygen saturation ($SpO_2$), body weight, (5) 12 lead electrocardiogram, (6) ECOG Performance Status, (7) Chest X-ray, (8) Ophthalmologic examination (visual acuity test, tonometry, slit lamp microscopy, and (9) CT examination

[Results of Efficacy Evaluation]

At the time when 8 months had elapsed since the registration of the first patient, one case was determined to be PR.

INDUSTRIAL APPLICABILITY

The present invention provides a novel method for treating cancer method and is useful.

## Claims

1. An agent for suppressing the progression of, suppressing the recurrence of and/or treating cancer comprising an $EP_4$ antagonist, as an active ingredient, which is **characterized by** being administered in combination with a standard

therapy and an administration of an immune checkpoint inhibitor, wherein the $EP_4$ antagonist is a compound represented by general formula (I) or a salt thereof:

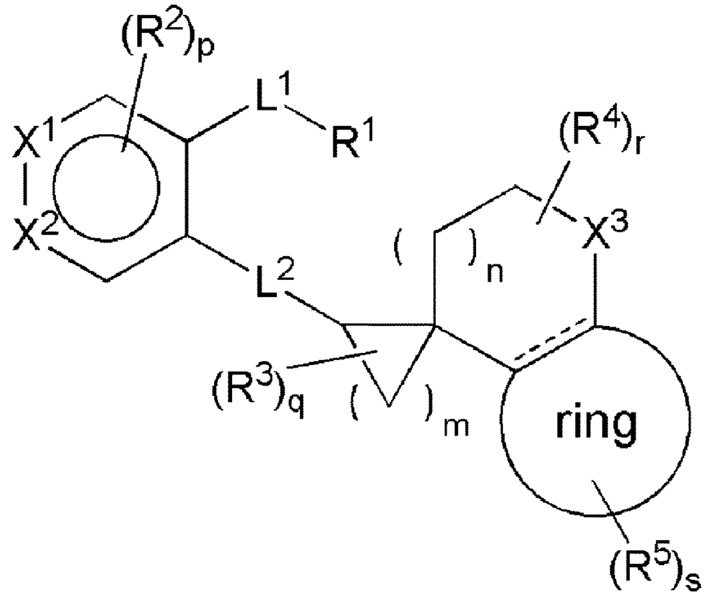

(I)

(wherein $R^1$ represents $COOR^8$, tetrazole, $SO_3H$, $SO_2NH_2$, $SO_2NHR^{8-1}$, $CONHSO_2R^{8-1}$, $SO_2NHCOR^{8-1}$, or hydroxamic acid,

wherein $R^8$ represents a hydrogen atom, C1-4 alkyl, or benzyl, and
$R^{8-1}$ represents C1-4 alkyl, C1-4 haloalkyl, a C3-10 carbon ring, or a three- to ten-membered heterocyclic ring, wherein the C3-10 carbon ring and the three- to ten-membered heterocyclic ring each may be substituted with C1-4 alkyl, C1-4 haloalkyl, C1-4 alkoxy, - O(C1-4 haloalkyl), C1-4 alkylthio, -S(C1-4 haloalkyl), halogen, or nitrile (here and below, "-CN"),
$L^1$ represents C1-5 alkylene, C2-5 alkenylene, or C2-5 alkynylene,
$R^2$ represents halogen, C1-4 alkyl, C1-4 alkoxy, C1-4 alkylthio, C2-4 alkenyl, C2-4 alkynyl, -O(C1-4 haloalkyl), -S(C1-4 haloalkyl), -C(O)(C1-4 alkyl), $-SO_2$(C1-4 alkyl),-CONH(C1-4 alkyl), -CON(C1-4 $alkyl)_2$, -NHC(O)(C1-4 alkyl), -N(C1-4 alkyl)C(O)(C1-4 alkyl), $-NHSO_2$(C1-4 alkyl), -N(C1-4 $alkyl)SO_2$(C1-4 alkyl), $-SO_2NH$(C1-4 alkyl),$-SO_2N$(C1-4 $alkyl)_2$, $-NR^{17}R^{17}$, nitro, nitrile, a hydroxyl group, aldehyde (here and below, formyl), or carboxyl, wherein the C1-4 alkyl groups each may be substituted with halogen, and
the (C1-4 $alkyl)_2$ in $R^2$ represents two independent C1-4 alkyl groups which may be the same or different,
$X^1$ represents $CR^6$ or a nitrogen atom, wherein $R^6$ represents a hydrogen atom or $R^2$,
$X^2$ represents $CR^7$ or a nitrogen atom, wherein $R^7$ represents a hydrogen atom, $R^2$, or $-L^3-R^9$, wherein $L^3$ represents methylene, an oxygen atom, or a sulfur atom which may be oxidized, and $R^9$ represents a four- to ten-membered heterocyclic ring which may be substituted with a substituent selected from the group consisting of halogen, C1-4 alkyl, and C1-4 haloalkyl,
$L^2$ represents $-CH_2CH_2-$, -CH=CH-, $-CH_2O-$, $-OCH_2-$, $-CH_2S-$, $-SCH_2-$, $-CH_2S(O)-$, $-S(O)CH_2-$, $-CH_2SO_2-$, $-SO_2CH_2-$, $-CH_2NH-$, $-NHCH_2-$, -NHCO-, -CONH-, $-NHSO_2-$, or$-SO_2NH-$,
$R^3$ represents C1-4 alkyl or halogen,
$R^4$ represents halogen, C1-4 alkyl, or C1-4 haloalkyl,
$X^3$ represents methylene, an oxygen atom, a sulfur atom which may be oxidized, or $NR^{10}$, wherein $R^{10}$ represents C1-4 alkyl, -C(O)(C1-4 alkyl), -C(O)O(C1-4 alkyl), or - $SO_2$(Cl-4 alkyl), wherein the C1-4 alkyl groups each may be substituted with halogen,
the ring represents a benzene ring or a five- or six-membered monocyclic aromatic heterocyclic ring,

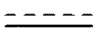

represents a single bond or a double bond,
$R^5$ represents (1) halogen, (2) C1-4 alkyl, (3) carboxyl, (4) nitrile, (5) $-CONHR^{11}$, (6) $-C(O)R^{12}$, (7) $-OR^{14}$, (8) $-S(O)_tR^{15}$, (9) $-CH_2R^{16}$, (10) $-NR^{17}R^{17}$, (11) $-NHCOR^{11}$, (12) a C4-10 carbon ring, or (13) a four- to ten-membered

heterocyclic ring, wherein the C4-10 carbon ring or the four- to ten-membered heterocyclic ring may be substituted with one to three $R^{18}$, wherein, when a plurality of $R^{18}$ exists, the plurality of $R^{18}$ each independently may be the same or different,
$R^{11}$ represents C1-6 alkyl, C3-6 cycloalkyl, phenyl, or a four- to six-membered heterocyclic ring and may be substituted with one to three $R^{13}$, wherein, when a plurality of $R^{13}$ exists, the plurality of $R^{13}$ each independently may be the same or different, and
$R^{13}$ represents halogen, C1-6 alkyl, C3-6 cycloalkyl, C1-4 alkoxy, a hydroxyl group, $-NR^{20}R^{21}$, benzene, or a four- to six-membered heterocyclic ring,
wherein $R^{20}$ and $R^{21}$ each independently represent a hydrogen atom or C1-4 alkyl,
$R^{12}$ represents C1-6 alkyl, C3-6 cycloalkyl, benzene, or a four- to six-membered heterocyclic ring, wherein the C3-6 cycloalkyl, the benzene, and the four- to six-membered heterocyclic ring each independently may be substituted with halogen, C1-4 alkyl, or C1-4 alkoxy,
$R^{14}$ represents a hydrogen atom, C1-6 alkyl, C3-6 cycloalkyl, benzene, or benzyl, wherein the C1-6 alkyl may be substituted with one to three $R^{19}$, wherein, when a plurality of $R^{19}$ exists, the plurality of $R^{19}$ each independently may be the same or different, and
$R^{19}$ represents C1-4 alkoxy, -CONH(C1-4 alkyl), $-CON(C1\text{-}4\ alkyl)_2$, or a five- or six-membered monocyclic aromatic heterocyclic ring which may be substituted with a substituent selected from the group consisting of C1-4 alkyl and C1-4 haloalkyl,
wherein the $(C1\text{-}4\ alkyl)_2$ in $R^{19}$ represents two independent C1-4 alkyl groups which may be the same or different,
$R^{15}$ represents C1-6 alkyl, C3-6 cycloalkyl, benzene, or benzyl,
$R^{16}$ represents a hydroxyl group or C1-4 alkoxy,
each $R^{17}$ independently represents a hydrogen atom, C1-6 alkyl, or C3-6 cycloalkyl, and
$R^{18}$ represents halogen, C1-6 alkyl, C3-6 cycloalkyl, C1-4 alkoxy, oxo, nitrile, a hydroxyl group, hydroxymethyl, 1-methyl-1-hydroxyethyl, $(C1\text{-}4\ alkyl)SO_2$-, a four- to six-membered heterocyclic ring, (C1-4 alkyl)NH-, or $(C1\text{-}4\ alkyl)_2N$-,
wherein the $(C1\text{-}4\ alkyl)_2$ in $R^{18}$ represents two independent C1-4 alkyl groups which may be the same or different,
m represents an integer of 1 to 4,
n represents an integer of 0 to 4,
p represents an integer of 0 to 2,
q represents an integer of 0 to 6,
r represents an integer of 0 to 6,
s represents an integer of 0 to 4,
t represents an integer of 0 to 2, and
$R^2$, $R^3$, $R^4$, and $R^5$ each independently may be the same or different when p, q, r, and s are each an integer of 2 or more).

2. An agent for suppressing the progression of, suppressing the recurrence of and/or treating cancer comprising an immune checkpoint inhibitor, as an active ingredient, which is **characterized by** being administered in combination with a standard therapy and an administration of an $EP_4$ antagonist, wherein the $EP_4$ antagonist is the compound represented by general formula (I) as defined in claim 1 or a salt thereof.

3. The agent according to claim 1 or 2, wherein the cancer is colorectal cancer.

4. The agent according to any one of claims 1 to 3, wherein the standard therapy is a Bevacizumab-and-XELOX therapy.

5. The agent according to any one of claims 1 to 4, wherein the Bevacizumab-and-XELOX therapy comprises:

(i) administering Bevacizumab at a dose of 7.5 mg/kg per administration at 3-week intervals;
(ii) administering Oxaliplatin at a dose of 130 $mg/m^2$ at 3-week intervals; and
(iii) administering Capecitabine orally at a dose of 1000 $mg/m^2$ twice a day for 14 days, with a 7-day rest period.

6. The agent according to claim 1 or 2, wherein the cancer is pancreatic cancer.

7. The agent according to claim 1, 2 or 6, wherein the standard therapy is a FOLFIRINOX therapy or a dose reduction regimen thereof.

8. The agent according to claim 7, wherein the FOLFIRINOX therapy comprises:

(i) administering 85 mg/$m^2$ of Oxaliplatin intravenously over 2 hours;
(ii) administering 200 mg/$m^2$ of Levofolinate calcium intravenously over 2 hours;
(iii) administering 180 mg/$m^2$ of Irinotecan hydrochloride hydrate intravenously over 1.5 hours from 30 minutes after start of the administration of Levofolinate calcium;
(iv) further administering 400 mg/$m^2$ of Fluorouracil rapidly intravenously after completion of the administration of the Levofolinate calcium; and
(v) further administering 2400 mg/$m^2$ of Fluorouracil continuously intravenously over 46 hours,

wherein the series of the administrations are performed at 2-week intervals.

**9.** The agent according to claim 7, wherein the dose reduction regimen of the FOLFIRINOX therapy comprises:

(i) administering 85 mg/$m^2$ of Oxaliplatin intravenously over 2 hours;
(ii) administering 200 mg/$m^2$ of Levofolinate calcium intravenously over 2 hours;
(iii) administering 150 mg/$m^2$ of Irinotecan hydrochloride hydrate intravenously over 1.5 hours from 30 minutes after start of the administration of Levofolinate calcium; and
(iv) further administering 2400 mg/$m^2$ of Fluorouracil continuously intravenously over 46 hours after the completion of the administration of the Levofolinate calcium,

wherein the series of the administrations are performed at 2-week intervals.

**10.** The agent according to claim 1, 2 or 6, wherein the standard therapy is a Gemcitabine-and-nab-Paclitaxel therapy.

**11.** The agent according to claim 10, wherein the Gemcitabine-and-nab-Paclitaxel therapy comprises:

(i) administering 1000 mg/$m^2$ of Gemcitabine intravenously over 30 minutes; and
(ii) administering 125 mg/$m^2$ of nab-Paclitaxel intravenously over 30 minutes, wherein the series of the administrations are performed at 1-week intervals, and continued for 3 weeks with a one-week rest period.

**12.** The agent according to claim 1 or 2, wherein the cancer is lung cancer.

**13.** The agent according to claim 1, 2 or 12, wherein the standard therapy is a Docetaxel-and/or-Ramucirumab therapy.

**14.** The agent according to claim 13, wherein the standard therapy is a Docetaxel therapy.

**15.** The agent according to claim 14, wherein the Docetaxel therapy is a therapy in which 60 mg/$m^2$ of Docetaxel is intravenously administered over 60 minutes or longer, wherein the administration is performed at 3-week intervals.

**16.** The agent according to claim 1, 2, 12 or 13, wherein the standard therapy is a Docetaxel-and-Ramucirumab therapy.

**17.** The agent according to claim 16, wherein the Docetaxel-and-Ramucirumab therapy comprises:

(i) administering 60 mg/$m^2$ of Docetaxel intravenously over 60 minutes or longer; and
(ii) administering 10 mg/kg of Ramucirumab intravenously over 60 minutes,
wherein the series of the administrations are performed at 3-week intervals.

**18.** An agent for suppressing the progression of, suppressing the recurrence of and/or treating colorectal cancer comprising an $EP_4$ antagonist, as an active ingredient, which is **characterized by** being administered to a cancer patient who has undergone preoperative chemoradiotherapy, wherein the $EP_4$ antagonist is a compound represented by general formula (I) as defined in claim 1 or a salt thereof.

**19.** The agent according to claim 18, wherein the agent is administered in further combination with an immune checkpoint inhibitor.

**20.** An agent for suppressing the progression of, suppressing the recurrence of and/or treating colorectal cancer comprising an immune checkpoint inhibitor, as an active ingredient, which is **characterized by** being administered to a cancer patient who has undergone preoperative chemoradiotherapy in combination with an $EP_4$ antagonist, wherein the $EP_4$ antagonist is a compound represented by general formula (I) as defined in claim 1 or a salt thereof.

**21.** The agent according to any one of claims 18 to 20, wherein the immune checkpoint inhibitor and/or the $EP_4$ antagonist is used as preoperative adjuvant therapy after preoperative chemoradiotherapy (CRT).

**22.** The agent according to any one of claims 18 to 21, wherein the preoperative chemoradiotherapy is a combination of a radiation therapy and Capecitabine.

**23.** The agent according to any one of claims 18 to 22, wherein the preoperative chemoradiotherapy is a therapy in which 45 Gy/25 times of irradiation to a pelvic cavity and 5.4 Gy/3 times of boost irradiation to a primary lesion are performed, and 825 mg/$m^2$ of Capecitabine is administered twice a day for 21 days or 42 times or more.

**24.** The agent according to any one of claims 1 to 23, wherein the $EP_4$ antagonist is 4-[4-cyano-2-({(2'R,4S)-6-[(propane-2-yl)carbamoyl]-2,3-dihydrospiro[1-benzopyran-4,1'-cyclopropane]-2'-carbonyl}amino)phenyl]butanoic acid or a salt thereof.

**25.** The agent according to claim 24, wherein the $EP_4$ antagonist is orally administered at a dose of 5 mg to 40 mg once a day.

**26.** The agent according to claim 25, wherein the $EP_4$ antagonist is administered orally at a dose of 20 mg or 40 mg per administration once a day.

**27.** The agent according to any one of claims 1 to 17 or 19 to 26, wherein the immune checkpoint inhibitor is an anti-PD-1 antibody, an anti-PD-L1 antibody, or an anti-CTLA-4 antibody.

**28.** The agent according to any one of claims 1 to 17 or 19 to 27, wherein the immune checkpoint inhibitor is an anti-PD-1 antibody.

**29.** The agent according to claim 28, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, MGA012, AGEN2034, CS1003, HLX10, BAT-1306, AK105, AK103, BI 754091, LZM009, CMAB819, Sym021, GB226, SSI-361, JY034, HX008, ISU106, ABBV181, BCD-100, PF-06801591, CX-188, JNJ-63723283 or AB122.

**30.** The agent according to claim 29, wherein the anti-PD-1 antibody is Nivolumab.

**31.** The agent according to claim 30, wherein Nivolumab is administered (i) at a dose of 240 mg per administration at 2-week intervals, (ii) at a dose of 360 mg per administration at 3-week intervals, or (iii) at a dose of 480 mg per administration at 4-week intervals.

**32.** An $EP_4$ antagonist for use in the suppression of the progression of, suppression of the recurrence of and/or treatment of cancer, wherein the $EP_4$ antagonist being administered in combination with a standard therapy and an administration of an immune checkpoint inhibitor, wherein the $EP_4$ antagonist is the compound represented by general formula (1) as defined in claim 1 or a salt thereof.

**33.** An immune checkpoint inhibitor for use in the suppression of the progression of, suppression of the recurrence of and/or treatment of cancer, wherein the immune checkpoint inhibitor being administered in combination with a standard therapy and an administration of an $EP_4$ antagonist, wherein the $EP_4$ antagonist is the compound represented by general formula (I) as defined in claim 1 or a salt thereof.

**34.** Use of an $EP_4$ antagonist for the manufacture of an agent for suppressing the progression of, suppressing the recurrence of and/or treating cancer, which is administered in combination with a standard therapy and an administration of an immune checkpoint inhibitor, wherein the $EP_4$ antagonist is the compound represented by the general formula (I) as defined in claim 1 or a salt thereof.

**35.** Use of an immune checkpoint inhibitor for the manufacture of an agent for suppressing the progression of, suppressing the recurrence of and/or treating cancer, which is administered in combination with a standard therapy and an administration of an $EP_4$ antagonist, wherein the $EP_4$ antagonist is the compound represented by general formula (I) as defined in claim 1 or a salt thereof.

**36.** A method for suppressing the progression of, suppressing the recurrence of and/or treating cancer, the method comprising administering an effective amount of a standard therapy, an effective amount of an immune checkpoint inhibitor and an effective amount of an $EP_4$ antagonist to a patient in need of cancer treatment separately or simultaneously, wherein the $EP_4$ antagonist is the compound represented by general formula (I) as defined in claim 1 or a salt thereof.

FIG. 1

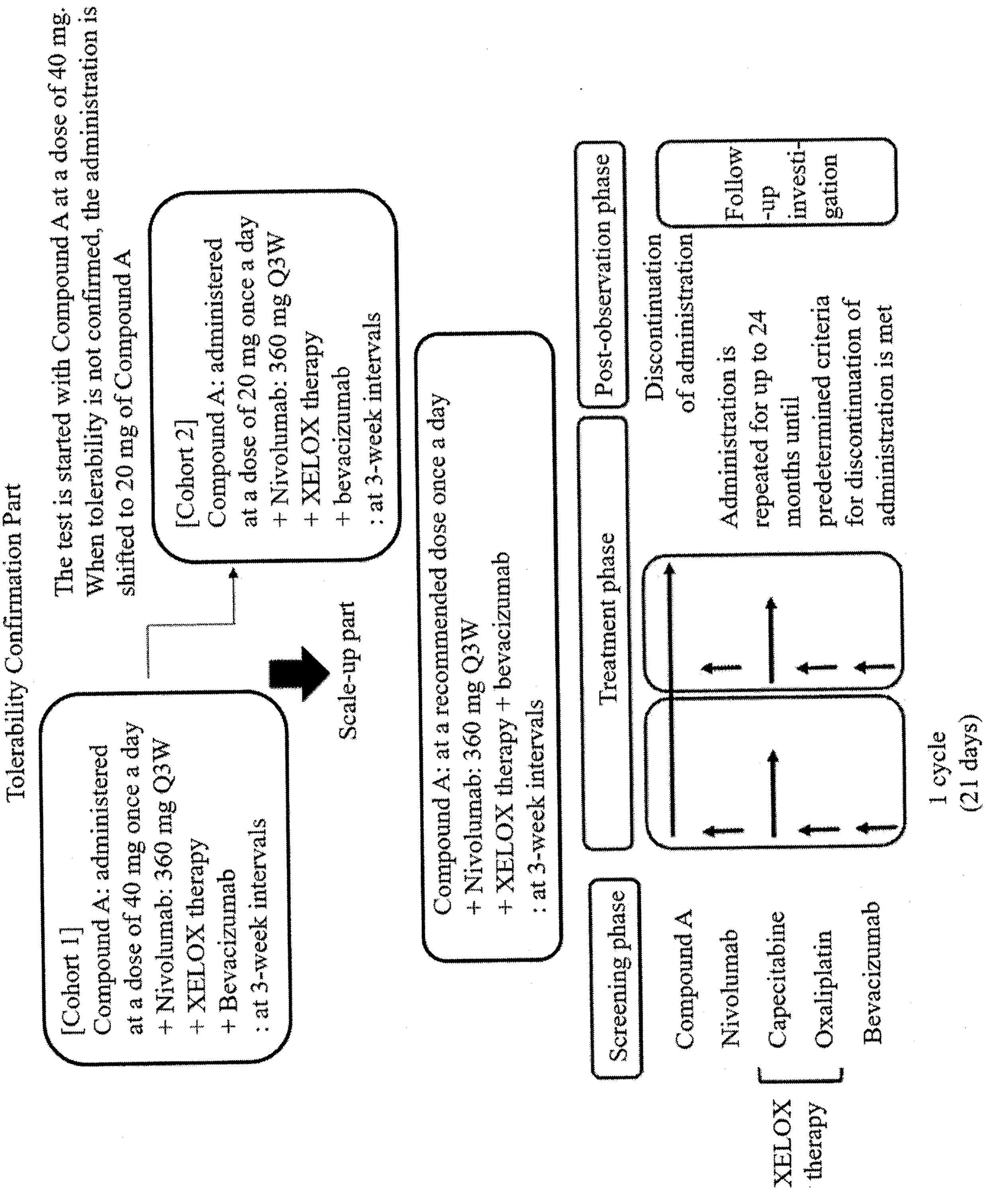

Tolerability Confirmation Part
The test is started with Compound A at a dose of 40 mg. When tolerability is not confirmed, the administration is shifted to 20 mg of Compound A
[Cohort 1]
Compound A: administered at a dose of 40 mg once a day
+ Nivolumab: 360 mg Q3W
+ XELOX therapy
+ Bevacizumab
: at 3-week intervals
[Cohort 2]
Compound A: administered at a dose of 20 mg once a day
+ Nivolumab: 360 mg Q3W
+ XELOX therapy
+ bevacizumab
: at 3-week intervals
Scale-up part
Compound A: at a recommended dose once a day
+ Nivolumab: 360 mg Q3W
+ XELOX therapy + bevacizumab
: at 3-week intervals
Screening phase
Treatment phase
Post-observation phase
Compound A
Nivolumab
Capecitabine
Oxaliplatin
Bevacizumab
XELOX therapy
1 cycle
(21 days)
Administration is repeated for up to 24 months until predetermined criteria for discontinuation of administration is met
Discontinuation of administration
Follow-up investigation

FIG. 2

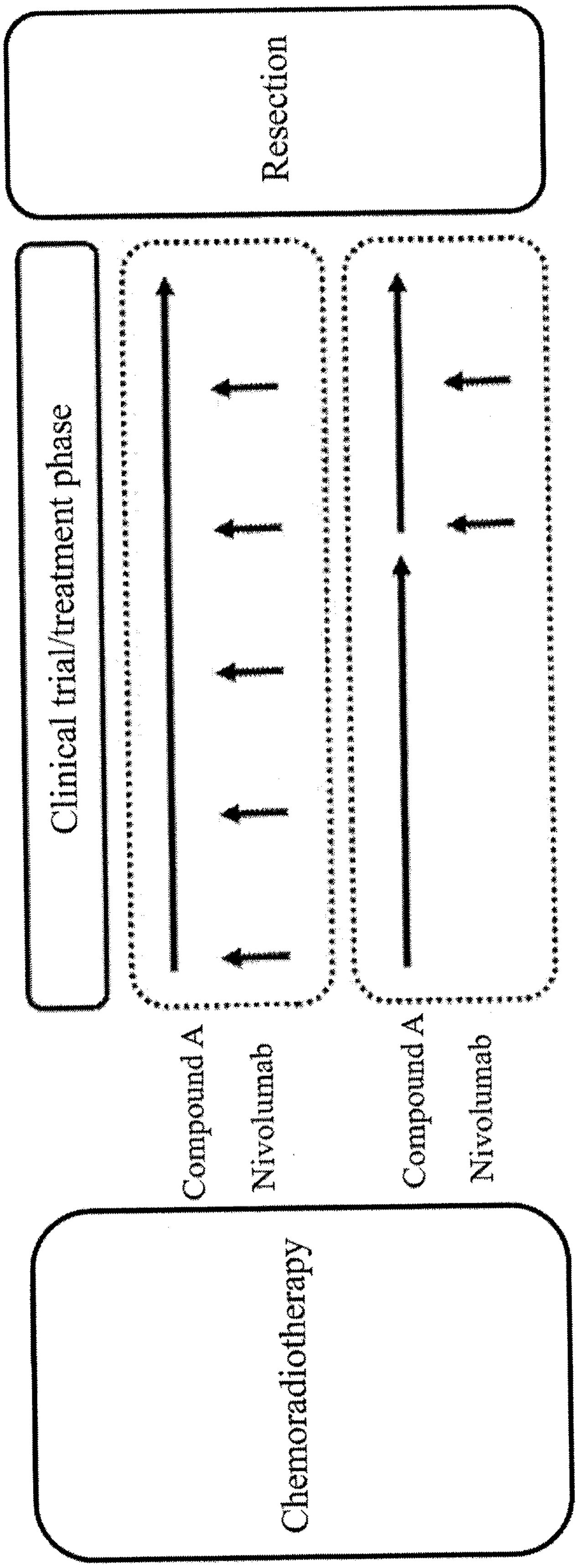
Chemoradiotherapy
Compound A
Nivolumab
Compound A
Nivolumab
Clinical trial/treatment phase
Resection

FIG. 3

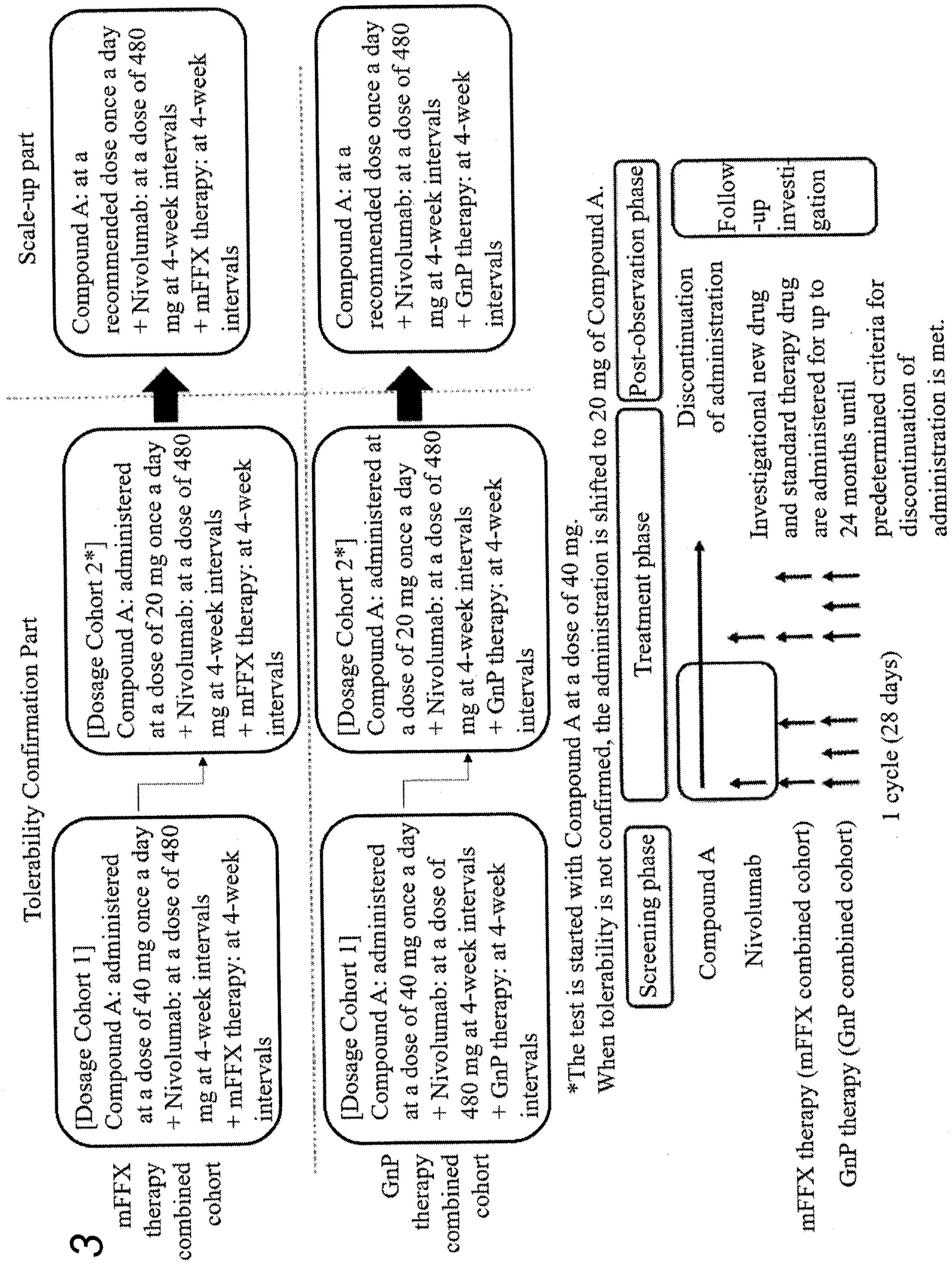
Tolerability Confirmation Part
Scale-up part
mFFX therapy combined cohort
[Dosage Cohort 1]
Compound A: administered at a dose of 40 mg once a day
+ Nivolumab: at a dose of 480 mg at 4-week intervals
+ mFFX therapy: at 4-week intervals
[Dosage Cohort 2*]
Compound A: administered at a dose of 20 mg once a day
+ Nivolumab: at a dose of 480 mg at 4-week intervals
+ mFFX therapy: at 4-week intervals
Compound A: at a recommended dose once a day
+ Nivolumab: at a dose of 480 mg at 4-week intervals
+ mFFX therapy: at 4-week intervals
GnP therapy combined cohort
[Dosage Cohort 1]
Compound A: administered at a dose of 40 mg once a day
+ Nivolumab: at a dose of 480 mg at 4-week intervals
+ GnP therapy: at 4-week intervals
[Dosage Cohort 2*]
Compound A: administered at a dose of 20 mg once a day
+ Nivolumab: at a dose of 480 mg at 4-week intervals
+ GnP therapy: at 4-week intervals
Compound A: at a recommended dose once a day
+ Nivolumab: at a dose of 480 mg at 4-week intervals
+ GnP therapy: at 4-week intervals
*The test is started with Compound A at a dose of 40 mg.
When tolerability is not confirmed, the administration is shifted to 20 mg of Compound A.
Screening phase
Treatment phase
Post-observation phase
Compound A
Nivolumab
mFFX therapy (mFFX combined cohort)
GnP therapy (GnP combined cohort)
1 cycle (28 days)
Investigational new drug and standard therapy drug are administered for up to 24 months until predetermined criteria for discontinuation of administration is met.
Discontinuation of administration
Follow-up investigation

FIG. 4

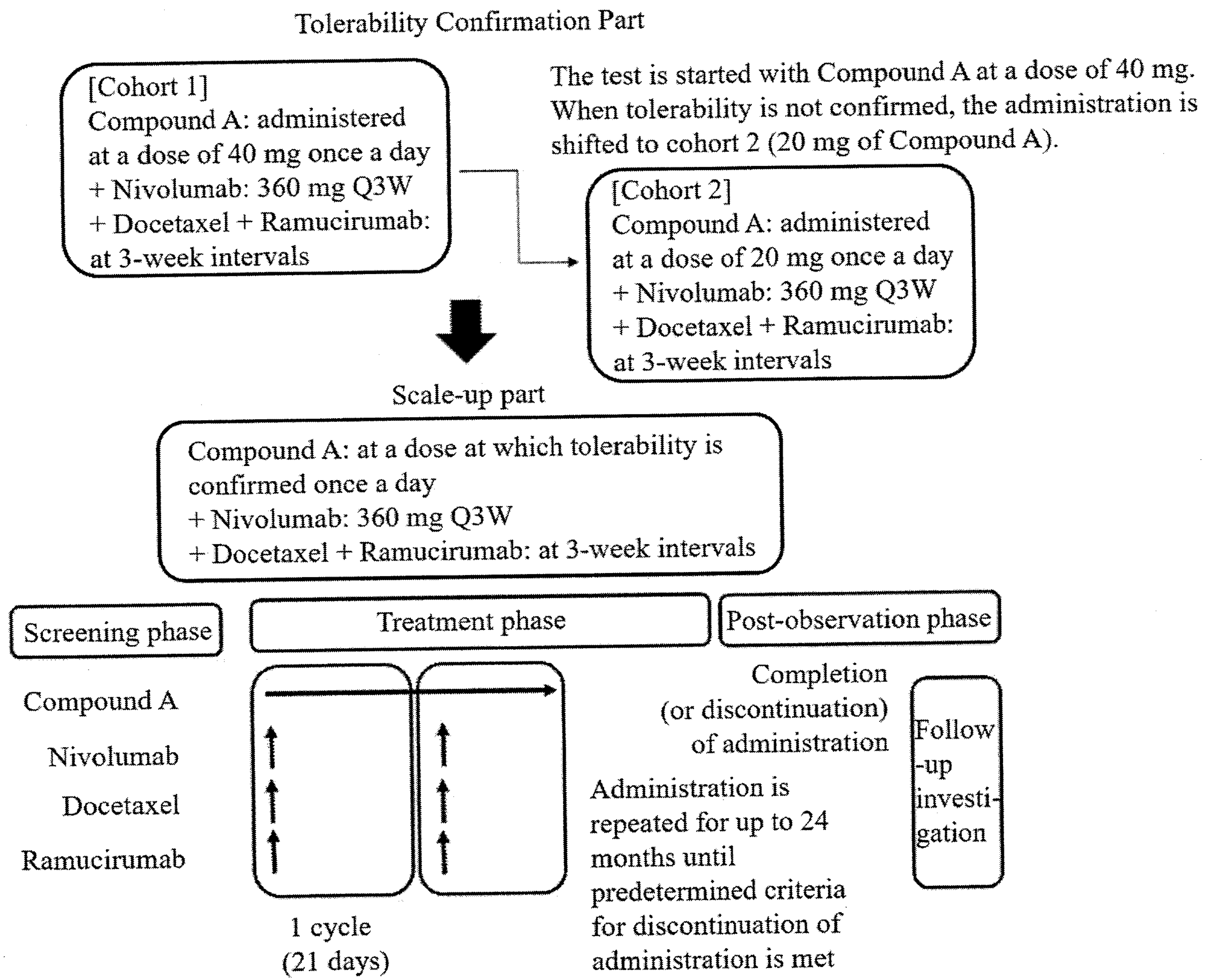
Tolerability Confirmation Part
[Cohort 1]
Compound A: administered at a dose of 40 mg once a day
+ Nivolumab: 360 mg Q3W
+ Docetaxel + Ramucirumab: at 3-week intervals
The test is started with Compound A at a dose of 40 mg. When tolerability is not confirmed, the administration is shifted to cohort 2 (20 mg of Compound A).
[Cohort 2]
Compound A: administered at a dose of 20 mg once a day
+ Nivolumab: 360 mg Q3W
+ Docetaxel + Ramucirumab: at 3-week intervals
Scale-up part
Compound A: at a dose at which tolerability is confirmed once a day
+ Nivolumab: 360 mg Q3W
+ Docetaxel + Ramucirumab: at 3-week intervals
Screening phase
Treatment phase
Post-observation phase
Compound A
Nivolumab
Docetaxel
Ramucirumab
1 cycle
(21 days)
Completion
(or discontinuation)
of administration
Administration is repeated for up to 24 months until predetermined criteria for discontinuation of administration is met
Follow-up investigation

## INTERNATIONAL SEARCH REPORT

International application No.
**PCT/JP2021/041650**

**A. CLASSIFICATION OF SUBJECT MATTER**

***A61K 31/353***(2006.01)i; ***A61K 31/282***(2006.01)i; ***A61K 31/337***(2006.01)i; ***A61K 31/513***(2006.01)i; ***A61K 31/519***(2006.01)i; ***A61K 31/7068***(2006.01)i; ***A61K 39/395***(2006.01)i; ***A61K 45/00***(2006.01)i; ***A61P 1/00***(2006.01)i; ***A61P 35/00***(2006.01)i; ***A61P 35/04***(2006.01)i; ***A61P 37/04***(2006.01)i; ***A61P 43/00***(2006.01)i

FI: A61K31/353; A61K31/282; A61K31/337; A61K31/513; A61K31/519; A61K31/7068; A61K39/395 N; A61K39/395 T; A61K39/395 U; A61K45/00; A61P1/00; A61P35/00; A61P35/04; A61P37/04; A61P43/00 111; A61P43/00 121

According to International Patent Classification (IPC) or to both national classification and IPC

**B. FIELDS SEARCHED**

Minimum documentation searched (classification system followed by classification symbols)

A61K31/353; A61K31/282; A61K31/337; A61K31/513; A61K31/519; A61K31/7068; A61K39/395; A61K45/00; A61P1/00; A61P35/00; A61P35/04; A61P37/04; A61P43/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2018/008711 A1 (ONO PHARMACEUTICAL CO.) 11 January 2018 (2018-01-11) claims 1, 16, paragraphs [0227], [0234], [0242], [0243], examples | 1-3, 6, 12, 24-36 |
| Y | claims 1, 16, paragraphs [0234], [0242], [0243], examples | 4-5, 7-11, 13-31 |
| Y | WO 2017/014177 A1 (TOPPAN PRINTING CO., LTD.) 26 January 2017 (2017-01-26) paragraphs [0056], [0065] | 4-5, 24-31 |
| Y | WO 2020/111018 A1 (ONO PHARMACEUTICAL CO.) 04 June 2020 (2020-06-04) claims | 7-9, 24-31 |
| Y | CHAN, Kelvin et al. A Bayesian Meta-Analysis of Multiple Treatment Comparisons of Systemic Regimens for Advanced Pancreatic Cancer, PLOS ONE, 2014, vol. 9, no. 10, e108749, pp. 1-9 p. 1, methods | 7-11, 24-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/041650**

**C. DOCUMENTS CONSIDERED TO BE RELEVANT**

| Category* | Citation of document, with indication, where appropriate, of the relevant passages | Relevant to claim No. |
|---|---|---|
| Y | WO 2019/117269 A1 (UNIVERSITY OF HIROSHIMA) 20 June 2019 (2019-06-20) paragraph [0022] | 7-11, 24-31 |
| Y | GARON, Edward B. et al. Ramucirumab plus docetaxel versus placebo plus docetaxel for second-line treatment of stage IV non-small-cell lung cancer after disease progression on platinum-based therapy (REVEL): a multicentre, double-blind, randomised phase 3 trial, Lancet, 2014, vol. 384, pp. 665-673 p. 665, Summary | 13-17, 24-31 |
| Y | 楊知明ほか, Capecitabineによる術前化学放射線療法が奏効し治癒切除が得られた局所進行Ｓ状結腸癌の１例, 癌と化学療法, 2011, vol. 38, no. 6, pp. 1021-1024 (YOH, Tomoaki. A Case of Effective Neoadjuvant Chemoradiotherapy with Capecitabine for Locally Advanced Sigmoid Colon Cancer. Japanese Journal of Cancer and Chemotherapy.) p. 1021, abstract | 18-31 |
| A | WO 2016/111347 A1 (ONO PHARMACEUTICAL CO.) 14 July 2016 (2016-07-14) | 1-36 |
| A | JP 2018-21013 A (ONO PHARMACEUTICAL CO) 08 February 2018 (2018-02-08) | 1-36 |

Form PCT/ISA/210 (second sheet) (January 2015)

## INTERNATIONAL SEARCH REPORT
**Information on patent family members**

International application No.
**PCT/JP2021/041650**

| Patent document cited in search report | Publication date (day/month/year) | Patent family member(s) | Publication date (day/month/year) |
|---|---|---|---|
| WO 2018/008711 A1 | 11 January 2018 | US 2019/0255013 A1<br>claims 1, 16, paragraphs [0342], [0351], [0359], [0360], example<br>EP 3482760 A1<br>CN 109475520 A<br>KR 10-2019-0026736 A | |
| WO 2017/014177 A1 | 26 January 2017 | US 2018/0135138 A1<br>paragraphs [0092], [0104] | |
| WO 2020/111018 A1 | 04 June 2020 | (Family: none) | |
| WO 2019/117269 A1 | 20 June 2019 | EP 3725881 A1<br>paragraph [0021] | |
| WO 2016/111347 A1 | 14 July 2016 | US 2018/0002308 A1<br>EP 3243813 A1<br>CN 107108480 A<br>KR 10-2017-0102885 A | |
| JP 2018-21013 A | 08 February 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016111347 A
- WO 2018008711 A
- ER 819762
- WO 2001062708 A
- WO 2002020462 A
- WO 2002032900 A
- WO 2002050031 A
- WO 2002050032 A
- WO 2002050033 A
- WO 2002016311 A
- WO 2003086390 A
- WO 2003087061 A
- WO 2003099857 A
- WO 2003016254 A
- WO 2005021508 A
- WO 2004067524 A
- WO 2005037812 A
- WO 2005061475 A
- WO 2005105732 A
- WO 2005105733 A
- WO 2006122403 A
- WO 2007121578 A
- WO 2008017164 A
- WO 2008104055 A
- WO 2008116304 A
- WO 2008123207 A
- WO 2007143825 A
- WO 2009005076 A
- WO 2009139373 A
- WO 2010019796 A
- WO 2010034110 A
- WO 2012039972 A
- WO 2012043634 A
- WO 2012076063 A
- WO 2012103071 A
- WO 2013004290 A
- WO 2013096496 A
- WO 2013096501 A
- WO 2013101733 A
- WO 2013101598 A
- WO 2014004229 A
- WO 2014004230 A
- WO 2014086739 A
- WO 2014126746 A
- WO 2014186218 A
- WO 2014200075 A
- WO 2015094902 A
- WO 2015094912 A
- WO 2015091475 A
- WO 2015113057 A
- WO 2015147020 A
- WO 2016021742 A
- WO 2016088903 A
- WO 2017014323 A
- WO 2017066633 A
- WO 2017085198 A
- WO 2018195123 A
- WO 2018210987 A
- WO 2018210988 A
- WO 2018210992 A
- WO 2018210994 A
- WO 2018210995 A
- WO 2018216640 A
- WO 2019038156 A
- WO 2019245590 A
- WO 2019101171 A
- WO 2019152982 A
- WO 2019166022 A
- CN 108929281
- WO 2020012305 A
- WO 2020014465 A
- WO 2020151566 A
- WO 2020160075 A
- WO 2020161275 A
- WO 2006007448 A
- WO 2006121168 A
- WO 2008156712 A
- WO 2007005874 A
- WO 2001014424 A


### Non-patent literature cited in the description


- *Journal of Lipid Mediators and Cell Signalling,* 1995, vol. 12, 379-391
- *Pharmacological Reviews,* July 2013, vol. 65, 1010-1052
- *FEBS Letters,* 1995, vol. 364, 339-341
- *Cancer Science,* 2014, vol. 105, 1142-1151
- *Cancer Research,* 2010, vol. 70, 1606-1615
- *Cancer Research,* 2002, vol. 62, 28-32

- **DEVELOPMENT OF DRUGS.** Molecular Design. Hirokawa Publishing Company, 1990, vol. 7, 163-198
- *Nature Reviews Cancer,* 2012, vol. 12, 252-264
- *Cancer Cell,* 2015, vol. 27, 450-461